# EUROPEAN PATENT SPECIFICATION

(11) **EP 2 480 577 B1**
(45) Date of publication and mention of the grant of the patent: **10.04.2019**
(21) Application number: 10761060.2
(22) Date of filing: 24.09.2010
(51) Int. Cl.: C07K 16/00, C07K 16/46, C07K 16/18, C07K 16/28, C07K 16/24, C07K 19/00

(54) **DISULFIDE STABILISED MULTIVALENT ANTIBODIES**
DISULFIDSTABILISIERTE MULTIVALENTE ANTIKÖRPER
ANTICORPS MULTIVALENTS STABILISÉS PAR UN PONT DISULFURE

(30) Priority: 25.09.2009 WO PCT/GB2009/002310; 25.03.2010 GB 201005063
(43) Date of publication of application: 01.08.2012
(73) Proprietor: UCB Biopharma SPRL, 1070 Brussels (BE)
(72) Inventor: ADAMS, Ralph, Slough Berkshire SL1 3WE (GB); HANCOCK, Laura, Slough Berkshire SL1 3WE (GB); HEYWOOD, Sam Philip, Slough Berkshire SL1 3WE (GB)
(74) Representative: Blanchard, Amanda Jane
(86) International application number: PCT/GB2010/001803
(87) International publication number: WO 2011/036460

(56) References cited:
- WO-A1-2009/040562
- WO-A1-2010/035012
- WO-A2-2007/109254
- REITER Y ET AL: "Engineering antibody Fv fragments for cancer detection and therapy: disulfide-stabilized Fv fragments", NATURE BIOTECHNOLOGY, NATURE PUBLISHING GROUP, NEW YORK, NY, US, vol. 14, 1 October 1996 (1996-10-01), pages 1239-1245, XP002467023, ISSN: 1087-0156, DOI: DOI:10.1038/NBT1096-1239
- JUNG S H ET AL: "Design of interchain disulfide bonds in the framework region of the Fv fragment of the monoclonal antibody B3.", PROTEINS MAY 1994 LNKD- PUBMED:8066084, vol. 19, no. 1, May 1994 (1994-05), pages 35-47, XP002621591, ISSN: 0887-3585 cited in the application
- REITER Y ET AL: "STABILIZATION OF THE FV FRAGMENTS IN RECOMBINANT IMMUNOTOXINS BY DISULFIDE BONDS ENGINEERED INTO CONSERVED FRAMEWORK REGIONS", BIOCHEMISTRY, AMERICAN CHEMICAL SOCIETY, US, vol. 33, no. 18, 10 May 1994 (1994-05-10), pages 5451-5459, XP002008021, ISSN: 0006-2960, DOI: DOI:10.1021/BI00184A014 cited in the application
- ZHU Z ET AL: "REMODELING DOMAIN INTERFACES TO ENHANCE HETERODIMER FORMATION", PROTEIN SCIENCE, CAMBRIDGE UNIVERSITY PRESS, CAMBRIDGE, GB, vol. 6, no. 4, 1 April 1997 (1997-04-01), pages 781-788, XP000929847, ISSN: 0961-8368 cited in the application
- YOUNG N M ET AL: "Thermal stabilization of a single-chain Fv antibody fragment by introduction of a disulphide bond", FEBS LETTERS, ELSEVIER, AMSTERDAM, NL, vol. 377, no. 2, 18 December 1995 (1995-12-18), pages 135-139, XP002520976, ISSN: 0014-5793, DOI: DOI:10.1016/0014-5793(95)01325-3 cited in the application
- WU CHENGBIN ET AL: "Simultaneous targeting of multiple disease mediators by a dual-variable-domain immunoglobulin", NATURE BIOTECHNOLOGY, NATURE PUBLISHING GROUP, NEW YORK, NY, US, vol. 25, no. 11, 1 November 2007 (2007-11-01), pages 1290-1297, XP009110104, ISSN: 1087-0156, DOI: DOI:10.1038/NBT1345

## Description

The present invention relates to new dual specificity antibody fusion proteins. Such antibodies comprise a first specificity to an antigen of interest, and a second specificity for a second antigen of interest, for example a serum carrier protein for use in extending their *in vivo* serum half-life. Methods for the production of such molecules and pharmaceutical compositions comprising them are also provided.

The high specificity and affinity of antibodies makes them ideal diagnostic and therapeutic agents, particularly for modulating protein:protein interactions. Advances in the field of recombinant antibody technology have resulted in the production of antibody fragments, such as Fv, Fab, Fab' and F(ab')₂ fragments and other antibody fragments. These smaller molecules retain the antigen binding activity of whole antibodies and can also exhibit improved tissue penetration and pharmacokinetic properties in comparison to whole immunoglobulin molecules. Indeed, antibody fragments are proving to be versatile therapeutic agents, as seen by the recent success of products such as ReoPro® and Lucentis®. Whilst such fragments appear to exhibit a number of advantages over whole immunoglobulins, they also suffer from an increased rate of clearance from serum since they lack the Fc domain that imparts a long lifetime *in vivo* (Medasan et al., 1997, J. Immunol. 158:2211-2217).

Antibodies with dual specificity, *i.e.* which bind to two different antigens have been previously described (for reviews, see Segal et al., 1999, Curr. Opin. Immunol. 11:558-562; Plückthun & Pack, 1997, Immunotechnology, 3:83-105; Fischer and Leger, 2007, Pathobiology, 74, 3-14). Dual specificity antibodies are also described in WO02/02773, US2007065440, US2006257406, US2006106203 and US2006280734. Previous approaches to making hetero-bispecific antibody-based molecules have generally employed chemical cross-linking or protein engineering techniques. Chemical cross-linking suffers from poor yields of hetero- and homo-dimer formation and the requirement for their subsequent chromatographic separation. Protein engineering approaches have either been highly elaborate (*e*.*g*. knobs-into-holes engineering; Ridgway et al., 1996, Protein Eng. 9(7):617-621) or have used molecules with inappropriate stability characteristics (*e*.*g*. diabodies, scFv). In some cases bispecific antibodies can also suffer from steric hindrance problems such that both antigens cannot bind simultaneously to each antibody arm.

WO2007/109254 discloses single-chain Fvs stabilised by a disulfide bond and molecules comprising the same. WO2009/040562 disclosed a Fab molecule attached to two single domain antibodies.

Single variable domain antibodies also known as single domain antibodies or dAbs, correspond to the variable regions of either the heavy (VH) or light (VL) chain of an antibody. Murine single-domain antibodies were described by Ward et al., 1989, Nature, 341, 544-546. Human and 'camelised' human single domain antibodies have also been described (Holt et al., 2003, Trends in Biotechnology, 21, 484-490). Single domain antibodies have also been obtained from the camelids (camels and llamas) and cartilaginous fish (wobbegong and nurse sharks). These organisms have evolved high affinity single V-like domains (called VhH in camelids and V-NAR in sharks), mounted on an Fc-equivalent constant domain framework as an integral and crucial component of their immune system (see Holliger & Hudson, for a review; 2005, Nature Biotechnology, 23(9):1126-1136).

Single domain antibody-enzyme fusions have been described in EP0368684. Single domain-effector group fusions have also been described in WO2004/058820 which comprise a single variable domain. Dual variable domain immunoglobulins have been described in WO2007/024715. Dual specific ligands comprising two single domain antibodies with differing specificities have been described in EP1517921.

Means to improve the half-life of antibody fragments, such as Fv, Fab, Fab', F(ab')₂ and other antibody fragments, are known. One approach has been to conjugate the fragment to polymer molecules. Thus, the short circulating half-life of Fab', F(ab')₂ fragments in animals has been improved by conjugation to polyethylene glycol (PEG; see, for example, WO98/25791, WO99/64460 and WO98/37200). Another approach has been to modify the antibody fragment by conjugation to an agent that interacts with the FcRn receptor (see, for example, WO97/34631). Yet another approach to extend half-life has been to use polypeptides that bind serum albumin (see, for example, Smith et al., 2001, Bioconjugate Chem. 12:750-756; EP0486525; US6267964; WO04/001064; WO02/076489; and WO01/45746). However, there still remains a need to produce antigen-binding immunoglobulin proteins that have a long *in vivo* half-life, as an alternative to those that have a long half life because they interact with the FcRn receptor, without being chemically modified by conjugation to PEG, or being conjugated to human serum albumin.

A variety of proteins exist in plasma and include thyroxine-binding protein, transthyretin, α1-acid glycoprotein, transferrin, fibrinogen and albumin, or a fragment of any thereof. Serum carrier proteins circulate within the body with half-lives measured in days, for example, 5 days for thyroxine-binding protein or 2 days for transthyretin (Bartalena & Robbins, 1993, Clinics in Lab. Med. 13:583-598), or 65 hours in the second phase of turnover of iodinated α1-acid glycoprotein (Bree et al., 1986, Clin. Pharmacokin. 11:336-342). Data from Gitlin et al. (1964, J. Clin. Invest. 10:1938-1951) suggest that in pregnant women, the half-life of α1-acid glycoprotein is 3.8 days, 12 days for transferrin and 2.5 days for fibrinogen. Serum albumin is an abundant protein in both vascular and extravascular compartments with a half-life in man of about 19 days (Peters, 1985, Adv Protein Chem. 37:161-245). This is similar to the half-life of IgG1, which is about 21 days (Waldeman & Strober, 1969, Progr. Allergy, 13:1-110).

The present disclosure provides improved dual specificity antibody fusion proteins which can be produced recombinantly and are capable of binding two antigens simultaneously, in particular two distinct/different antigens.

Thus, the present invention provides a multivalent antibody fusion protein consisting of a Fab or Fab' fragment, with a first specificity for an antigen of interest, and two single domain antibodies (dAb) which are a VH/VL pair with specificity for a second antigen of interest, wherein the two single domain antibodies are linked by a disulfide bond between two cysteine residues, one in VH and one in VL, wherein the position of the two cysteine residues is VH44 and VL100 according to Kabat numbering, and wherein the V_{H} dAb is directly or indirectly connected to the C-terminus of the Fab or Fab' heavy chain by genetic fusion and the V_{L} dAb is directly or indirectly connected to the C-terminus of the Fab or Fab' light chain by genetic fusion.

A single domain antibody as employed to herein does not refer to a single chain Fv. A single chain Fv is characterised by two variable domains which are linked to each other thereby forming an independent entity or linked to another entity through only one of the variable domains therein.

Multivalent as employed herein is intended to refer to an entity that has two or more binding sites, for example two or three binding sites, such as two binding sites. Each binding site may bind the same epitope or different epitopes on the same antigen, or may bind different (distinct) antigens.

A dual specificity antibody fusion of the invention will be capable of selectively binding to two antigens of interest.

In one embodiment the first and second antigen are the same antigen.

In one embodiment, an antigen of interest bound by the Fab or Fab' fragment may be a cell-associated protein, for example a cell surface protein on cells, such as bacterial cells, yeast cells, T-cells, endothelial cells or tumour cells, or it may be a soluble protein. Antigens of interest may also be any medically relevant protein such as those proteins upregulated during disease or infection, for example receptors and/or their corresponding ligands. Particular examples of cell surface proteins include adhesion molecules, for example integrins such as β1 integrins e.g. VLA-4, E-selectin, P selectin or L-selectin, CD2, CD3, CD4, CD5, CD7, CD8, CD11a, CD11b, CD18, CD19, CD20, CD23, CD25, CD33, CD38, CD40, CD45, CDW52, CD69, CD134 (OX40), ICOS, BCMP7, CD137, CD27L, CDCP1, DPCR1, DPCR1, dudulin2, FLJ20584, FLJ40787, HEK2, KIAA0634, KIAA0659, KIAA1246, KIAA1455, LTBP2, LTK, MAL2, MRP2, nectin-like2, NKCC1, PTK7, RAIG1, TCAM1, SC6, BCMP101, BCMP84, BCMP11, DTD, carcinoembryonic antigen (CEA), human milk fat globulin (HMFG1 and 2), MHC Class I and MHC Class II antigens, and VEGF, and where appropriate, receptors thereof.

Soluble antigens include interleukins such as IL-1, IL-2, IL-3, IL-4, IL-5, IL-6, IL-8, IL-12, IL-16 or IL-17, viral antigens for example respiratory syncytial virus or cytomegalovirus antigens, immunoglobulins, such as IgE, interferons such as interferon α, interferon β or interferon γ, tumour necrosis factor-a, tumor necrosis factor-β, colony stimulating factors such as G-CSF or GM-CSF, and platelet derived growth factors such as PDGF-α, and PDGF-β and where appropriate receptors thereof. Other antigens include bacterial cell surface antigens, bacterial toxins, viruses such as influenza, EBV, HepA, B and C, bioterrorism agents, radionuclides and heavy metals, and snake and spider venoms and toxins.

In one example, the antibody fusion protein of the disclosure may be used to functionally alter the activity of the antigen of interest. For example, the antibody fusion protein may neutralize, antagonize or agonise the activity of said antigen, directly or indirectly.

In one example, a second antigen of interest bound by the single domain antibody or antibodies in the dual specificity antibody fusion proteins of the disclosure may be a cell-associated protein, for example a cell surface protein on cells such as bacterial cells, yeast cells, T-cells, endothelial cells or tumour cells, or it may be a soluble protein. Antigens of interest may also be any medically relevant protein such as those proteins upregulated during disease or infection, for example receptors and/or their corresponding ligands. Particular examples of cell surface proteins include adhesion molecules, for example integrins such as β1 integrins e.g. VLA-4, E-selectin, P selectin or L-selectin, CD2, CD3, CD4, CD5, CD7, CD8, CD11a, CD11b, CD18, CD19, CD20, CD23, CD25, CD33, CD38, CD40, CD45, CDW52, CD69, CD134 (OX40), ICOS, BCMP7, CD137, CD27L, CDCP1, DPCR1, DPCR1, dudulin2, FLJ20584, FLJ40787, HEK2, KIAA0634, KIAA0659, KIAA1246, KIAA1455, LTBP2, LTK, MAL2, MRP2, nectin-like2, NKCC1, PTK7, RAIG1, TCAM1, SC6, BCMP101, BCMP84, BCMP11, DTD, carcinoembryonic antigen (CEA), human milk fat globulin (HMFG1 and 2), MHC Class I and MHC Class II antigens, and VEGF, and where appropriate, receptors thereof.

Soluble antigens include interleukins such as IL-1, IL-2, IL-3, IL-4, IL-5, IL-6, IL-8, IL-12, IL-16 or IL-17, viral antigens for example respiratory syncytial virus or cytomegalovirus antigens, immunoglobulins, such as IgE, interferons such as interferon α, interferon β or interferon γ, tumour necrosis factor-α, tumor necrosis factor-β, colony stimulating factors such as G-CSF or GM-CSF, and platelet derived growth factors such as PDGF-α, and PDGF-β and where appropriate receptors thereof. Other antigens include bacterial cell surface antigens, bacterial toxins, viruses such as influenza, EBV, HepA, B and C, bioterrorism agents, radionuclides and heavy metals, and snake and spider venoms and toxins.

Other antigens which may be bound by the single domain antibody or antibodies include serum carrier proteins, polypeptides which enable cell-mediated effector function recruitment and nuclide chelator proteins.

Thus, in one example the present disclosure provides dual specificity antibody fusion proteins which comprise an immunoglobulin moiety with a first specificity for an antigen of interest, and further comprise a single domain antibody with specificity for a second protein, the latter providing the ability to recruit effector functions, such as complement pathway activation and/or effector cell recruitment. Further, fusion proteins of the present disclosure may be used to chelate radionuclides by virtue of a single domain antibody which binds to a nuclide chelator protein. Such fusion proteins are of use in imaging or radionuclide targeting approaches to therapy.

Accordingly, in one example there is provided an isolated dual specificity antibody fusion protein comprising an antibody Fab or Fab' fragment with specificity for an antigen of interest, said fragment being fused to at least one dAb which has specificity for a recruitment polypeptide, said dAb providing the ability to recruit cell-mediated effector function(s), directly or indirectly, by binding to said recruitment polypeptide.

The recruitment of effector function may be direct in that effector function is associated with a cell, said cell bearing a recruitment molecule on its surface. Indirect recruitment may occur when binding of a dAb to a recruitment molecule causes release of, for example, a factor which in turn may directly or indirectly recruit effector function, or may be via activation of a signalling pathway. Examples include TNFα, IL2, IL6, IL8, IL17, IFNγ, histamine, C1q, opsonin and other members of the classical and alternative complement activation cascades, such as C2, C4, C3-convertase, and C5 to C9.

As used herein, 'a recruitment polypeptide' includes a FcγR such as FcγRI, FcγRII and FcγRIII, a complement pathway protein such as, but without limitation, C1q and C3, a CD marker protein (Cluster of Differentiation marker) such as, but without limitation, CD68, CD115, CD16, CD80, CD83, CD86, CD56, CD64, CD3, CD4, CD8, CD28, CD45, CD19, CD20 and CD22. Further recruitment polypeptides which are CD marker proteins include CD1, CD1d, CD2, CD5, CD8, CD9, CD10, CD11, CD11a, CD11b, CD11c, CD13, CD14, CD15, CD16, CD18, CD19, CD20, CD21, CD22, CD23, CD24, CD25, CD26, CD27, CD28, CD29, CD30, CD31, CD32, CD33, CD34, CD35, CD36, CD37, CD38, CD40, CD43, CD44, CD45, CD46, CD49, CD49a, CD49b, CD49c, CD49d, CD52, CD53, CD54, CD55, CD56, CD58, CD59, CD61, CD62, D62E, CD62L, CD62P, CD63, CD64, CD66e, CD68, CD70, CD71, CD72, CD79, CD80, CD81, CD82, CD83, CD84, CD85, CD86, CD88, CD89, CD90, CD94, CD95, CD98, CD106, CD114, CD116, CD117, CD118, CD120, CD122, CD130, CD131, CD132, CD133, CD134, CD135, CD137, CD138, CD141, CD142, CD143, CD146, CD147, CD151, CD152, CD153, CD154, CD155, CD162, CD164, CD169, CD184, CD206, CD209, CD257, CD278, CD281, CD282, CD283 and CD304, or a fragment of any thereof which retains the ability to recruit cell-mediated effector function either directly or indirectly. A recruitment polypeptide also includes immunoglobulin molecules such as IgG1, IgG2, IgG3, IgG4, IgE and IgA which possess effector function.

In one example, the second protein for which the dAb has specificity is a complement pathway protein, with C1q being particularly preferred.

In a preferred example, the second protein for which the dAb has specificity is a CD marker protein, with CD68, CD80, CD86, CD64, CD3, CD4, CD8 CD45, CD16 and CD35 being particularly preferred.

Accordingly, also provided is an isolated dual specificity antibody fusion protein comprising an antibody fragment with specificity for an antigen of interest, said fragment being fused to at least one dAb which has specificity for a CD molecule selected from the group consisting of CD68, CD80, CD86, CD64, CD3, CD4, CD8 CD45, CD16 and CD35.

In one example the single domain antibody or antibodies provide an extended half-life to the immunoglobulin moiety with the first specificity.

Accordingly, in one example there is provided a dual specificity antibody fusion protein comprising an antibody Fab or Fab' fragment with specificity for an antigen of interest, said fragment being fused to at least one single domain antibody which has specificity for a serum carrier protein, a circulating immunoglobulin molecule, or CD35/CR1, said single domain antibody providing an extended half-life to the antibody fragment with specificity for said antigen of interest by binding to said serum carrier protein, circulating immunoglobulin molecule or CD35/CR1.

In one example there is provided an isolated dual specificity antibody fusion protein comprising an antibody Fab or Fab' fragment with specificity for an antigen of interest, said fragment being fused to at least one single domain antibody which has specificity for a serum carrier protein, a circulating immunoglobulin molecule, or CD35/CR1, said single domain antibody providing an extended half-life to the antibody fragment with specificity for said antigen of interest by binding to said serum carrier protein, circulating immunoglobulin molecule or CD35/CR1.

As used herein, 'serum carrier proteins' include thyroxine-binding protein, transthyretin, α1-acid glycoprotein, transferrin, fibrinogen and albumin, or a fragment of any thereof.

As used herein, a 'circulating immunoglobulin molecule' includes IgG1, IgG2, IgG3, IgG4, sIgA, IgM and IgD, or a fragment of any thereof.

CD35/CR1 is a protein present on red blood cells which have a half life of 36 days (normal range of 28 to 47 days; Lanaro et al., 1971, Cancer, 28(3):658-661).

In a preferred example, the second protein for which the dAb has specificity is a serum carrier protein, with a human serum carrier protein being particularly preferred. In a most preferred example, the serum carrier protein is human serum albumin.

Accordingly provided is a dual specificity antibody fusion protein comprising an antibody Fab or Fab' fragment with specificity for an antigen of interest, said fragment being fused to at least one single domain antibody which has specificity for human serum albumin.

In one aspect the present disclosure provides an isolated dual specificity antibody fusion protein comprising an antibody Fab or Fab' fragment with specificity for an antigen of interest, said fragment being fused to at least one single domain antibody which has specificity for human serum albumin.

In one aspect, the antibody fragment with specificity for an antigen of interest is a Fab fragment. In another aspect, the antibody fragment with specificity for an antigen of interest is a Fab' fragment.

Thus, in one most preferred example, the antibody fusion proteins of the invention are translation fusion proteins, *i*.*e*. genetic fusions, the sequence of each of which is encoded by an expression vector.

In one example, the antibody fragments are Fab' fragments which possess a native or a modified hinge region. Where the antibody fragment for use in preparing a dual specificity antibody fusion protein of the invention is a Fab' fragment, said fragment is generally extended at the C-terminus of the heavy chain by one or more amino acids. Thus, an antibody fusion of the disclosure can comprise a Fab' fragment translation fused (or chemically fused) to a dAb, directly or via a linker. Further, examples of suitable antibody Fab' fragments include those described in WO2005/003170 and WO2005/003171.

In another example, the antibody fragments are Fab fragments. Thus, an antibody fusion of the disclosure can comprise a Fab fragment translation fused (or chemically fused) to a linker sequence which in turn is translation fused (or chemically fused) to a dAb. Preferably, the Fab fragment is a Fab fragment which terminates at the interchain cysteines, as described in WO2005/003169. Accordingly, in one example the Fab fragment terminates at position 233 of IgG1.

The antibody Fab or Fab' fragments of use in the present invention can be from any species but are preferably derived from a monoclonal antibody, a human antibody, or are humanised fragments. An antibody fragment for use in the present invention can be derived from any class (e.g. IgG, IgE, IgM, IgD or IgA) or subclass of immunoglobulin molecule and may be obtained from any species including for example mouse, rat, shark, rabbit, pig, hamster, camel, llama, goat or human.

In one example, the antibody Fab or Fab' fragment is a monoclonal, fully human, humanized or chimeric antibody fragment. In one example the antibody Fab or Fab' fragments are fully human or humanised.

Monoclonal antibodies may be prepared by any method known in the art such as the hybridoma technique (Kohler & Milstein, Nature, 1975, 256, 495-497), the trioma technique, the human B-cell hybridoma technique (Kozbor et al., Immunology Today, 1983, 4, 72) and the EBV-hybridoma technique (Cole et al., "Monoclonal Antibodies and Cancer Therapy", pp. 77-96, Alan R. Liss, Inc., 1985).

Antibodies for use in the invention may also be generated using single lymphocyte antibody methods by cloning and expressing immunoglobulin variable region cDNAs generated from single lymphocytes selected for the production of specific antibodies by, for example, the methods described by Babcook, J. et al., Proc. Natl. Acad. Sci. USA, 1996, 93(15), 7843-7848, WO 92/02551, WO2004/051268 and WO2004/106377.

Humanized antibodies are antibody molecules from non-human species having one or more complementarity determining regions (CDRs) from the non-human species and a framework region from a human immunoglobulin molecule (see, for example, US 5,585,089).

The antibodies for use in the present invention can also be generated using various phage display methods known in the art and include those disclosed by Brinkman et al., J. Immunol. Methods, 1995, 182, 41-50; Ames et al., J. Immunol. Methods, 1995, 184, 177-186; Kettleborough et al. Eur. J. Immunol., 1994, 24, 952-958; Persic et al., Gene, 1997 187, 9-18; and Burton et al., Advances in Immunology, 1994, 57, 191-280; WO90/02809; WO91/10737; WO92/01047; WO92/18619; WO93/11236; WO95/15982; and WO95/20401; and US5,698,426; US5,223,409; US5,403,484; US5,580,717; US5,427,908; US5,750,753; US5,821,047; US5,571,698; US5,427,908; US5,516,637; US5,780,225; US5,658,727; US5,733,743; and US5,969,108. Also, transgenic mice, or other organisms, including other mammals, may be used to generate humanized antibodies.

Fully human antibodies are those antibodies in which the variable regions and the constant regions (where present) of both the heavy and the light chains are all of human origin, or substantially identical to sequences of human origin, not necessarily from the same antibody. Examples of fully human antibodies may include antibodies produced for example by the phage display methods described above and antibodies produced by mice in which the murine immunoglobulin variable and/or constant region genes have been replaced by their human counterparts eg. as described in general terms in EP0546073, US5,545,806, US5,569,825, US5,625,126, US5,633,425, US5,661,016, US5,770,429, EP0438474 and EP0463151.

The antibody Fab or Fab' fragment starting material for use in the present invention may be obtained from any whole antibody, especially a whole monoclonal antibody, using any suitable enzymatic cleavage and/or digestion techniques, for example by treatment with pepsin. Alternatively, or in addition the antibody starting material may be prepared by the use of recombinant DNA techniques involving the manipulation and re-expression of DNA encoding antibody variable and/or constant regions. Standard molecular biology techniques may be used to modify, add or delete amino acids or domains as desired. Any alterations to the variable or constant regions are still encompassed by the terms 'variable' and 'constant' regions as used herein.

The antibody fragment starting material may be obtained from any species including for example mouse, rat, rabbit, hamster, camel, llama, goat or human. Parts of the antibody fragment may be obtained from more than one species, for example the antibody fragments may be chimeric. In one example, the constant regions are from one species and the variable regions from another. The antibody fragment starting material may also be modified. In another example, the variable region of the antibody fragment has been created using recombinant DNA engineering techniques. Such engineered versions include those created for example from natural antibody variable regions by insertions, deletions or changes in or to the amino acid sequences of the natural antibodies. Particular examples of this type include those engineered variable region domains containing at least one CDR and, optionally, one or more framework amino acids from one antibody and the remainder of the variable region domain from a second antibody. The methods for creating and manufacturing these antibody fragments are well known in the art (see for example, Boss et al., US 4,816,397; Cabilly et al., US6,331,415; Shrader et al., WO92/02551; Ward et al., 1989, Nature, 341, 544; Orlandi et al., 1989, Proc. Natl. Acad. Sci. USA, 86, 3833; Riechmann et al., 1988, Nature, 322, 323; Bird et al, 1988, Science, 242, 423; Queen et al., US5,585,089; Adair, WO91/09967; Mountain and Adair, 1992, Biotechnol. Genet. Eng. Rev, 10, 1-142; Verma et al., 1998, Journal of Immunological Methods, 216, 165-181).

In the present invention each single domain antibody fused to the Fab or Fab' fragment may linked directly or via a linker.

Linked directly as employed herein is intended to refer to the fact that the "last" amino acid of the Fab or Fab' is joined by a peptide bond to the "first" amino acid of the single domain antibody (or indeed vice versa)

Examples of suitable linker regions for linking a dAb to a Fab or Fab' include, but are not limited to, flexible linker sequences and rigid linker sequences. Flexible linker sequences include those disclosed in Huston et al.,1988, PNAS 85:5879-5883; Wright & Deonarain, Mol. Immunol., 2007, 44(11):2860-2869; Alfthan et al., Prot. Eng., 1995, 8(7):725-731; Luo et al., J. Biochem., 1995, 118(4):825-831; Tang et al., 1996, J. Biol. Chem. 271(26):15682-15686; and Turner et al., 1997, JIMM 205, 42-54 (see Table 1 for representative examples).

**Table 1. Flexible linker sequences**

| **SEQ ID NO:** | **SEQUENCE** |
|---|---|
| 1 | SGGGGSE |
| 2 | DKTHTS |
| 3 | (S)GGGGS |
| 45 | (S)GGGGSGGGGS |
| 46 | (S)GGGGSGGGGSGGGGS |
| 47 | (S)GGGGSGGGGSGGGGSGGGGS |
| 48 | (S)GGGGSGGGGSGGGGSGGGGSGGGGS |
| 4 | AAAGSG-GASAS |
| 5 | AAAGSG-XGGGS-GASAS |
| 49 | AAAGSG-XGGGSXGGGS -GASAS |
| 50 | AAAGSG- XGGGSXGGGSXGGGS -GASAS |
| 51 | AAAGSG- XGGGSXGGGSXGGGSXGGGS-GASAS |
| 6 | AAAGSG-XS-GASAS |
| 7 | PGGNRGTTTTRRPATTTGSSPGPTQSHY |
| 8 | ATTTGSSPGPT |
| 9 | ATTTGS |
| - | GS |
| 10 | EPSGPISTINSPPSKESHKSP |
| 11 | GTVAAPSVFIFPPSD |
| 12 | GGGGIAPSMVGGGGS |
| 13 | GGGGKVEGAGGGGGS |
| 14 | GGGGSMKSHDGGGGS |
| 15 | GGGGNLITIVGGGGS |
| 16 | GGGGVVPSLPGGGGS |
| 17 | GGEKSIPGGGGS |
| 18 | RPLSYRPPFPFGFPSVRP |
| 19 | YPRSIYIRRRHPSPSLTT |
| 20 | TPSHLSHILPSFGLPTFN |
| 21 | RPVSPFTFPRLSNSWLP A |
| 22 | SPAAHFPRSIPRPGPIRT |
| 23 | APGPSAPSHRSLPSRAFG |
| 24 | PRNSIHFLHPLLVAPLGA |
| 25 | MPSLSGVLQVRYLSPPDL |
| 26 | SPQYPSPLTLTLPPHPSL |
| 27 | NPSLNPPSYLHRAPSRIS |
| 28 | LPWRTSLLPSLPLRRRP |
| 29 | PPLFAKGPVGLLSRSFPP |
| 30 | VPPAPVVSLRSAHARPPY |
| 31 | LRPTPPRVRSYTCCPTP- |
| 32 | PNVAHVLPLLTVPWDNLR |
| 33 | CNPLLPLCARSPAVRTFP |

| | |
|---|---|
| (S) is optional in sequence 3 and 45 to 48. | |

Hence in one embodiment the linker has the sequence GGGGSGGGGS (SEQ ID NO:224. In one embodiment the linker has the sequence GGGGSGGGGSGGGGS (SEQ ID NO:225)

Examples of rigid linkers include the peptide sequences GAPAPAAPAPA (SEQ ID NO:34), PPPP (SEQ ID NO:35) and PPP.

Other linkers include ASTKGP (SEQ ID NO: 228), TVAAP (SEQ ID NO:229)

In one embodiment the peptide linker is an albumin binding peptide. Examples of albumin binding peptides are provided in WO 2007/106120 and include:

**Table 2**

| **SEQ ID NO:** | **SEQUENCE** |
|---|---|
| 208 | DLCLRDWGCLW |
| 209 | DICLPRWGCLW |
| 210 | MEDICLPRWGCLWGD |
| 211 | QRLMEDICLPRWGCLWEDDE |
| 212 | QGLIGDICLPRWGCLWGRSV |
| 213 | QGLIGDICLPRWGCLWGRSVK |
| 214 | EDICLPRWGCLWEDD |
| 215 | RLMEDICLPRWGCLWEDD |
| 216 | MEDICLPRWGCLWEDD |
| 217 | MEDICLPRWGCLWED |
| 218 | RLMEDICLARWGCLWEDD |
| 219 | EVRSFCTRWPAEKSCKPLRG |
| 220 | RAPESFVCYWETICFERSEQ |
| 221 | EMCYFPGICWM |

In one example the molecules of the present invention comprises an albumin binding peptide in a location in addition to or as an alternative to an albumin binding peptide linker. *In vivo* the peptide binds albumin, which increases the half-life of the molecule.

The albumin binding peptide may be appended from one or more variable regions (for example in the Fab and/or in the domain antibody/antibodies), a hinge, a linker, the N-terminal or C-terminal of the molecule, or a combination of the same, or any location that does not interfere with the molecules antigen binding properties.

In one example, an antibody hinge sequence or part thereof is used as a linker, e.g. the upper hinge sequence. Typically, antibody Fab' fragments for use in the present invention possess a native or a modified hinge region. Such hinge regions are used as a natural linker to the dAb moiety. The native hinge region is the hinge region normally associated with the C_{H}1 domain of the antibody molecule. A modified hinge region is any hinge that differs in length and/or composition from the native hinge region. Such hinges can include hinge regions from any other species, such as human, mouse, rat, rabbit, hamster, camel, llama or goat hinge regions. Other modified hinge regions may comprise a complete hinge region derived from an antibody of a different class or subclass from that of the C_{H}1 domain. Thus, for instance, a C_{H}1 domain of class γ1 may be attached to a hinge region of class γ4. Alternatively, the modified hinge region may comprise part of a natural hinge or a repeating unit in which each unit in the repeat is derived from a natural hinge region. In a further alternative, the natural hinge region may be altered by converting one or more cysteine or other residues into neutral residues, such as alanine, or by converting suitably placed residues into cysteine residues. By such means the number of cysteine residues in the hinge region may be increased or decreased. In addition, other characteristics of the hinge can be controlled, such as the distance of the hinge cysteine(s) from the light chain interchain cysteine, the distance between the cysteines of the hinge and the composition of other amino acids in the hinge that may affect properties of the hinge such as flexibility e.g. glycines may be incorporated into the hinge to increase rotational flexibility or prolines may be incorporated to reduce flexibility. Alternatively, combinations of charged or hydrophobic residues may be incorporated into the hinge to confer multimerisation properties, see for example, Richter et al., 2001, Prot. Eng. 14(10):775-783 for use of charged or ionic tails, *e*.*g*., acidic tails as linkers and Kostelny et al., 1992, J. Immunol. 5(1):1547-1553 for leucine zipper sequences. Other modified hinge regions may be entirely synthetic and may be designed to possess desired properties such as length, composition and flexibility.

A number of modified hinge regions have already been described for example, in US5,677,425, US6642356, WO9915549, WO2005003170, WO2005003169, WO2005003170, WO9825971 and WO2005003171 Such hinges generally follow on from the CH1 region, but may also be incorporated onto the end of constant region of a light chain kappa or lambda fragment; see Table 3 for examples.

**Table 3. Hinge linker sequences**

| **SEQ ID NO:** | **SEQUENCE** |
|---|---|
| 36 | DKTHTCAA |
| 37 | DKTHTCPPCPA |
| 38 | DKTHTCPPCPATCPPCPA |
| 39 | DKTHTCPPCPATCPPCPATCPPCPA |
| 40 | DKTHTCPPCPAGKPTLYNSLVMSDTAGTCY |
| 41 | DKTHTCPPCPAGKPTHVNVSVVMAEVDGTCY |
| 42 | DKTHTCCVECPPCPA |
| 43 | DKTHTCPRCPEPKSCDTPPPCPRCPA |
| 44 | DKTHTCPSCPA |

Single variable domains also known as single domain antibodies or dAbs for use in the present invention can be generated using methods known in the art and include those disclosed in WO2005/118642, Ward et al., 1989, Nature, 341, 544-546 and Holt et al., 2003, Trends in Biotechnology, 21, 484-490. Each light chain domain may be either of the kappa or lambda subgroup. Methods for isolating VH and VL domains have been described in the art, see for example EP0368684 and Ward *et al*., *supra.* Such domains may be derived from any suitable species or antibody starting material. The single domain antibody may be derived from a rodent, a human or other species. In one example the single domain antibody is humanised.

In one example the single domain antibody is derived from a phage display library, using the methods described in for example, WO2005/118642, Jespers et al., 2004, Nature Biotechnology, 22, 1161-1165 and Holt et al., 2003, Trends in Biotechnology, 21, 484-490. Preferably, such single domain antibodies are fully human but may also be derived from other species. In one example the single variable domain is chimeric in that the framework is human or substantially human in origin and the CDR(s) is/are of non-human origin. It will be appreciated that the sequence of the single domain antibody once isolated may be modified to improve the characteristics of the single domain antibody, for example solubility, as described in Holt *et al*., *supra.*

Substantially human as employed herein is intended to refer that the human character of the original material is retained, which may be relevant to immunogenicity. Substantially human material would include wherein one amino acid in the framework sequence is added deleted or replaced by another amino acid.

In one example the dAb is a human sequence obtained from scFv phage-display or from a transgenic Humouse™ or Velocimouse™ or a humanised rodent.

In one example, the dAb is obtained from a human or humanised rodent, a camelid or a shark. Such a dAb will preferably be humanised. In one example the single domain antibody is a VHH domain based on camelid immunoglobulins as described in EP0656946. In one example, a camel or a llama is immunised with an antigen of interest and blood collected when the titre is appropriate. The gene encoding the dAb may be cloned by single cell PCR, or the B cell(s) encoding the dAb may be immortalised by EBV transformation, or by fusion to an immortal cell line.

As described herein above, the present disclosure provides dual specificity antibody fusion proteins comprising an antibody Fab or Fab' fragment with specificity for an antigen of interest, said fragment being fused to at least one single domain antibody, directly or via a linker, which has specificity for a second antigen of interest.

The antibody Fab or Fab' fragment is fused at the C-terminus of the heavy or light chain to a dAb directly or via a linker. In another aspect the heavy and light chains of the antibody Fab or Fab' fragment are each fused at the C-terminus to a dAb directly or via a linker. The linkage can be a chemical conjugation but is most preferably a translation fusion, *i*.*e*. a genetic fusion where the sequence of each is encoded in sequence by an expression vector.

Typically the N-terminus of the single domain antibody will be fused to the C-terminus of the heavy or light chain of the Fab or Fab' fragment, directly or via a linker, and where the single domain antibody is fused to the N-terminus of the Fab or Fab' it will be fused via its C-terminus, optionally via a linker.

In one example the description provides a dual specificity antibody fusion protein comprising or consisting of an antibody Fab or Fab' fragment with specificity for an antigen of interest, said fragment being fused to a single domain antibody at the C-terminus of the heavy or light chain which has specificity for a second antigen of interest.

The present disclosure provides a dual specificity antibody fusion protein comprising or consisting of an antibody Fab or Fab' fragment with specificity for an antigen of interest, said fragment being fused to at least one single domain antibody at the C-terminus of the heavy or light chain which has specificity for a second antigen of interest.

In one aspect the present disclosure
provides a dual specificity antibody fusion protein comprising or consisting of an antibody Fab or Fab' fragment with specificity for an antigen of interest, said fragment being fused to two single domain antibodies wherein one single domain antibody is fused to the C-terminus of the light chain of the Fab or Fab' fragment and the other single domain antibody is fused to the C-terminus of the heavy chain of the Fab or Fab' fragment, said single domain antibodies together having specificity for a second antigen of interest.

In one aspect where the heavy and light chains of the Fab or Fab' fragment each comprise a single domain antibody at the C-terminus the two single domain antibodies are identical i.e. have the same binding specificity for the same antigen. In one example, they bind the same epitope on the same antigen. The present disclosure includes where the single domain antibodies may both be the same VH dAb, the same VHH dAb or the same VL dAb.

The heavy and light chains of the Fab or Fab' fragment each comprise a single domain antibody at the C-terminus the two single domain antibodies are a complementary VH/VL pair which bind the antigen co-operatively i.e. they are a complementary VH/VL pair which have the same binding specificity. In one example the VH/VL pair are monospecific. Typically, they will be a VH/VL pair derived from the same antibody. In one example the VH/VL pair are a pair of variable domains isolated as a pair from a 'library of pairs', such as a Fab phage display library.

The dual specificity antibody fusion protein of the present invention comprises two single domain antibodies which are a complementary VH/VL pair, the VH single domain antibody is fused to the C-terminus of the heavy chain constant region (CH1) and the VL single domain antibody is fused to the C-terminus of the light chain constant region (C kappa or C lambda). The disclosure also includes, where the dual specificity antibody fusion protein of the present invention comprises two single domain antibodies which are a complementary VH/VL pair, the VL single domain antibody is fused to the C-terminus of the heavy chain constant region (CH1) and the VH single domain antibody is fused to the C-terminus of the light chain constant region (C kappa or C lambda).

The dual specificity antibody fusion protein of the present invention comprises two single domain antibodies which are linked by one or more disulfide bonds as defined in the claims, two single domain antibodies which are a complementary VH/VL pair linked by one or more (such as 1 or 2) disulfide bonds, such that the VH single domain antibody is fused to the C-terminus of the heavy chain constant region (CH1) and the VL single domain antibody is fused to the C-terminus of the light chain constant region (C kappa or C lambda), characterised by the presence of a disulfide bond between said VH/VL pair. Alternatively the VL single domain antibody may be fused to the C-terminus of the heavy chain constant region (CH1) and the VH single domain antibody may be fused to the C-terminus of the light chain constant region (C kappa or C lambda), characterised by the presence of a disulfide bond between said VH/VL pair.

In one embodiment the VH single domain antibody is fused to the N-terminus of the heavy chain and the VL single domain antibody is fused to the N-terminus of the light chain, characterised by the presence of a disulfide bond between said VH/VL pair. Alternatively, the VL single domain antibody may be fused to the N-terminus of the heavy chain and the VH single domain antibody may be fused to the N-terminus of the light chain, characterised by the presence of a disulfide bond between said VH/VL pair.

The present invention provides a multivalent antibody fusion protein which comprises a Fab or Fab' fragment, with a first specificity for an antigen of interest, and further comprises a VH/VL pair with specificity for a second antigen of interest, wherein the VH/VL pair are linked by a disulfide bond between two cysteine residues, one in VH and one in VL, as defined in the claims.

The disulfide bond is thought to provide additional stabilisation to the construct, which may be advantageous. Preferably the VH/VL pair are linked by a single disulfide bond.

Typically, the VH/VL pair will be linked to each other by a single disulfide bond between two engineered cysteines, one in VH and one in VL.

In the context of the present invention, the variable domain pair (VH/VL) is linked by a disulfide bond between two engineered cysteine residues wherein the engineered cysteine residue of VH is at position 44 and the engineered cysteine residue of VL is at position 100 according to the Kabat numbering.

Suitable positions for introducing engineered cysteines are known in the art, some of which are listed below. It will be appreciated that other suitable positions may exist.

In the present disclosure the disulfide bond may be between (unless the context indicates otherwise Kabat numbering (Kabat et al., 1987, in Sequences of Proteins of Immunological Interest, US Department of Health and Human Services, NIH, USA) is employed in the list below):
- VH37 + VL95C see for example Protein Science 6, 781-788 Zhu et al (1997);
- VH44 + VL100 see for example; Biochemistry 33 5451-5459 Reiter et al (1994); or Journal of Biological Chemistry Vol. 269 No. 28 pp.18327-18331 Reiter et al (1994); or Protein Engineering, vol.10 no.12 pp.1453-1459 Rajagopal et al (1997);
- VH44 + VL105 see for example J Biochem. 118, 825-831 Luo et al (1995);
- VH45 + VL87 see for example Protein Science 6, 781-788 Zhu et al (1997);
- VH55 + VL101 see for example FEBS Letters 377 135-139 Young et al (1995);
- VH100 + VL50 see for example Biochemistry 29 1362-1367 Glockshuber et al (1990);
- VH100b + VL49;
- VH98 + VL 46 see for example Protein Science 6, 781-788 Zhu et al (1997);
- VH101+ VL 46 or
- VH105 + VL43 see for example; Proc. Natl. Acad. Sci. USA Vol. 90 pp.7538-7542 Brinkmann et al (1993); or Proteins 19, 35-47 Jung et al (1994).
- VH106 + VL57 see for example FEBS Letters 377 135-139 Young et al (1995)
The amino acid pairs listed above are in the positions conducive to replacement by cysteines such that disulfide bonds can be formed. Cysteines can be engineered into these positions by known techniques.

In one or more aspects the disulfide bond between the heavy and light chain such as between the CH domain and CL or CK domain is not present, for example because one or more cysteines which form the bond are replaced. Said one or more cysteines may be replaced with, for example serine.

In one or more aspects an interchain disulfide bond between the heavy and light chain between the CH domain and CL or CK domain is present.

In one aspect there is provided a F(ab)₂ fragment comprising one, two, three or four single domain antibodies, for example a two separate VH/VL pairs which may be directed to the same or different antigens.

In one example the antibody fusion proteins of the invention do not comprise an Fc domain. In one example the antibody fusion proteins of the invention do not comprise a CH2 or CH3 domain.

In dual specificity fusion proteins of the present invention the single domain antibody or antibodies bind to a second antigen, different from that bound by the Fab or Fab' fragment component.

In one example the dAbs for use in the present invention exhibit specificity for a complement pathway protein, a CD marker protein or an FcγR. In this case the dAb is preferably specific for a CD molecule. Most preferably, the dAb exhibits specificity for a CD molecule selected from the group consisting of CD68, CD80, CD86, CD64, CD3, CD4, CD8 CD45, CD16 and CD35.

In a preferred example the dAbs for use in the present invention exhibit specificity for a serum carrier protein, a circulating immunoglobulin molecule, or CD35/CR1, the serum carrier protein preferably being a human serum carrier protein such as thyroxine-binding protein, transthyretin, α1-acid glycoprotein, transferrin, fibrinogen or serum albumin. Most preferably, the dAb exhibits specificity for human serum albumin. Thus, in one example, a rabbit, mouse, rat, camel or a llama is immunised with a serum carrier protein, a circulating immunoglobulin molecule, or CD35/CR1 (e.g. human serum albumin) and blood collected when the titre is appropriate. The gene encoding the dAb may be cloned by single cell PCR, or the B cell(s) encoding the dAb may be immortalised by EBV transformation, or by fusion to an immortal cell line. Alternatively, the single domain antibody may be obtained by phage display as described herein above.

In one embodiment the single domain antibody or antibodies for use in the present invention bind human serum albumin. In one embodiment the single domain antibody or antibodies for use in the present invention bind human serum albumin, murine serum albumin and rat serum albumin.

In one embodiment the single domain antibody which binds serum albumin is a dAb provided in WO2005/118642 (see for example figures 1c and 1d) or a VHH provided in WO2004/041862 or a humanised nanobody described in, for example table III of WO2006/122787.

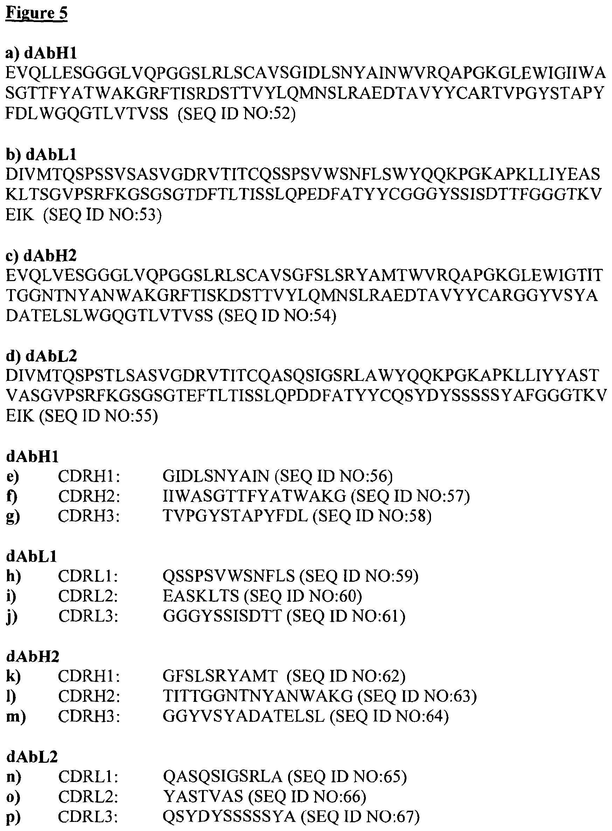

In one embodiment a single domain antibody which binds human serum albumin for use in the present invention is a heavy chain VH single domain antibody which comprises at least one of a CDR having the sequence given in Figure 5 (e) SEQ ID NO:56 or Figure 5 (k) SEQ ID NO:62 for CDR-H1, a CDR having the sequence given in Figure 5(f) SEQ ID NO:57 or Figure 5 (1) SEQ ID NO:63 for CDR-H2 and a CDR having the sequence given in Figure 5 (g) SEQ ID NO:58 or Figure 5 (m) SEQ ID NO:64 for CDR-H3.

In another embodiment a single domain antibody which binds human serum albumin for use in the present invention is a heavy chain VH antibody, wherein at least two of CDR-H1, CDR-H2 and CDR-H3 of the VH domain are selected from the following: the sequence given in SEQ ID NO:56 or SEQ ID NO:62 for CDR-H1, the sequence given in SEQ ID NO:57 or SEQ ID NO:63 for CDR-H2 and the sequence given in SEQ ID NO:58 or SEQ ID NO:64 for CDR-H3. For example, the single domain antibody may comprise a VH domain wherein CDR-H1 has the sequence given in SEQ ID NO:56 and CDR-H2 has the sequence given in SEQ ID NO:57. Alternatively, the single domain antibody may comprise a VH domain wherein CDR-H1 has the sequence given in SEQ ID NO:56 and CDR-H3 has the sequence given in SEQ ID NO:58. For the avoidance of doubt, it is understood that all permutations are included.

In another embodiment a single domain antibody which binds human serum albumin for use in the present invention is a heavy chain VH single domain antibody, wherein the VH domain comprises the sequence given in SEQ ID NO:56 for CDR-H1, the sequence given in SEQ ID NO:57 for CDR-H2 and the sequence given in SEQ ID NO:58 for CDR-H3.

In another embodiment a single domain antibody which binds human serum albumin for use in the present invention is a heavy chain VH single domain antibody, wherein the VH domain comprises the sequence given in SEQ ID NO:62 for CDR-H1, the sequence given in SEQ ID NO:63 for CDR-H2 and the sequence given in SEQ ID NO:64 for CDR-H3.

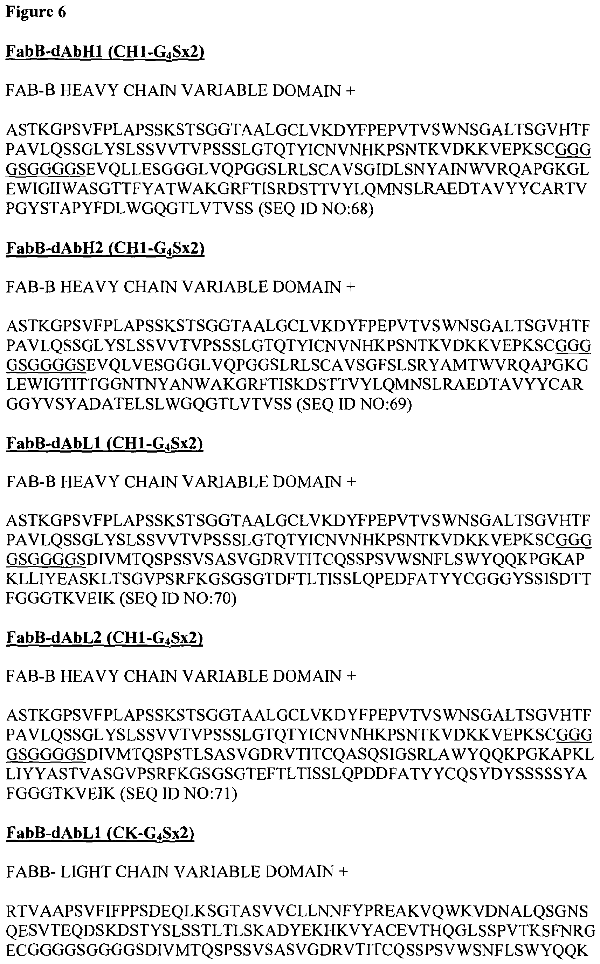

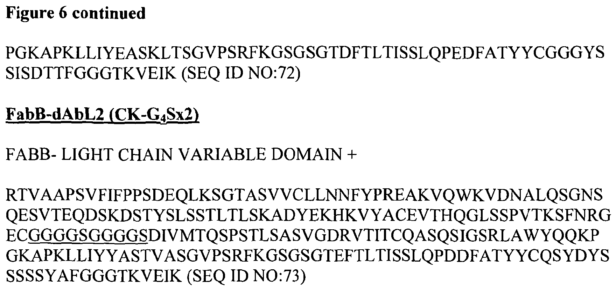

In one embodiment a single domain antibody which binds human serum albumin for use in the present invention is a humanised heavy chain VH single domain antibody, dAbH1, having the sequence given in Figure 5 (a) (SEQ ID NO:52). An example of a suitable CH1-dAbH1 fusion comprising a G₄S linker is given in Figure 6 (SEQ ID NO:68).

In one embodiment the single domain antibody which binds human serum albumin for use in the present invention is a humanised heavy chain VH single domain antibody, dAbH2, having the sequence given in Figure 5 (c) (SEQ ID NO:54). An example of a suitable CH1-dAbH2 fusion comprising a G₄S linker is given in Figure 6 (SEQ ID NO:69).

The residues in antibody variable domains are conventionally numbered according to a system devised by Kabat *et al.* This system is set forth in Kabat et al., 1987, in Sequences of Proteins of Immunological Interest, US Department of Health and Human Services, NIH, USA (hereafter "Kabat *et al.* (*supra*)"). This numbering system is used in the present specification except where otherwise indicated.

The Kabat residue designations do not always correspond directly with the linear numbering of the amino acid residues. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence.

The CDRs of the heavy chain variable domain are located at residues 31-35 (CDR-H1), residues 50-65 (CDR-H2) and residues 95-102 (CDR-H3) according to the Kabat numbering system. However, according to Chothia (Chothia, C. and Lesk, A.M. J. Mol. Biol., 196, 901-917 (1987)), the loop equivalent to CDR-H1 extends from residue 26 to residue 32. Thus 'CDR-H1', as used herein, comprises residues 26 to 35, as described by a combination of the Kabat numbering system and Chothia's topological loop definition.

The CDRs of the light chain variable domain are located at residues 24-34 (CDR-L1), residues 50-56 (CDR-L2) and residues 89-97 (CDR-L3) according to the Kabat numbering system.

In one embodiment a single domain antibody which binds human serum albumin for use in the present invention is a light chain VL single domain antibody which comprises at least one of a CDR having the sequence given in Figure 5 (h) SEQ ID NO:59 or Figure 5 (n) SEQ ID NO:65 for CDR-L1, a CDR having the sequence given in Figure 5(i) SEQ ID NO:60 or Figure 5 (o) SEQ ID NO:66 for CDR-L2 and a CDR having the sequence given in Figure 5 (j) SEQ ID NO:61 or Figure 5 (p) SEQ ID NO:67 for CDR-L3.

In another embodiment a single domain antibody which binds human serum albumin for use in the present invention is a light chain VL antibody, wherein at least two of CDR-L1, CDR-L2 and CDR-L3 of the VL domain are selected from the following: the sequence given in SEQ ID NO:59 or SEQ ID NO:65 for CDR-L1, the sequence given in SEQ ID NO:60 or SEQ ID NO:66 for CDR-L2 and the sequence given in SEQ ID NO:61 or SEQ ID NO:67 for CDR-L3. For example, the domain antibody may comprise a VL domain wherein CDR-L1 has the sequence given in SEQ ID NO:59 and CDR-L2 has the sequence given in SEQ ID NO:60. Alternatively, the domain antibody may comprise a VL domain wherein CDR-L1 has the sequence given in SEQ ID NO:59 and CDR-L3 has the sequence given in SEQ ID NO:61. For the avoidance of doubt, it is understood that all permutations are included.

In another embodiment a single domain antibody which binds human serum albumin for use in the present invention is a light chain VL domain antibody, wherein the VL domain comprises the sequence given in SEQ ID NO:59 for CDR-L1, the sequence given in SEQ ID NO:60 for CDR-L2 and the sequence given in SEQ ID NO:61 for CDR-L3.

In another embodiment a single domain antibody which binds human serum albumin for use in the present invention is a light chain VL domain antibody, wherein the VL domain comprises the sequence given in SEQ ID NO:65 for CDR-L1, the sequence given in SEQ ID NO:66 for CDR-L2 and the sequence given in SEQ ID NO:67 for CDR-L3.

In one embodiment a single domain antibody which binds human serum albumin for use in the present invention is a humanised light chain VL single domain antibody, dAbL1, having the sequence given in Figure 5 (b) (SEQ ID NO:53). An example of a suitable CH1-dAbL1 fusion and a Ck1-dAbL1 fusion both comprising a G₄S linker is given in Figure 6 (SEQ ID NO:70 and SEQ ID NO:72).

In one embodiment a single domain antibody which binds human serum albumin for use in the present invention is a humanised light chain VL single domain antibody, dAbL2, having the sequence given in Figure 5 (d) (SEQ ID NO:55). An example of a suitable CH1-dAbL2 fusion and a Ck1-dAbL2 fusion both comprising a G₄S linker is given in Figure 6 (SEQ ID NO:71 and SEQ ID NO:73).

In one embodiment where the heavy and light chains of the Fab or Fab' fragment each comprise a single domain antibody at the C-terminus and the two single domain antibodies are a complementary VH/VL pair which bind the antigen co-operatively as described herein above, the VH dAb is dAbH1 (SEQ ID NO:52) and the VL dAb is dAbL1 (SEQ ID NO:53).

In one embodiment where the heavy and light chains of the Fab or Fab' fragment each comprise a single domain antibody at the C-terminus the two single domain antibodies are a complementary VH/VL pair which bind the antigen co-operatively as described herein above, the VH dAb is dAbH2 (SEQ ID NO:54) and the VL dAb is dAbL2 (SEQ ID NO:55).

In one embodiment where the heavy and light chains of the Fab or Fab' fragment each comprise a single domain antibody at the C-terminus and the two single domain antibodies are a complementary VH/VL pair which bind the antigen co-operatively as described herein above, the VH dAb has the sequence given in SEQ ID NO:202 and the VL dAb has the sequence given in SEQ ID NO:203.

In one embodiment where the heavy and light chains of the Fab or Fab' fragment each comprise a single domain antibody at the C-terminus the two single domain antibodies are a complementary VH/VL pair which bind the antigen co-operatively as described herein above, the VH dAb has the sequence given in SEQ ID NO:204 and the VL dAb has the sequence given in SEQ ID NO:205.

In one embodiment where the heavy and light chains of the Fab or Fab' fragment each comprise a single domain antibody at the N-terminus and the two single domain antibodies are a complementary VH/VL pair which bind the antigen co-operatively as described herein above, the VH dAb has the sequence given in SEQ ID NO:202 and the VL dAb has the sequence given in SEQ ID NO:203.

In one embodiment where the heavy and light chains of the Fab or Fab' fragment each comprise a single domain antibody at the N-terminus the two single domain antibodies are a complementary VH/VL pair which bind the antigen co-operatively as described herein above, the VH dAb has the sequence given in SEQ ID NO:204 and the VL dAb has the sequence given in SEQ ID NO:205.

In another aspect, the present disclosure provides albumin binding antibodies or fragments thereof containing one or more of the CDRs provided herein above and in Figure 5 (e-p), in particular comprising a CDRH1 with the sequence shown in SED ID NO. 56, a CDRH2 with the sequence shown in SEQ ID NO. 57, a CDRH3 with the sequence shown in SED ID NO. 58, a CDRL1 with the sequence shown in SEQ ID NO. 59, a CDRL2 with the sequence shown in SEQ ID NO. 60, and/or a CDRL3 with the sequence shown in SEQ ID NO. 61. In one example the albumin binding antibodies or fragments comprise a CDRH1 with the sequence shown in SEQ ID NO. 62, a CDRH2 with the sequence shown in SEQ ID NO. 63, a CDRH3 with the sequence shown in SEQ ID NO. 64, a CDRL1 with the sequence shown in SEQ ID NO. 65, a CDRL2 with the sequence shown in SEQ ID NO. 66, and/or a CDRL3 with the sequence shown in SEQ ID NO. 67. Said CDRs may be incorporated into any suitable antibody framework and into any suitable antibody format. Such antibodies include whole antibodies and functionally active fragments or derivatives thereof which may be, but are not limited to, monoclonal, humanised, fully human or chimeric antibodies. Accordingly, such albumin binding antibodies may comprise a complete antibody molecule having full length heavy and light chains or a fragment thereof and may be, but are not limited to Fab, modified Fab, Fab', F(ab')₂, Fv, single domain antibodies, scFv, bi, tri or tetra-valent antibodies, Bis-scFv, diabodies, triabodies, tribodies, tetrabodies and epitope-binding fragments of any of the above (see for example Holliger and Hudson, 2005, Nature Biotech. 23(9):1126-1136; Adair and Lawson, 2005, Drug Design Reviews - Online 2(3), 209-217). The methods for creating and manufacturing these antibody fragments are well known in the art (see for example Verma et al., 1998, Journal of Immunological Methods, 216, 165-181). Multi-valent antibodies may comprise multiple specificities or may be monospecific (see for example WO92/22853 and WO05/113605). It will be appreciated that this aspect also extends to variants of these albumin binding antibodies.

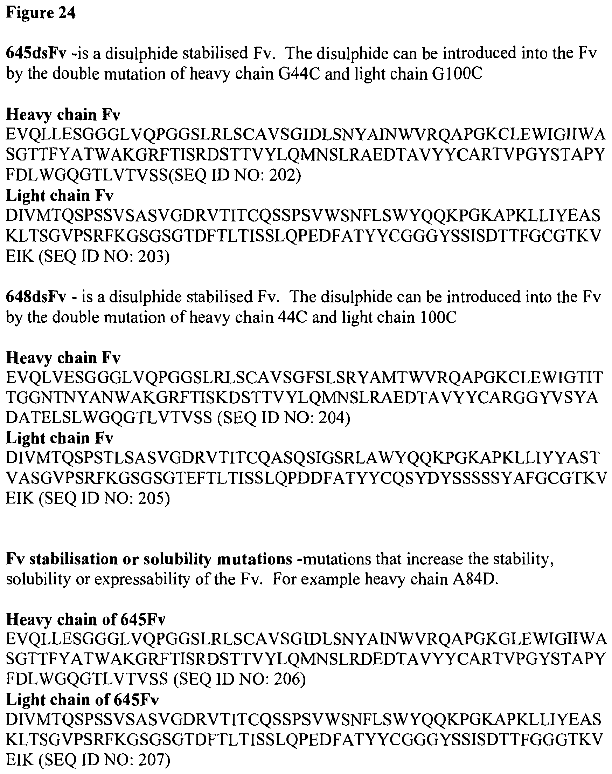

It will be appreciated that such albumin binding antibodies, in particular single domain antibodies may be conjugated to any other antibody or protein or other molecule, as desired or used in any other suitable context. In one example the single domain antibodies dAbH1, dAbL1, dAbH2, dAbL2 as described above and shown in Figure 5 (a-d) and Figure 24 may be incorporated into any suitable antibody format or used as single domain antibodies in any suitable context, such as a fusion or conjugate.

In one example antibodies of this aspect of the disclosure comprise the sequence given in Figure 5(e) for CDR-H1, the sequence given in Figure 5(f) for CDR-H2 and the sequence given in Figure 5(g) for CDR-H3.

In one example
antibodies of this aspect of the disclosure comprise the sequence given in Figure 5(k) for CDR-H1, the sequence given in Figure 5(l) for CDR-H2 and the sequence given in Figure 5(m) for CDR-H3.

In one example antibodies of this aspect of the disclosure comprise the sequence given in Figure 5(h) for CDR-L1, the sequence given in Figure 5(i) for CDR-L2 and the sequence given in Figure 5(j) for CDR-L3.

In one example antibodies of this aspect of the disclosure comprise the sequence given in Figure 5(n) for CDR-L1, the sequence given in Figure 5(o) for CDR-L2 and the sequence given in Figure 5(p) for CDR-L3.

The disclosure also provides an Fv or scFv comprising a VH domain and/or a VL domain having a sequence given in Figure 5(a) to (d) or Figure 24. In one aspect the Fv or scFv comprises a VH having the sequence given in SEQ ID NO:202 and a VH having the sequence given in SEQ ID NO:203. In one aspect the Fv or scFv comprises a VH having the sequence given in SEQ ID NO:204 and a VL having the sequence given in SEQ ID NO:205.

The VH and VL of the scFv, for example are in the VHVL orientation (N to C-terminus). In one aspect the VH and VL are in the VLVH orientation (N to C-terminus).

As described above, the scFv or Fv fragments may be further incorporated into any suitable antibody format. For example, they may fused or conjugated to one or more other antibody fragments.

In the antibody formats below each of the sequences from the sequence listing herein may be located in the position corresponding to the natural position or a non-natural position. Natural position will be for the relevant sequence in the listing labelled CDRH1 position H1, for the relevant sequence in the listing labelled CDRH2 position H2, for the relevant sequence in the listing labelled CDRH3 position H3, for the relevant sequence in the listing labelled CDRL1 position L1, for the relevant sequence in the listing labelled CDRL2 position L2, and for the relevant sequence in the listing labelled CDRL3 position L3. Combinations thereof are also envisaged such as H1 and H2, H1 and H3, H1 and L1, H1 and L2, H1 and L3, H2 and L1, H2 and L2, H2 and L3, H2 and H3, H3 and L1, H3 and L2, H3 and L3, H1 and H2 and H3, H1 and H2 and L1, H1 and H2 and L2, H1 and H2 and L3, H2 and H3 and L1, H2 and H3 and L2, H2 and H3 and L3, H3 and L1 and L2, H3 and L1 and L3, H3 and L2 and L3, L1 and L2 and L3, H1 and H2 and H3 and L1, H1 and H2 and H3 and L2, H1 and H2 and H3 and L3, H2 and H3 and L1 and L2, H2 and H3 and L1 and L3, and H2 and H3 and L2 and L3, H3 and L1 and L2 and L3, H1 and H2 and H3 and L1 and L2, H1 and H2 and H3 and L2 and L3, H1 and H2 and H3 and L1 and L3, L1 and L2 and L3 and H1 and H2, L1 and L2 and L3 and H1 and H3, L1 and L2 and L3 and H2 and H3, H1 and H2 and H3 and L1 and L2 and L3.

In one aspect the antibody fusion protein of the disclosure comprises a sequence, for example 1, 2, 3, 4, 5 or 6 sequence(s) selected from Sequence ID NO: 222, 223, 90 to 93.

In one aspect the antibody fusion protein of the disclosure comprises a sequence, for example 1, 2, 3, 4, 5 or 6 sequence(s) selected from Sequence ID NO: 94 to 99.

In one aspect the antibody fusion protein of the disclosure comprises a sequence, for example 1, 2, 3, 4, 5 or 6 sequence(s) selected from Sequence ID NO: 100 to 105.

In one aspect the antibody fusion protein of the disclosure comprises a sequence, for example 1, 2, 3, 4, 5 or 6 sequence(s) selected from Sequence ID NO: 106 to 111.

In one aspect the antibody fusion protein of the disclosure comprises a sequence, for example 1, 2, 3, 4, 5 or 6 sequence(s) selected from Sequence ID NO: 112 to 117.

In one aspect the antibody fusion protein of the disclosure comprises a sequence, for example 1, 2, 3, 4, 5 or 6 sequence(s) selected from Sequence ID NO: 118 to 123.

In one aspect the antibody fusion protein of the disclosure comprises a sequence, for example 1, 2, 3, 4, 5 or 6 sequence(s) selected from Sequence ID NO: 124 to 129.

In one aspect the antibody fusion protein of the disclosure comprises a sequence, for example 1, 2, 3, 4, 5 or 6 sequence(s) selected from Sequence ID NO: 130 to 135.

In one aspect the antibody fusion protein of the disclosure comprises a sequence, for example 1, 2, 3, 4, 5 or 6 sequence(s) selected from Sequence ID NO: 136 to 141.

In one aspect the antibody fusion protein of the disclosure comprises a sequence, for example 1, 2, 3, 4, 5 or 6 sequence(s) selected from Sequence ID NO:

In one aspect the antibody fusion protein of the disclosure comprises a sequence, for example 1, 2, 3, 4, 5 or 6 sequence(s) selected from Sequence ID NO: 148 to 153.

In one aspect the antibody fusion protein of the disclosure comprises a sequence, for example 1, 2, 3, 4, 5 or 6 sequence(s) selected from Sequence ID NO: 154 to 159.

In one aspect the antibody fusion protein of the disclosure comprises a sequence, for example 1, 2, 3, 4, 5 or 6 sequence(s) selected from Sequence ID NO: 160 to 165.

In one aspect the antibody fusion protein of the disclosure comprises a sequence, for example 1, 2, 3, 4, 5 or 6 sequence(s) selected from Sequence ID NO: 166 to 171.

In one aspect the antibody fusion protein of the disclosure comprises a sequence, for example 1, 2, 3, 4, 5 or 6 sequence(s) selected from Sequence ID NO: 172 to 177.

In one aspect the antibody fusion protein of the disclosure comprises a sequence, for example 1, 2, 3, 4, 5 or 6 sequence(s) selected from Sequence ID NO: 178 to 183.

In one aspect the antibody fusion protein of the disclosure comprises a sequence, for example 1, 2, 3, 4, 5 or 6 sequence(s) selected from Sequence ID NO: 184 to 189.

In one aspect the antibody fusion protein of the disclosure comprises a sequence, for example 1, 2, 3, 4, 5 or 6 sequence(s) selected from Sequence ID NO: 190 to 195.

In one aspect the antibody fusion protein of the disclosure comprises a sequence, for example 1, 2, 3, 4, 5 or 6 sequence(s) selected from Sequence ID NO: 196 to 201.

In one aspect the antibody fusion protein of the disclosure comprises Sequence ID No: 202.

In one aspect the antibody fusion protein of the disclosure comprises Sequence ID No: 203.

In one aspect the antibody fusion protein of the disclosure comprises Sequence ID Nos: 202 and 203.

In one aspect the antibody fusion protein of the disclosure comprises Sequence ID No: 204.

In one aspect the antibody fusion protein of the disclosure comprises Sequence ID No: 205.

In one aspect the antibody fusion protein of the disclosure comprises Sequence ID Nos: 204 and 205.

In one aspect the antibody fusion protein of the disclosure comprises Sequence ID No: 206.

In one aspect the antibody fusion protein of the disclosure comprises Sequence ID No: 207.

In one aspect the antibody fusion protein of the disclosure comprises Sequence ID No: 206 and 207.

In one aspect the antibody fusion protein of the disclosure comprises the sequence given in SEQ ID NO:202 in which the A at position 84 has been substituted by D.

In one aspect the antibody fusion protein of the disclosure comprises the sequence given in SEQ ID NO:204 in which the A at position 84 has been substituted by D.

Where the single domain antibody or antibodies of the dual specificity fusion protein of the present disclosure bind to albumin the binding affinity of the single domain antibody for albumin will be sufficient to extend the half-life of the Fab or Fab' *in vivo.* It has been reported that an affinity for albumin of less than or equal to 2.5µM affinity will extend half-life *in vivo* (Nguyen, A. et al (2006) Protein Engineering, Design & Selection, 19(7), 291-297). The single domain antibody molecules preferably have a binding affinity suited to their purpose and the antigen to which they bind. In one example the single domain antibodies have a high binding affinity, for example picomolar. In one example the single domain antibodies have a binding affinity for antigen which is nanomolar or micromolar. Affinity may be measured using any suitable method known in the art, including BIAcore as described in the Examples herein using natural or recombinant antigen.

Preferably the single domain antibody molecules which bind albumin have a binding affinity of about 2µM or better. In one aspect the single domain antibody molecule has a binding affinity of about 1µM or better. In one aspect the single domain antibody molecule has a binding affinity of about 500nM or better. In one aspect the single domain antibody molecule has a binding affinity of about 200nM or better. In one aspect the domain antibody molecule has a binding affinity of about 1nM or better. It will be appreciated that the affinity of single domain antibodies for use in the present invention and known in the art may be altered using any suitable method known in the art. The present disclosure therefore also relates to variants of the domain antibody molecules which have an improved affinity for albumin. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E. coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996) and sexual PCR (Crameri et al., Nature, 391, 288-291, 1998). Vaughan *et al.* (*supra*) discusses these methods of affinity maturation.

The single domain antibody or antibodies of the dual specificity fusion protein may be provided as monomers, dimmers or trimers, as required. The desired product may be obtained by adjusting the downstream processing steps the material is subjected to. In one aspect the processed material is provided as a substantially homogenous monomer. In one aspect the processed material is provided a substantially homogenous dimer. In one aspect the processed material is provided as a substantially homogenous trimer.

The present disclosure also provides an isolated DNA sequence encoding a dual specificity antibody fusion protein of the present invention. The DNA sequences may comprise synthetic DNA, for instance produced by chemical processing, cDNA, genomic DNA or any combination thereof.

DNA sequences which encode the dual specificity antibody fusion protein of the present invention can be obtained by methods well known to those skilled in the art. For example, DNA sequences coding for part or all of the antibody fragments, linkers and/or dAbs may be synthesised as desired from the determined DNA sequences or on the basis of the corresponding amino acid sequences.

Standard techniques of molecular biology may be used to prepare DNA sequences coding for the dual specificity antibody fusion protein of the present invention. Desired DNA sequences may be synthesised completely or in part using oligonucleotide synthesis techniques. Site-directed mutagenesis and polymerase chain reaction (PCR) techniques may be used as appropriate.

The present disclosure further relates to a cloning or expression vector comprising one or more DNA sequences. Accordingly, provided is a cloning or expression vector comprising one or more DNA sequences encoding a dual specificity antibody fusion protein of the present invention. In one preferred aspect, the cloning or expression vector comprises a single DNA sequence encoding the entire dual specificity antibody fusion protein. Thus, the cloning or expression vector comprises DNA encoded transcription units in sequence such that a translation fusion protein is produced.

Indeed, it will be understood by those skilled in the art that a fusion protein of the invention can have the dAb at the N-terminus or the C-terminus and thus, the dAb DNA encoded transcription unit will be first or last, respectively, within the DNA sequence encoding the translation fusion. Thus, a translation fusion may comprise an N-terminal dAb and a C-terminal Fab or Fab'. Further, a translation fusion may comprise an N-terminal Fab or Fab' and a C-terminal dAb.

It will be appreciated that the heavy chain and light chain of the Fab or Fab' may be incorporated into the same or different vectors. In one aspect one vector may comprise a translation fusion comprising a Fab or Fab' heavy chain and a C-terminal dAb and another vector may comprise a translation fusion comprising a Fab or Fab' light chain and a C-terminal dAb.

For example, where the desire is to produce a dual specificity antibody fusion protein with the dAb moiety at the N-terminal end of the antibody fragment, the vector will comprise DNA transcription units in sequence order; a DNA transcription unit encoding the dAb moiety, optionally a DNA transcription unit encoding a linker sequence, and a DNA transcription unit encoding an antibody fragment. Where the desire is to produce a dual specificity antibody fusion protein with the dAb moiety at the C-terminal end of the antibody fragment, the vector will comprise DNA transcription units in sequence order; a DNA transcription unit encoding an antibody fragment, optionally a DNA transcription unit encoding a linker sequence, and a DNA transcription unit encoding dAb moiety with specificity for a serum carrier protein, a circulating immunoglobulin molecule, or CD35/CR1, for example, human serum albumin. Thus, a translation fusion can be in different configurations including, for example but without limitation, dAb-linker-Fab, Fab-linker-dAb, dAb-Fab, Fab-dAb, Fab'-dAb, dAb-Fab', dAb-linker Fab', Fab'-linker-dAb. Where two vectors are used for example, the first may comprise the heavy chain of a Fab or Fab' fused to a dAb and the second may comprise the light chain of a Fab or Fab' fused to a dAb.

DNA code for an antibody fragment comprised within a translation fusion can be incorporated into a vector as a transcription unit in configurations as known to the person skilled in the art, for example a transcription unit can comprise code for the light chain followed by the heavy chain code, or vice versa; see, in particular, Humphreys et al., 2002, Protein Expression and Purification, 26:309-320.

Preferably, a vector comprises an appropriate leader sequence, such as an antibody leader sequence. Such leader sequences are well known in the art.

General methods by which the vectors may be constructed, transfection and transformation methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

Also provided is a host cell comprising one or more cloning or expression vectors comprising one or more DNA sequences encoding a dual specificity antibody fusion protein of the present invention. Any suitable host cell/vector system may be used for expression of the DNA sequences encoding the dual specificity antibody fusion protein. Bacterial, for example *E. coli,* and other microbial systems may be used or eukaryotic, for example mammalian, host cell expression systems may also be used. Suitable mammalian host cells include NS0, CHO, myeloma or hybridoma cells. Accordingly in one example the fusion protein of the present invention is expressed in *E.coli.* In another example the fusion protein of the present invention is expressed in mammalian cells.

The present disclosure also provides a process for the production of a dual specificity antibody fusion protein comprising culturing a host cell comprising a vector of the present disclosure under conditions suitable for the expression of protein from the DNA sequence encoding said dual specificity antibody fusion protein. The disclosure further provides methods for isolating the dual specificity antibody fusion protein.

On production, a dual specificity antibody fusion protein of the present invention may be purified, where necessary, using any suitable method known in the art. For example, but without limitation, chromatographic techniques such as ion exchange, size exclusion, protein G or hydrophobic interaction chromatography may be used.

The size of a dual specificity antibody fusion protein may be confirmed by conventional methods known in the art such as size exclusion chromatography and non-reducing SDS-PAGE. Such techniques can be used, for example to confirm that the protein has not dimerised and/or does not have a portion missing, e.g. the dAb portion. If dimers are detected and a homogenous monomeric product is required then the monomeric dual specificity antibody fusion protein may be purified away from the dimeric species using conventional chromatography techniques as described above.

Dual specificity antibody fusion proteins of the invention are useful in the treatment of diseases or disorders including inflammatory diseases and disorders, immune disease and disorders, fibrotic disorders and cancers.

The term "inflammatory disease" or "disorder" and "immune disease or disorder" includes rheumatoid arthritis, psoriatic arthritis, still's disease, Muckle Wells disease, psoriasis, Crohn's disease, ulcerative colitis, SLE (Systemic Lupus Erythematosus), asthma, allergic rhinitis, atopic dermatitis, multiple sclerosis, vasculitis, Type I diabetes mellitus, transplantation and graft-versus-host disease.

The term "fibrotic disorder" includes idiopathic pulmonary fibrosis (IPF), systemic sclerosis (or scleroderma), kidney fibrosis, diabetic nephropathy, IgA nephropathy, hypertension, end-stage renal disease, peritoneal fibrosis (continuous ambulatory peritoneal dialysis), liver cirrhosis, age-related macular degeneration (ARMD), retinopathy, cardiac reactive fibrosis, scarring, keloids, burns, skin ulcers, angioplasty, coronary bypass surgery, arthroplasty and cataract surgery.

The term "cancer" includes a malignant new growth that arises from epithelium, found in skin or, more commonly, the lining of body organs, for example: breast, ovary, prostate, lung, kidney, pancreas, stomach, bladder or bowel. Cancers tend to infiltrate into adjacent tissue and spread (metastasise) to distant organs, for example: to bone, liver, lung or the brain.

Thus, according to a further aspect of the disclosure, there is provided a pharmaceutical composition which comprises an antibody fusion of the invention in association with one or more pharmaceutically acceptable carriers, excipients or diluents. Also disclosed is the use of an antibody fusion protein of the invention for the manufacture of a medicament for the treatment of a disease or disorder. Most preferably, the disease or disorder is an inflammatory disease or disorder.

Pharmaceutical compositions may take a form suitable for oral, buccal, parenteral, subcutaneous, nasal, topical, ophthalmic or rectal administration, or a form suitable for administration by inhalation or insufflation.

Where appropriate, for example if the single domain antibody or antibodies of the antibody fusion protein bind to albumin, it may be desirable to pre-formulate the dual specificity fusion protein with human or recombinant serum albumin, using any suitable method known in the art.

Where the pharmaceutical formulation is a liquid, for example a solution or suspension then the formulation may further comprise albumin, for example human serum albumin, in particular recombinant albumin such as recombinant human serum albumin. Suitable amounts may be in the range of less than 2% w/w of the total formulation, in particular less than 1, 0.5, or 0.1% w/w. This may assist in stabilizing the antibody component in the formulation. The pharmaceutical composition may be lyophilized for reconstitution later, with an aqueous solvent.

In one aspect there is provided a unit dose container, such as a vial, comprising a lyophilized "antibody" according to the invention.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methyl cellulose); fillers (e.g. lactose, microcrystalline cellulose or calcium hydrogenphosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles or preservatives. The preparations may also contain buffer salts, flavouring agents, colouring agents or sweetening agents, as appropriate.

Preparations for oral administration may be suitably formulated to give controlled release of the active compound.

For buccal administration, the compositions may take the form of tablets or lozenges formulated in conventional manner.

The bispecific antibodies of the invention may be formulated for parenteral administration by injection, e.g. by bolus injection or infusion. Formulations for injection may be presented in unit dosage form, e.g. in glass ampoules or multi-dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the bispecific antibodies of the invention may also be formulated as a depot preparation. Such long-acting formulations may be administered by implantation or by intramuscular injection.

For nasal administration or administration by inhalation, the compounds according to the present invention may be conveniently delivered in the form of an aerosol spray presentation for pressurised packs or a nebuliser, with the use of a suitable propellant, e.g. dichlorodifluoromethane, fluorotrichloromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

For topical administration the compounds according to the present invention may be conveniently formulated in a suitable ointment containing the active component suspended or dissolved in one or more pharmaceutically acceptable carriers. Particular carriers include, for example, mineral oil, liquid petroleum, propylene glycol, polyoxyethylene, polyoxypropylene, emulsifying wax and water. Alternatively, the compounds according to the present invention may be formulated in a suitable lotion containing the active component suspended or dissolved in one or more pharmaceutically acceptable carriers. Particular carriers include, for example, mineral oil, sorbitan monostearate, polysorbate 60, cetyl esters wax, cetearyl alcohol, benzyl alcohol, 2-octyldodecanol and water.

In one example the formulation is provided as a formulation for topical administrations including inhalation.

Suitable inhalable preparations include inhalable powders, metering aerosols containing propellant gases or inhalable solutions free from propellant gases. Inhalable powders according to the disclosure containing the active substance may consist solely of the abovementioned active substances or of a mixture of the abovementioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 µm, in particular from 1 to 5 µm. The particle size of the active ingredient (such as the antibody or fragment) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols may contain up to 5 % by weight of active substance. Aerosols contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient.

Alternatively, topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

The antibody formats of the invention can be delivered dispersed in a solvent, e.g., in the form of a solution or a suspension. It can be suspended in an appropriate physiological solution, e.g., saline or other pharmacologically acceptable solvent or a buffered solution. Buffered solutions known in the art may contain 0.05 mg to 0.15 mg disodium edetate, 8.0 mg to 9.0 mg NaCl, 0.15 mg to 0.25 mg polysorbate, 0.25 mg to 0.30 mg anhydrous citric acid, and 0.45 mg to 0.55 mg sodium citrate per 1 ml of water so as to achieve a pH of about 4.0 to 5.0. A suspension can employ, for example, lyophilised antibody.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent/solution used for the for the formulation, aseptic suspension of the antibody in the sterile buffered solvent solution, and dispensing of the formulation into sterile receptacles by methods familiar to those of ordinary skill in the art.

Nebulizable formulation according to the present disclosure may be provided, for example, as single dose units (e.g., sealed plastic containers or vials) packed in foil envelopes. Each vial contains a unit dose in a volume, e.g., 2 ml, of solvent/solution buffer.

The antibodies formats of the present disclosure are thought to be suitable for delivery via nebulisation.

For ophthalmic administration the compounds according to the present invention may be conveniently formulated as microionized suspensions in isotonic, pH-adjusted sterile saline, either with or without a preservative such as a bactericidal or fungicidal agent, for example phenylmercuric nitrate, benzylalkonium chloride or chlorhexidine acetate. Alternatively, for ophthalmic administration compounds may be formulated in an ointment such as petrolatum.

For rectal administration the compounds according to the present invention may be conveniently formulated as suppositories. These can be prepared by mixing the active component with a suitable non-irritating excipient which is solid at room temperature but liquid at rectal temperature and so will melt in the rectum to release the active component. Such materials include, for example, cocoa butter, beeswax and polyethylene glycols.

The quantity of a compound of the invention required for the prophylaxis or treatment of a particular condition will vary depending on the compound chosen and the condition of the patient to be treated. In general, however, daily dosages may range from around 10 ng/kg to 1000 mg/kg, typically from 100 ng/kg to 100 mg/kg, e.g. around 0.01 mg/kg to 40 mg/kg body weight for oral or buccal administration, from around 10 ng/kg to 50 mg/kg body weight for parenteral administration, and from around 0.05 mg to around 1000 mg, e.g. from around 0.5 mg to around 1000 mg, for nasal administration or administration by inhalation or insufflation.

Comprising in the context of the present specification is intended to meaning including.

Where technically appropriate embodiments of the invention may be combined.

Embodiments are described herein as comprising certain features/elements. The disclosure also extends to separate embodiments consisting or consisting essentially of said features/elements.

The invention will now be described with reference to the following examples, which are merely illustrative and should not in any way be construed as limiting the scope of the present invention, which in the broadest sense is as defined in the claims.

### List of Figures:

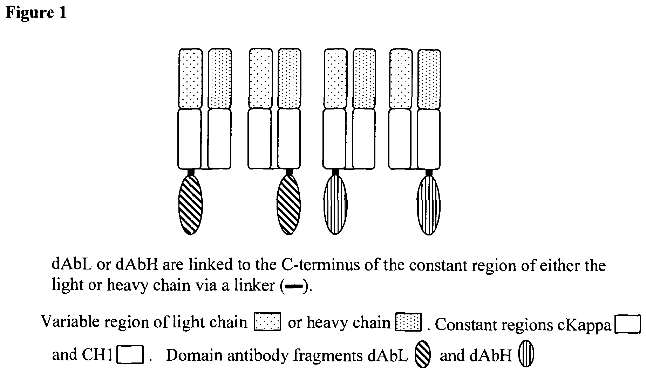

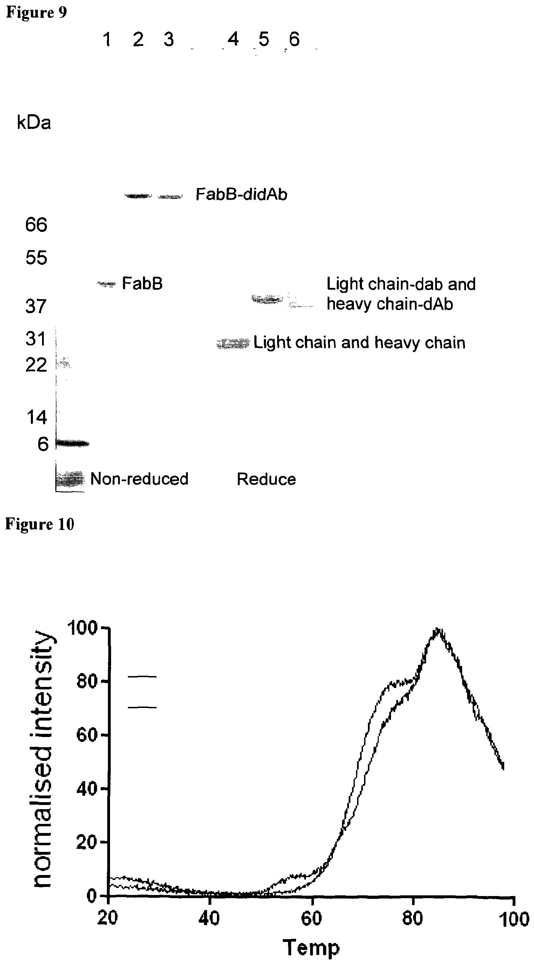

- **Figure 1:**: Diagrammatic representation of Fab-dAbs where the dAb is at the C-terminus

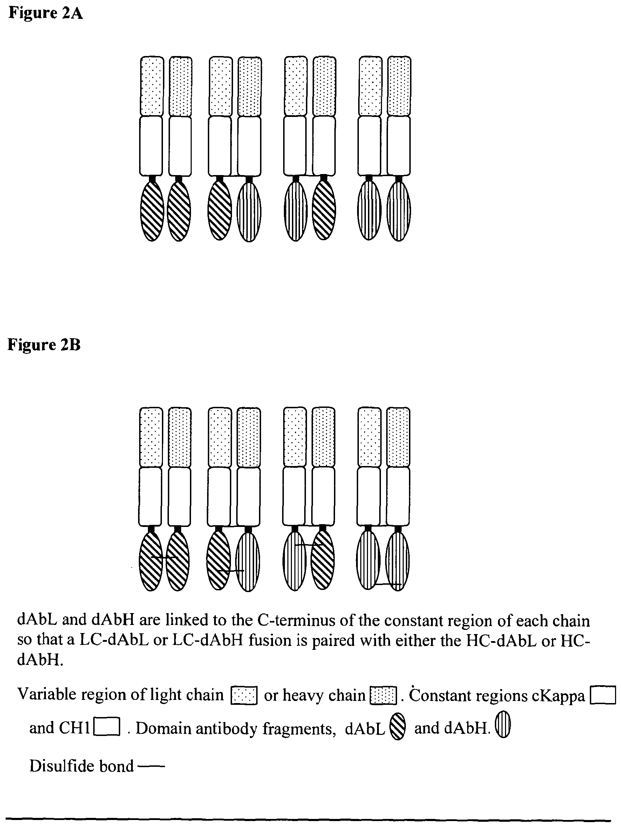

- **Figure 2A:**: Diagrammatic representation of Fab-didAbs
- **Figure 2B:**: Diagrammatic representation of Fab-didAbs with additional disulfide stabilisation between the dAbs.
- **Figure 3:**: SDS PAGE analysis of FabA-dAbL3 (CK-SG₄SE) (1) and FabA-dAbL3 (CK-G[APAPA]₂) (2).
- **Figure 4:**: Western blot analysis of FabA-dAbL3 (CK-SG₄SE) (1) and FabA-dAbL3 (CK-G[APAPA]₂) (2).
- **Figure 4a:**: SDS PAGE of FabB-didAbs
Lane M = SeeBlue markers
Lanes 1 & 2 = IgG control
Lane 3 = FabB
Lane 4 = FabB-didAb, -dAbL1 (CK-G4Sx2) & dAbH1 (CH1-G4Sx2)
Lane 5 = FabB-didAb, -dAbL2 (CK-G4Sx2) & dAbH2 (CH1-G4Sx2)
- **Figure 5:**: Sequences of domain antibodies dAbH1, dAbH2, dAbL1 and dAbL2 and the CDRs derived from each of those antibodies.
- **Figure 6:**: FabB-dAb constructs comprising FabB heavy or light chain variable domain fused to a domain antibody.
- **Figure 7**: Fab'A heavy and light chain sequences and FabA heavy chain sequence..
- **Figure 8a, 8b & 8c**: Murinised Fab-didAb amino acid sequences.

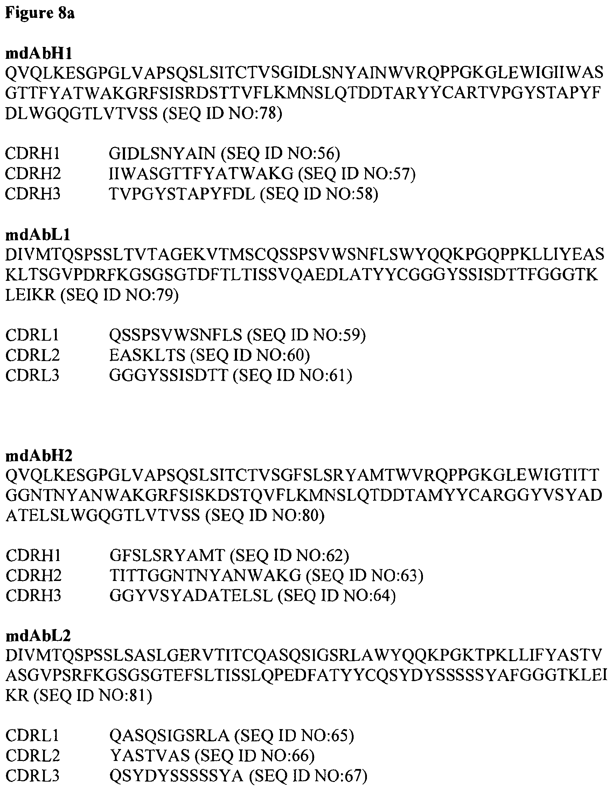

- **Figure 8a**: shows the amino acid sequence of CDRs in various murine dAbs.

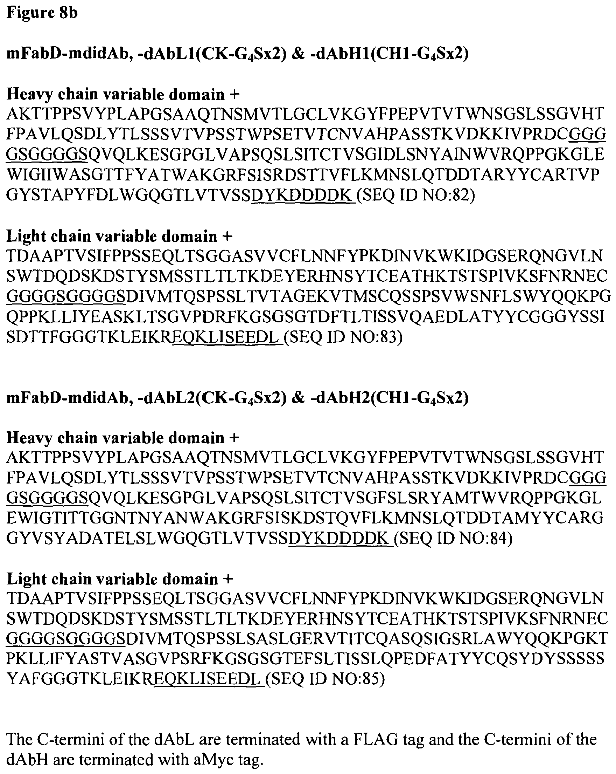

- **Figure 8b**: shows the amino acid sequence of mFabD-mdidAb:
dAbL1(CK-G4Sx2)
dAbH1(CH1-G4Sx2)
dAbL2(CK-G4Sx2) &
dAbH2(CH1-G4Sx2)

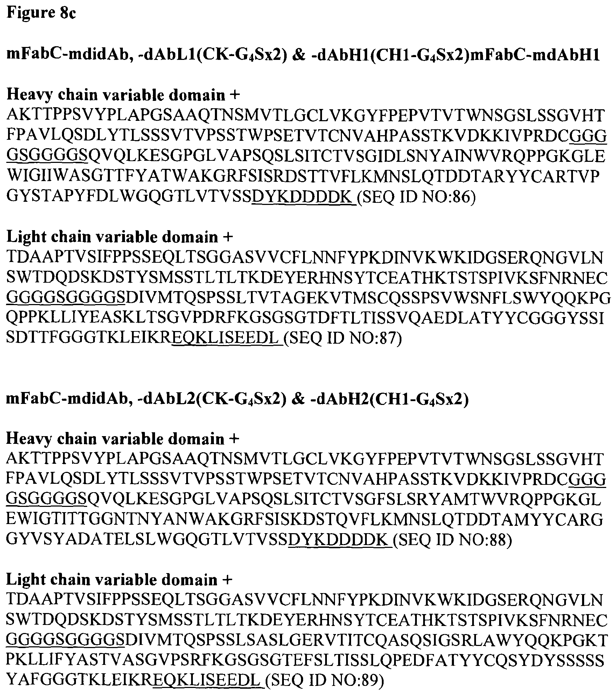

- **Figure 8c**: shows the amino acid sequence of mFabD-mdidAb:dAbL1(CK-G4Sx2) &
dAbH1(CH1-G4Sx2)mFabC-mdAbH1
dAbL2(CK-G4Sx2) &
dAbH2(CH1-G4Sx2
- **Figure 9**: shows SDS PAGE of FabB-didAbs
Lanes 1 & 4 are Fab'B
Lanes 2 & 5 are FabB-didAb, -dAbL1(CK-G4Sx2) & -dAbH1(CH1-G4Sx2)
Lanes 3 & 6 are FabB-didAb, -dAbL2(CK-G4Sx2) & -dAbH2(CH1-G4Sx2)
- **Figure 10**: shows a diagrammatic representation of a Thermofluor thermal stability assay.

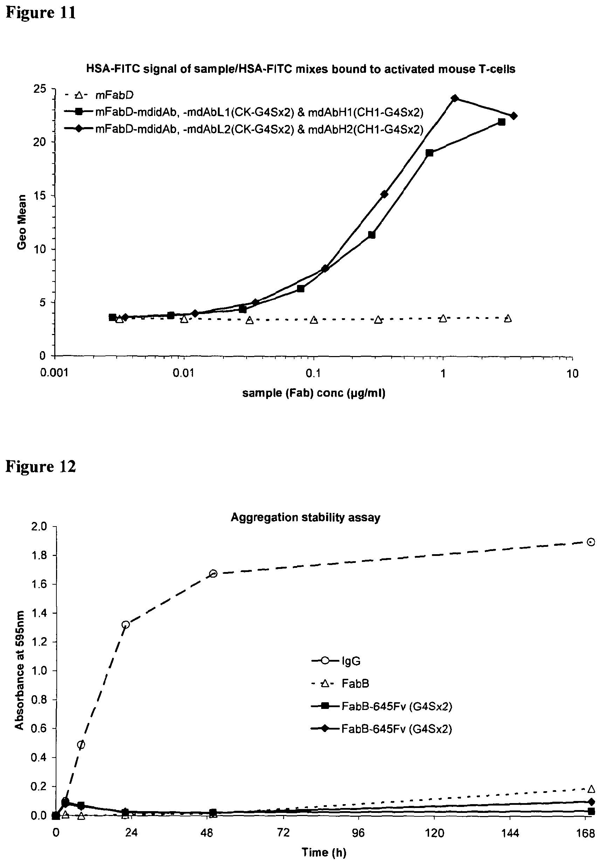

- **Figure 11**: shows a plot of HAS-FITC signal/HAS-FITC mixes bound to activated mouse T cells.
- **Figure 12**: shows a plot of an aggregation stability assay.

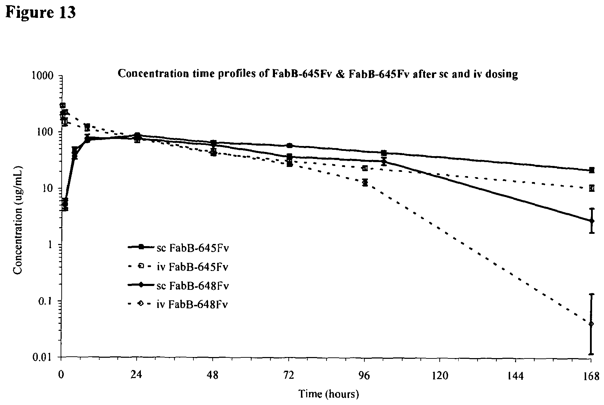

- **Figure 13**: shows in vivo concentration profiles over time after subcutaneous and intravenous dosing

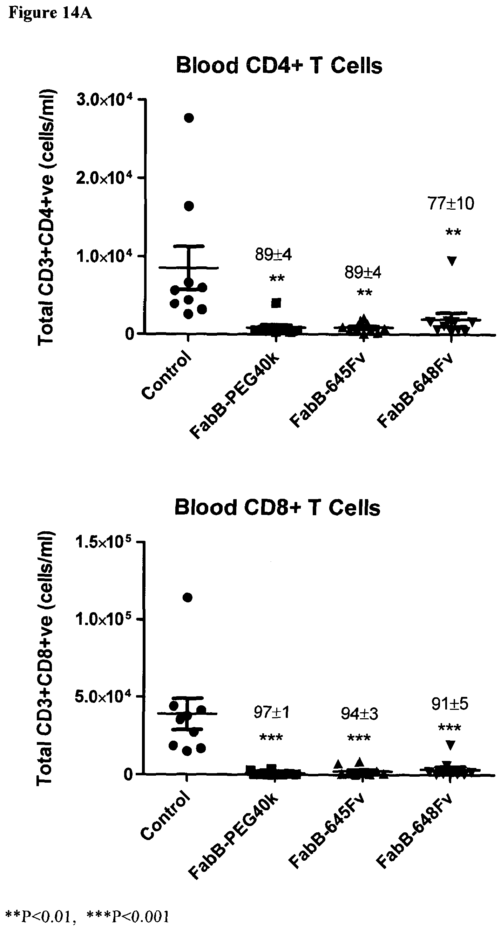

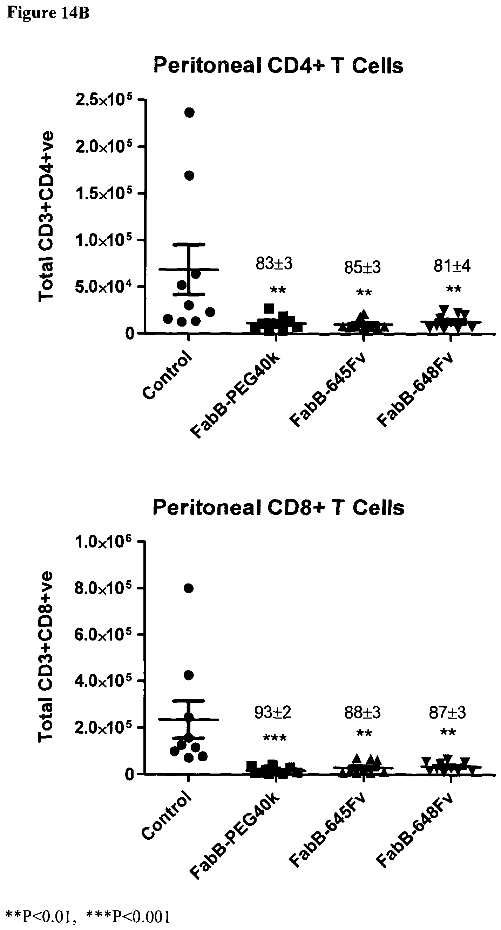

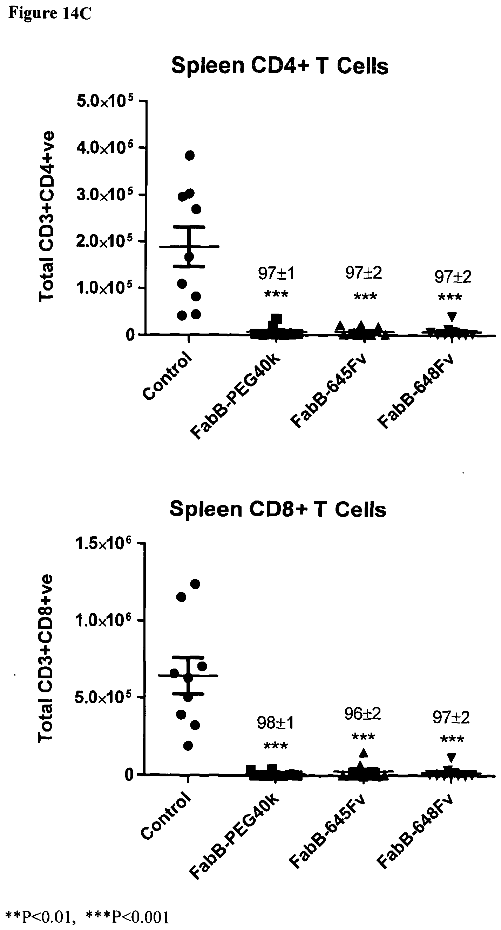

- **Figure 14A, B and C**: show certain CD4+ cell and CD8+ cell readouts

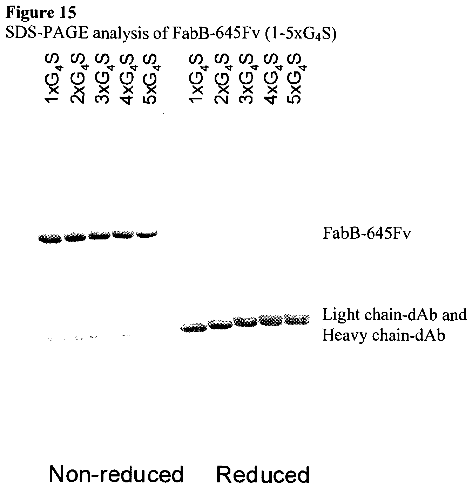

- **Figure 15**: shows SDS-PAGE analysis of FabB-645Fv

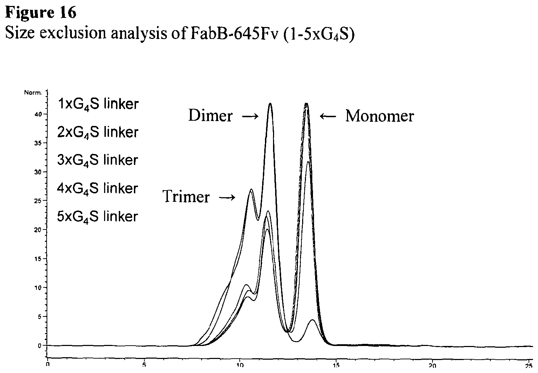

- **Figure 16**: shows size exclusion analysis of FabB-645Fv

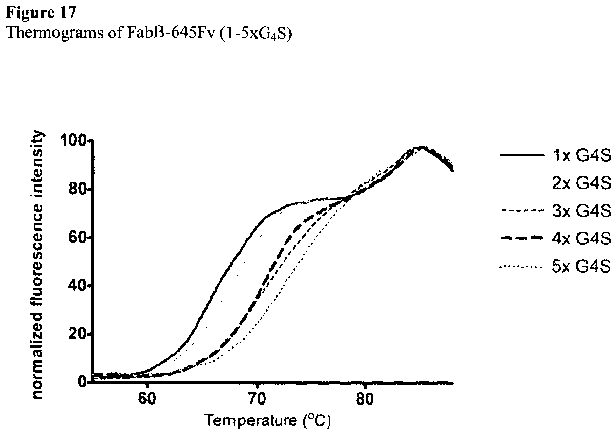

- **Figure 17**: shows thermograms of FabB-645Fv with various linker lengths.

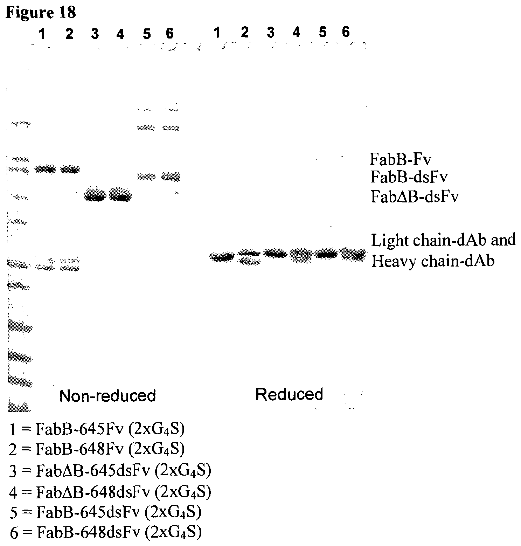

- **Figure 18**: shows SDS-PAGE analysis of certain FabB constructs

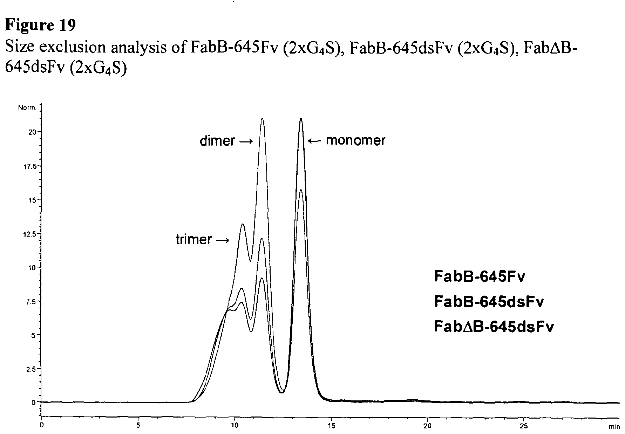

- **Figure 19**: shows size exclusion analysis of various FabB-645Fv constructs

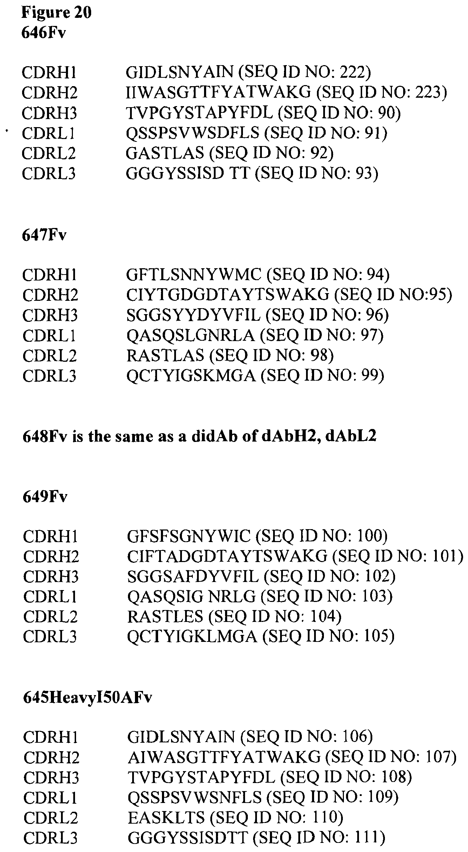

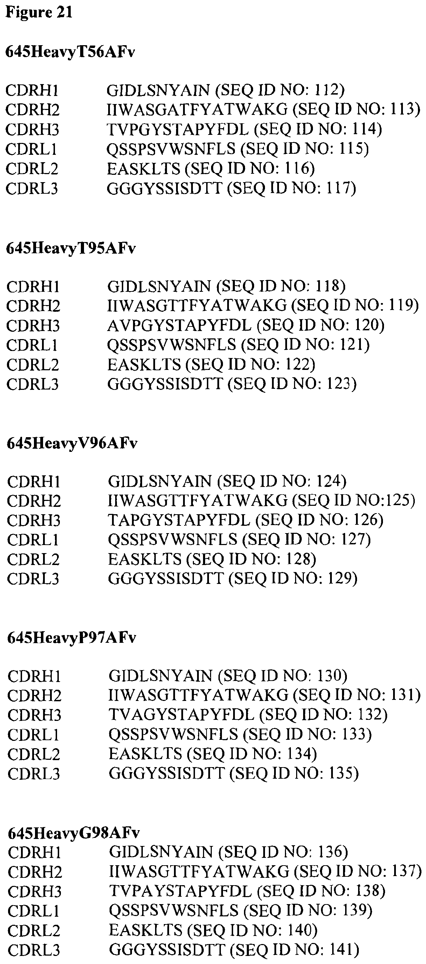

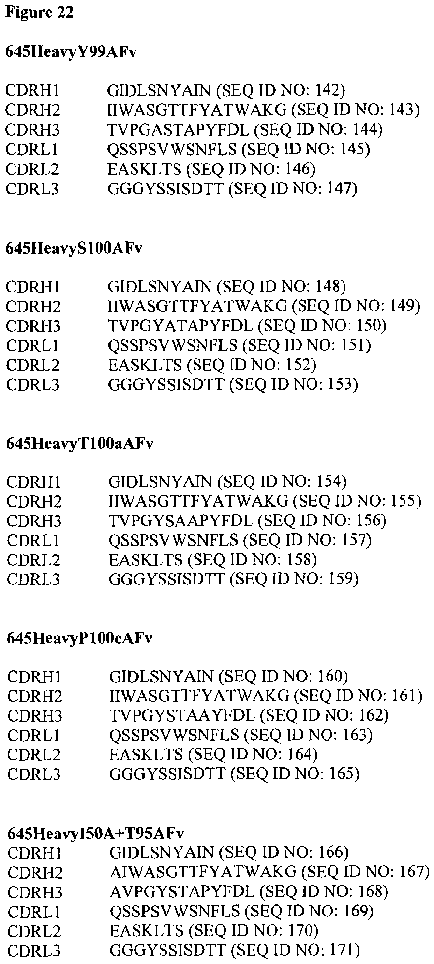

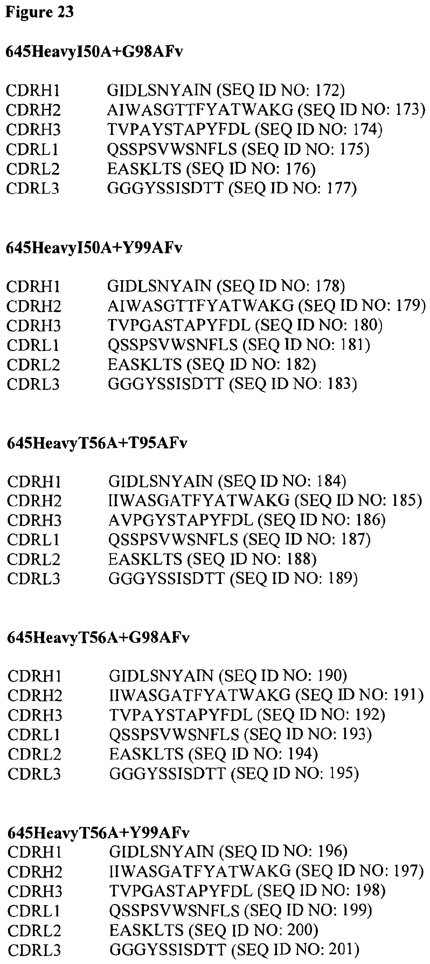

- **Figures 20 to 24**: show sequences for certain formats.

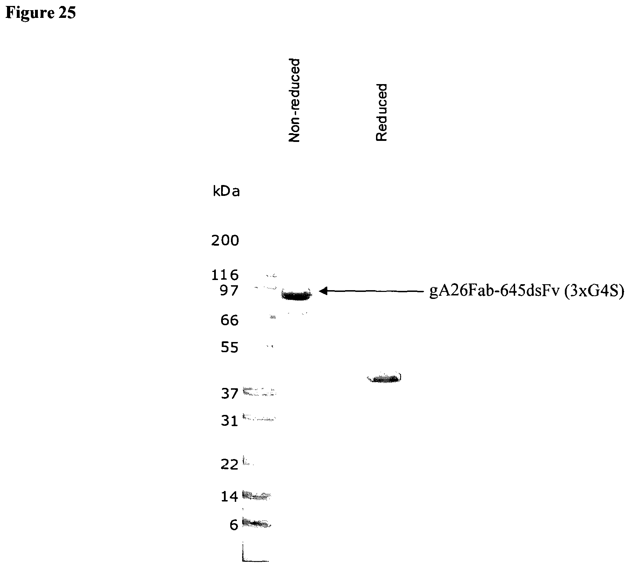

- **Figure 25**: shows an SDS page analysis for a construct a Fab-645dsFV wherein the VH/VL pair a located at the C-terminal of the Fab and are disulfide stabilised.

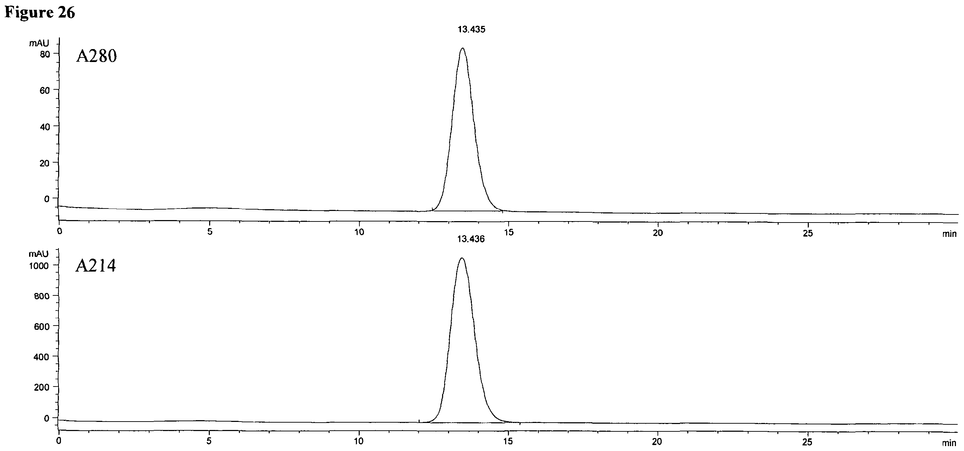

- **Figure 26**: shows a size exclusion analysis for a construct iof Figure 25.

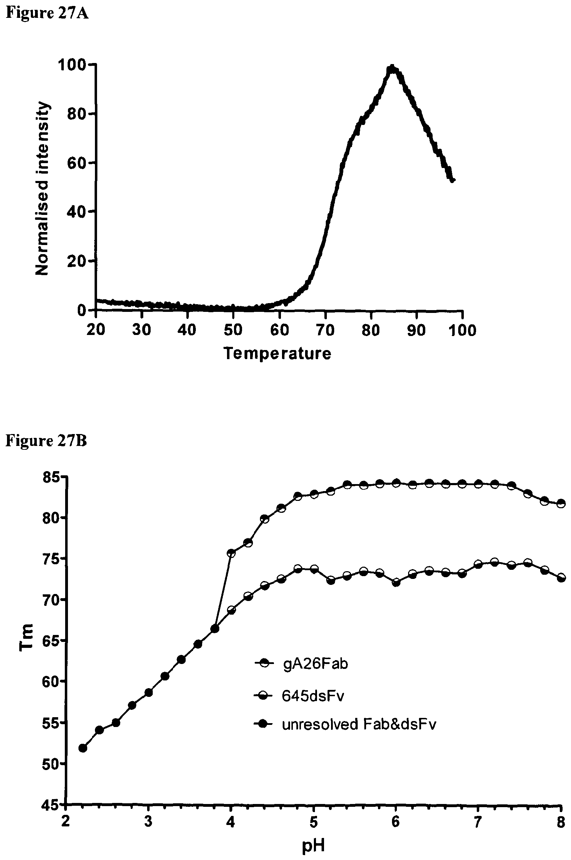

- **Figure 27A**: shows a thermofluor analysis for a construct according to the present disclosure.
- **Figure 27B**: shows a Tm versus pH plot.

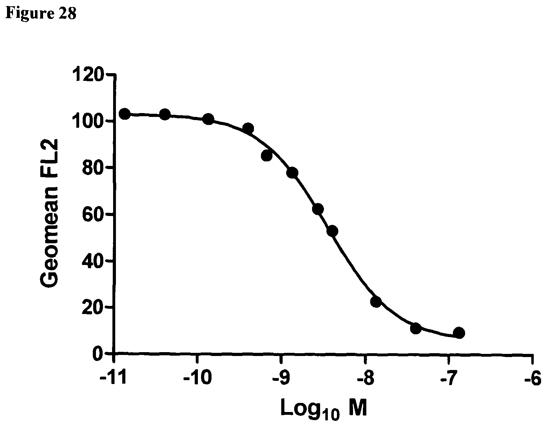

- **Figure 28**: shows an in vitro assay, for a construct according to the present disclosure, based on inhibition of human OX40 ligand binding on human PCMBs.

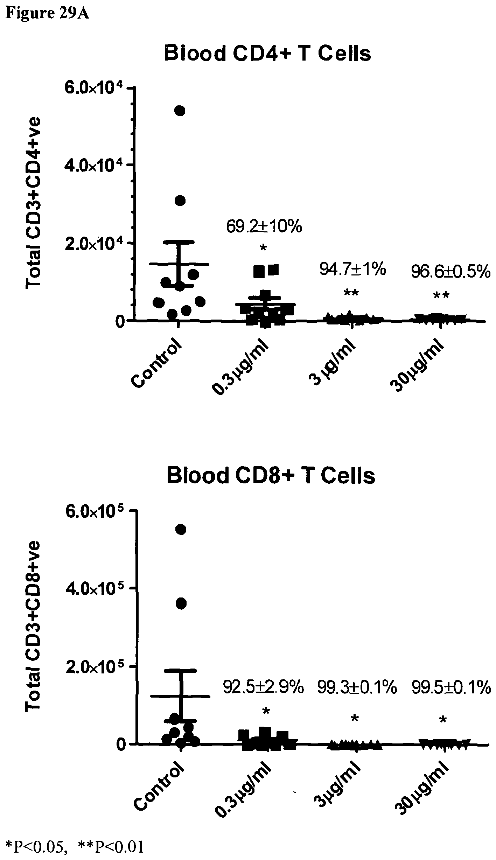

- **Figure 29A-D**: show the in vivo efficacy of a construct according to the present disclosure and in particular the effect on CD4+ and CD8+, blood, peritoneal and spleen cells.

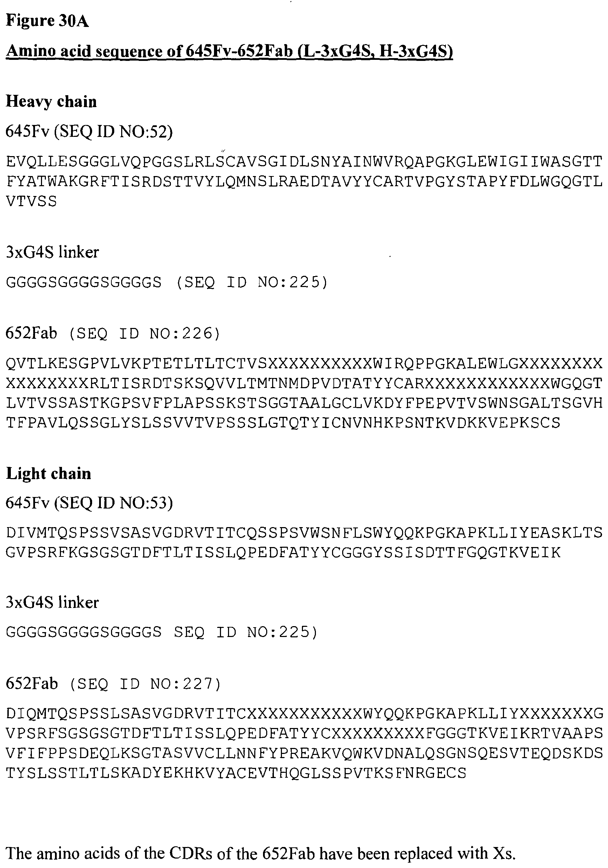

- **Figure 30A-D**: show sequences for certain formats according to the disclosure

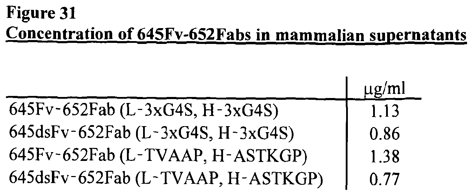

- **Figure 31**: shows expression data for certain constructs

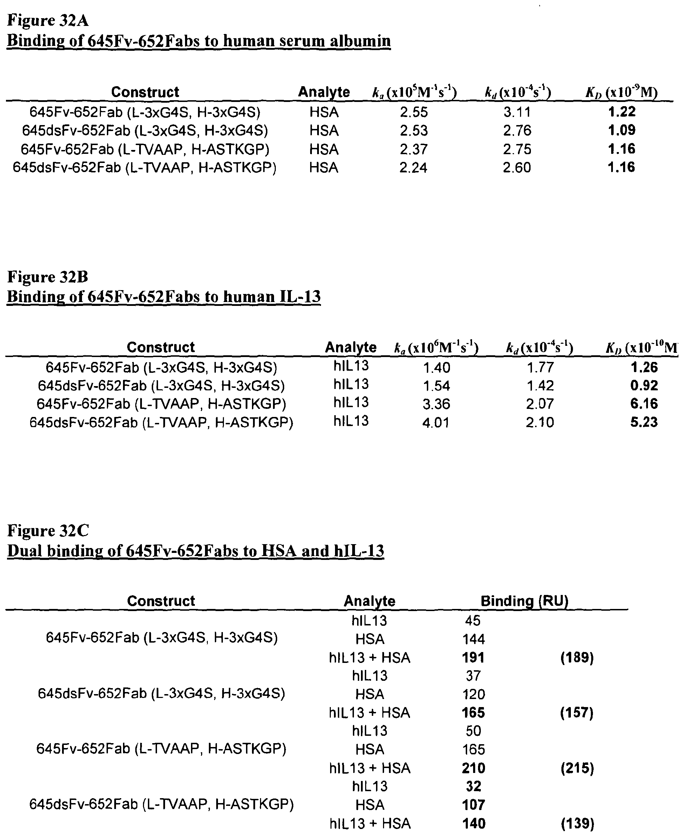

- **Figure 32A-C**: shows binding data for certain constructs.

### KEY

| | |
|---|---|
| **-645Fv** | equates to didAbL1 and H1 (the linker used for each dAB will be the same unless indicated otherwise). |
| **648Fv** | equates to didAbL2 and H2 (the linker used for each dAB will be the same unless indicated otherwise). |
| **-645dsFv** | equates to didAbLland H1 (the linker used for each dAB will be the same unless indicated otherwise) wherein L1 and H1 are stabilised by a disulfide bond. |
| **-648dsFv** | equates to didAbL2and H2 (the linker used for each dAB will be the same unless indicated otherwise) wherein L2 and H3 are stabilised by a disulfide bond. |
| **FabΔ** | are Fabs which lack the interchain cysteine bond (ie between CH and CL or CK) |

### Experimental:

**Abbreviations:** unless the context indicates otherwise "m" as a pre-fix is intended to refer to murine.

Unless the context indicates otherwise "h" as a pre-fix is intended to refer to human. Fab A, Fab B, Fab C and Fab D components may be provided below in different formats.

### EXAMPLE 1. Production of a dAb specific for human serum albumin

An in-frame DNA encoded transcription unit encoding a dAb with specificity for human serum albumin was produced using recombinant DNA technology.

Where desired an in-frame DNA encoded transcription unit encoding a dAb with specificity for a recruitment protein can be produced using recombinant DNA technology.

### EXAMPLE 2. Production of antibody fragment

For fusion of a dAb to the C-terminus of the light chain, DNA was synthesised encoding a human kappa light chain constant region (with the Km3 allotype of the kappa constant region), a peptide linker and a dAb and cloned as a SacI-PvuII restriction fragment into the UCB-Celltech in-house expression vector pTTOD(Fab) (a derivative of pTTO-1, described in Popplewell et al., Methods Mol. Biol. 2005; 308: 17-30) which contains DNA encoding the human gamma-1 CH1 constant region. This gave rise to a dicistronic gene arrangement consisting of the gene for the humanised light chain fused via a linker to a dAb followed by the gene for the humanised heavy chain Fab fragment, both under the control of the tac promoter. Also encoded is a unique BspE1 site upstream of the Gly4Ser linker, or an AscI site upstream of the Ala-Pro-rich linker.

For fusion of a dAb the C-terminus of the heavy chain, DNA was synthesised encoding a human CH1 fragment (of the γ1 isotype) followed by a linker encoding sequence and a dAb. This was subcloned as an ApaI-EcoRI restriction fragment into the UCB-Celltech in-house expression vector pTTOD(Fab) (a derivative of pTTO-1, described in Popplewell *et al*., above) which contains DNA encoding the human gamma-1 CH1 constant region. This gave rise to a dicistronic gene arrangement consisting of the gene for the humanised light chain a non-coding intergenic sequence and followed by a heavy chain fused via a linker to a dAb, both under the control of the tac promoter. The recombinant expression plasmid was transformed into the *E*. *coli* strain W3110 in which expression is induced by addition of IPTG. Expression experiments were performed at small scale initially (5ml culture volumes) with addition of 200uM IPTG at OD(600nm) of approx. 0.5, cells were harvested 2 hours post induction and extracted overnight at 30°C in Tris/EDTA. Clarified extracts were used for affinity analysis by Biacore. Constructs giving promising expression yields and activities were selected for fermentation.

### Methods applicable to the following Examples and Comparative Data

In the following examples the antibody chain to which the dAb is fused is denoted either as CK or LC for the cKappa light chain and as CH1 or HC for the heavy chain constant domain, CH1.

### Construction of FabA-dAb fusion plasmids for expression in E.coli

Fab-dAb fusion proteins were constructed by fusing dAbL3 or dAbH4 to the C-terminus of the constant region of either the light or heavy chain of FabA. A flexible (SGGGGSE (SEQ ID NO:1)) or a rigid (G(APAPA)₂ (SEQ ID NO: 34)) linker was used to link the dAb to the cKappa region (SEQ ID NO:75) whereas the linker DKTHTS (SEQ ID NO:2) was used to link the dAb to the CHI region (SEQ ID NO:76). The DNA sequence coding for the constant region-dAb fusion was manufactured synthetically as fragments to enable sub-cloning into the FabA sequence of the in-house pTTOD vector.

Light chain-dAb fusions were constructed by sub-cloning the *Sac*I*-Apa*I fragment of the synthesized genes, encoding a C-terminal cKappa fused to either dAbL3 or dAbH4 via either a (SGGGGSE (SEQ ID NO:1)) or a rigid (G(APAPA)₂ (SEQ ID NO: 34)) linker, into the corresponding sites of a plasmid capable of expressing FabA. Heavy chain-dAb fusions were constructed by sub-cloning the *Apa*I*-Eco*RI fragment of the synthesised genes, encoding a C-terminal CHI fused to either dAbL3 or dAbH4 via a DKTHTS linker, into the corresponding sites of a plasmid capable of expressing FabA.

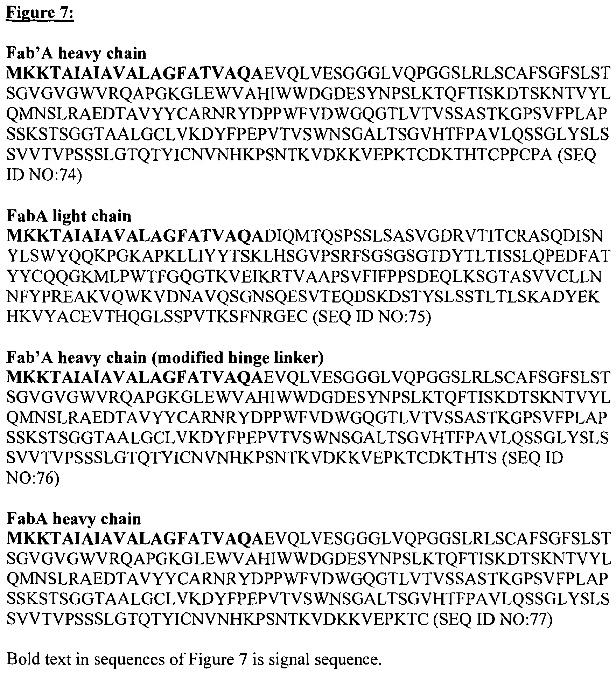

Fab' A is derived from an IL-1 beta binding antibody, the heavy and light chain sequences of which are provided in SEQ ID NOs:74 and 75 respectively as shown in Figure 7. In Fab'A where the light chain has a dAb attached, the hinge of the heavy chain was altered to DKTHTS even where no dAb is attached to the heavy chain (SEQ ID NO:76).

FabA comprises the same light chain sequence (SEQ ID NO:75) and a truncated heavy chain sequence which terminates at the interchain cysteine (SEQ ID NO:77).

**dAbL3** and **dAbH4** are light and heavy chain domain antibodies respectively which bind human serum albumin.

### Construction of FabA-didAb fusion plasmids for expression in E.coli

FabA-didAb with dAbL3 or dAbH4 on both light and heavy chains were constructed by sub-cloning the *Apa*I*-Eco*RI fragment coding for CH1-dAb fusions into the existing Fab-dAb plasmids where the dAb is fused to the light chain via the flexible linker.

### Construction of FabB-dAb fusion plasmids for expression in mammalian cells

The FabB-dAbs, FabB-dAbH1 (CH1-G₄Sx2), FabB-dAbH2 (CH1-G₄Sx2), FabB-dAbL1 (CH1-G₄Sx2), FabB-dAbL2 (CH1-G₄Sx2) were all assembled by PCR then cloned into a mammalian expression vector under the control of the HCMV-MIE promoter and SV40E polyA sequence. These were paired with a similar vector containing the FabB light chain for expression in mammalian cells (see below).

FabB is derived from an antibody which bids a cell surface co-stimulatory molecule.

dAbH1, dAbH2, dAbL1 and dAbL2 were obtained as described in Example 3.

### Mammalian expression of FabB-dAbs and didAbs

HEK293 cells were transfected with the heavy and light chain plasmids using Invitrogen's 293fectin transfection reagent according to the manufacturer's instructions. Briefly, 2µg heavy chain plasmid + 2µg light chain plasmid was incubated with 10µl 293fectin + 340µl Optimem media for 20mins at RT. The mixture was then added to 5x10⁶ HEK293 cells in suspension and incubated for 4 days with shaking at 37°C.

### Biacore

Binding affinities and kinetic parameters for the interactions of Fab-dAb constructs were determined by surface plasmon resonance (SPR) conducted on a Biacore T100 using CM5 sensor chips and HBS-EP (10mM HEPES (pH7.4), 150mM NaCl, 3mM EDTA, 0.05% v/v surfactant P20) running buffer. Fab-dAb samples were captured to the sensor chip surface using either a human F(ab')₂-specific goat Fab (Jackson ImmunoResearch, 109-006-097) or an in-house generated anti human CH1 monoclonal antibody. Covalent immobilisation of the capture antibody was achieved by standard amine coupling chemistry.

Each assay cycle consisted of firstly capturing the Fab-dAb using a 1 min injection, before an association phase consisting of a 3 min injection of antigen, after which dissociation was monitored for 5 min. After each cycle, the capture surface was regenerated with 2 x 1 min injections of 40mM HCl followed by 30s of 5mM NaOH. The flow rates used were 10µl/min for capture, 30µl/min for association and dissociation phases, and 10µl/min for regeneration.

For kinetic assays, a titration of antigen (for human serum albumin typically 62.5nM-2µM, for IL-1β 1.25-40nM) was performed, a blank flow-cell was used for reference subtraction and buffer-blank injections were included to subtract instrument noise and drift.

Kinetic parameters were determined by simultaneous global-fitting of the resulting sensorgrams to a standard 1:1 binding model using Biacore T100 Evaluation software. In order to test for simultaneous binding, 3 min injections of either separate 5µM HSA or 100nM IL-1β, or a mixed solution of 5µM HSA and 100nM IL-1β were injected over the captured Fab-dAb.

### Fab-dAb Purification from E.coli

### Periplasmic extraction

*E.coli* pellets containing the Fab-dAbs within the periplasm were re-suspended in original culture volume with 100mM Tris/HCl, 10mM EDTA pH 7.4. These suspensions were then incubated at 4°C for 16 hours at 250rpm. The re-suspended pellets were centrifuged at 10000xg for 1 hour at 4°C. The supernatants were removed and 0.45 µm filtered.

### Protein-G capture

The Fab-dAbs were captured from the filtered supernatant by Protein-G chromatography. Briefly the supernatants were applied, with a 20 minute residence time, to a Gammabind Plus Sepharose (GE Healthcare) column equilibrated in 20mM phosphate, 150mM NaCl pH7.1. The column was washed with 20mM phosphate, 150mM NaCl pH7.1 and the bound material eluted with 0.1M glycine/HCl pH2.8. The elution peak was collected and pH adjusted to ∼pH5 with 1M sodium acetate. The pH adjusted elutions were concentrated and diafiltered into 50mM sodium acetate pH4.5 using a 10k MWCO membrane.

### Ion Exchange

The Fab-dAbs were further purified by cation exchange chromatography at pH4.5 with a NaCl elution gradient. Briefly the diafiltered Protein-G eluates were applied to a Sourcel5S (GE Healthcare) column equilibrated in 50mM sodium acetate pH4.5. The column was washed with 50mM sodium acetate pH4.5 and the bound material eluted with a 20 column volume linear gradient from 0 to 1M NaCl in 50mM sodium acetate pH4.5. Third column volume fractions were collected through out the gradient. The fractions were analysed by A280 and SDS-PAGE and relevant fractions pooled.

### Gel filtration

If required the Fab-dAbs were further purified by gel filtration. Briefly the FabA-dAbL3 (CK-SG₄SE) pooled ion exchange elution fractions were applied to a Superdex200 (GE Healthcare) column equilibrated in 50mM sodium acetate, 125mM NaCl pH 5.0 and eluted with an isocratic gradient of 50mM sodium acetate, 125mM NaCl pH 5.0. 1/120 column volume fractions were collected through out the gradient. The fractions were analysed by A280 and SDS-PAGE and relevant fractions pooled.

For Fab-dAbs that did not undergo gel filtration, the pooled ion exchange elution fractions were concentrated and diafiltered into 50mM sodium acetate, 125mM NaCl pH 5.0 using a 10k MWCO membrane.

### SDS-PAGE

Samples were diluted with water where required and then to 10µl was added 10µL 2X sample running buffer. For non-reduced samples, 2µL of 100mM NEM was added at this point, for reduced samples 2µL of 10X reducing agent was added. The sample were vortexed, incubated at 85°C for 5 mins, cooled and centrifuged at 12500 rpm for 30secs. The prepared samples were loaded on to a 4-20% acrylamine Tris/Glycine SDS gel and run for 100mins at 125V. The gels were either transferred onto PVDF membranes for Western blotting or stained with Coomassie Blue protein stain.

### Western Blotting

Gels were transferred to PVDF membranes in 12mM Tris, 96mM glycine pH8.3 for 16 hours at 150mA. The PVDF membrane was block for lhr with 2% Marvel™ in PBS + 0.1% Tween20 (Blocking buffer)
*anti-light chain*
   HRP-rabbit anti-human kappa light chains, 1/5000 dilution in blocking buffer for 1hr.
*anti-heavy chain*
   mouse anti-human heavy chain, 1/7000 dilution in blocking buffer for lhr. Followed by HRP-goat anti-mouse, 1/2000 dilution in blocking buffer for 1hr.
*anti-His tag*
   rabbit anti-His6, 1/1000 dilution in blocking buffer for lhr. Followed by HRP-goat anti-rabbit IgG, 1/1000 dilution in blocking buffer for 1hr.

All blots were washed 6 times with 100ml PBS + 0.1% Tween20 for 10 minutes per wash. The blots were developed with either ECL reagent for 1min before being exposed to Amersham Hyperfilm, or metal enhanced DAB reagent for 20-30 minutes followed by water.

### High temperature reverse phase HPLC

Samples (2µg) were analysed on a 2.1mm C8 Poroshell column at 80°C, with a flow rate of 2ml/min and a gradient of 18-38% B over 4mins. A = 0.1% TFA in H₂0 B = 0.065% TFA in 80:20 IPA:MeOH. Detection is by absorption at 214nm.

### ELISA

The yields of Fab-dAb were measured using a sandwich ELISA. Briefly, the Fab-dAb was captured with an anti-CHI antibody then revealed with an anti-kappa-HRP.

### FACS

Samples (mFabD-didAb's) were incubated with 5µg/ml FITC (fluorescein isothiocyanate) labelled HSA for 45 min. The sample/HSA-FITC incubations were then added to activated mouse CD4+ T-cells and incubated for a further 45 min. The cells were washed with PBS and the cell associated fluorescence measured by FACS (fluorescence activated cell sorting).

### EXAMPLE 3

### Generating anti-albumin antibodies

½ lop rabbits were immunised with recombinant chromapure human serum albumin (purchased from Jackson). Rabbits received 3 immunisations of 100ug HSA protein subcutaneously, the first immunisation in complete Freunds adjuvant and subsequent immunisations in incomplete Freunds. Antibodies 1 and 2, 646, 647, and 649 which bind human, mouse and rat serum albumin were isolated using the methods described in WO04/051268. Genes for the heavy chain variable domain (VH) and light chain variable domain (VL) of antibodies 1 and 2 were isolated and sequenced following cloning via reverse transcription PCR.

The light chain grafted sequences were sub-cloned into the rabbit light chain expression vector pVRbcK which contains the DNA encoding the rabbit C-Kappa constant region. The heavy chain grafted sequences were sub-cloned into the rabbit heavy chain expression vector pVRbHFab, which contains the DNA encoding the rabbit Fab' heavy chain constant region. Plasmids were co-transfected into CHO cells and the antibodies produced screened for albumin binding and affinity (Table 1). Transfections of CHO cells were performed using the Lipofectamine™ 2000 procedure according to manufacturer's instructions (InVitrogen, catalogue No. 11668).

### Generating Humanised domain antibodies dAbL1, dAbH1, dAbL2 and dAbH2

Humanised VL and VH regions were designed using human V-region acceptor frameworks and donor residues in the framework regions. One grafted VL region (L1 (SEQ ID NO:53) and L2 (SEQ ID NO:55)) and one VH region (H1 (SEQ ID NO:52) and H2 (SEQ ID NO:54)) were designed for each of antibodies 1 and 2 respectively and genes were built by oligonucleotide assembly and PCR mutagenesis. The grafted domain antibodies and their CDRs are shown in Figure 5.

**Table 1: Affinities of anti-albumin antibodies**

| | **as rabbit Fab** | | **as humanised IgG** | | |
|---|---|---|---|---|---|
| | HSA | MurineSA | HumanSA | Murine SA | RatSA |
| | nM | nM | nM | nM | nM |
| **Antibody 1** (Antibody 645) | 0.31 | 2.6 | 0.82 | 2.9 | 7.9 |
| **Antibody 2** (Antibody 648) | 0.33 | 12 | 0.13 | 23 | 54 |
| Antibody 646 | 0.14 | 1.6 | 0.57 | 1.7 | 4.5 |
| Antibody 647 | 0.60 | 3.6 | 1.3 | 26 | 10 |
| Antibody 649 | 0.54 | 13 | 0.32 | 17 | 44 |

### COMPARATIVE DATA E4: Analysis of FabB-dAbs expressed in mammalian cells

FabB-dAb constructs were produced as described in the methods and the supernatants from the tranfected HEK293 cells containing the FabB-dAbs were tested directly in BIAcore.

Kinetic analysis was conducted to assess the interaction of HSA with FabB-dAb constructs. These consisted of either dAbL1, dAbH2 or dAbL3 fused to the C-terminus of CH1 of FabB (See Figure 6). The FabB-dAbL1 has a higher affinity for HSA, *K_{D}* = 170nM, than FabB-dAbL3, *K_{D}* = 392nM. The FabB-dAbH2 was shown to possess the poorest affinity towards HSA, *K_{D}* = 1074nM, see Table 2.

**Table 2**

| **Construct** | ***kₐ* (x10⁴M⁻¹S⁻¹)** | ***k_{d}* (x10⁻³s⁻¹)** | ***K_{D}* (x10⁻⁹M)** |
|---|---|---|---|
| FabB-dAbL1 (CH1-G₄Sx2) | 1.91 ± 0.74 | 2.18 ± 1.21 | **170 ± 78** |
| FabB-dAbH2 (CH1-G₄Sx2) | 2.66 ± 0.39 | 29 ± 4.76 | **1074 ± 42** |
| FabB-dAbL3 (CH1-G₄Sx2) | 2.63 ± 0.39 | 9.87 ± 1.63 | **392 ± 119** |

Affinity and kinetic parameters determined for the binding of HSA to FabBs fused to dAbL1, dAbH2 or dAbL3. The data shown are mean values ± SEM. (For FabB-dAbL1 and FabB-dAbH2 n=4. For FabB-dAbL3 n=2).

SDS-PAGE and western blotting of the FabB-dAb proteins confirmed that the FabB-dAbs produced were of the expected size.

### EXAMPLE 5: Analysis of FabB-didAbs expressed in mammalian cells

FabB-didAb constructs were produced as described in the methods and the supernatants from the tranfected HEK293 cells containing the didAbs tested directly in BIAcore.

Further analysis was performed using didAb constructs in which single dAbs were fused to both heavy and light C-termini of Fab. Constructs in which the didAb was derived from a natural heavy and light variable domain pairing showed a marked improvement in affinity compared to the single dAb alone (table 2 and 3). The didAb fusion consisting of two identical dAbLls showed no improvement in affinity over that seen for the single dAbL1 (data not shown).

**Table 3**

| **Construct** | ***kₐ* (x10⁴M⁻¹s⁻¹)** | ***k_{d}* (x10⁻³s⁻¹)** | ***K_{D}* (x10⁻⁹M)** |
|---|---|---|---|
| FabB-didAb, -dAbL1 (CK-G₄Sx2) *&* dAbH1 (CH1-G₄Sx2) | 1.78 | 0.16 | **9** |
| FabB-didAb, -dAbL2 (CK-G₄Sx2) *&* dAbH2 (CH1-G₄Sx2) | 0.54 | 0.21 | **39** |

Affinity and kinetic parameters determined for the binding of HSA to FabBs fused to both dAbL1 & dAbH1 or dAbL2 & dAbH2.

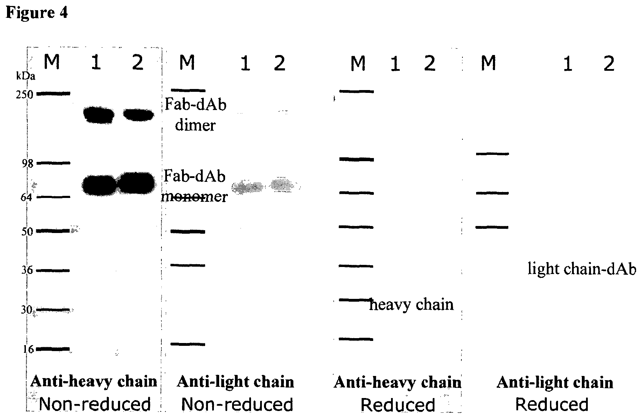

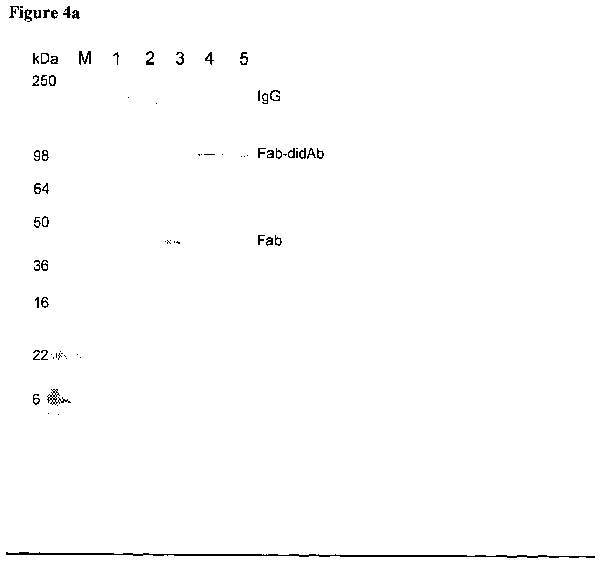

SDS-PAGE of the FabB-didAb proteins confirmed that the FabB-didAbs expressed well and were of the expected size (See Figure 4a). Note this SDS PAGE gel is total protein expressed by the cell.

### EXAMPLE 6 (including comparative data)

### Analysis of purified FabA-dAbs

Plasmids for expression of the Fab-dAbs, Fab'A-dAbL3 (CK-SG₄SE) Fab'A-dAbL3 (CK-G[APAPA]₂) in *E.coli* were constructed as described in the methods. The Fab-dAbs were expressed into the periplasm of the *E. coli* and purified to homogeneity as described in the methods. The purity of the Fab-dAbs were assessed by high temperature reverse phase HPLC, SDS-PAGE and Western blotting. The Fab-dAbs were also assessed for antigen binding by Biacore.

### High temperature reverse phase HPLC

High temperature reverse phase HPLC as performed as described in the methods gave quantitative analysis of all species contained in FabA-dAbL3 (CK-SG₄SE) and FabA-dAbL3 (CK-G[APAPA]₂). The percentage of each species present is shown in table 4.

**Table 4: Quantification of species present in Fab-dAb batches**

| **Species** | **Fab'A-dAbL3 (CK-SG₄SE)** | **Fab'A-dAbL3 (CK-G[APAPA]₂)** |
|---|---|---|
| Peak 1 | 0.6% | 1.8% |
| Peak 2 | 0.6% | 0.0% |
| Peak 3 | 1.0% | 0.3% |
| Peak 4 | 0.9% | 0.8% |
| Fab-dAb peak | 85.5% | 92.9% |
| Di Fab-dAb peak | 11.5% | 4.2% |

### SDS-PAGE

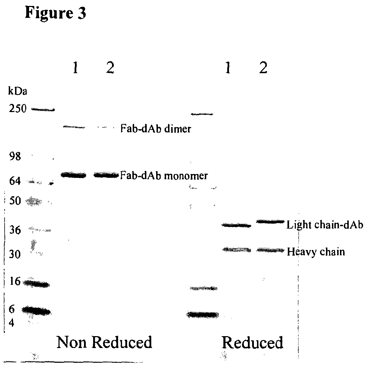

Fab-dAb samples were prepared under non-reduced and reduced conditions and run on a gel as described in the methods. The gel was Coomassie stained. The banding profile of both Fab-dAb samples, Fab'A-dAbL3 (CK-SG₄SE) and Fab'A-dAbL3 (CK-G[APAPA]₂), corresponds well to the profile observed by high temperature reverse phase HPLC (figure 3).

### Western Blot

Fab-dAb samples were subjected to non-reduced SDS-PAGE followed by western blot analysis with anti-light chain and anti-heavy chain antibodies as described in the methods. This confirmed that the dAb was on the light chain of the Fab and that the heavy chain was unmodified in both samples (figure 4). It also demonstrates that all bands detected by coomassie stained, non-reduced SDS PAGE are Fab-dAb related products.

### Biacore

Kinetic analysis by SPR as described in the methods was used to assess the binding of human serum albumin to Fab'A-dAbL3 (CK-SG₄SE) and Fab'A-dAbL3 (CK-G[APAPA]₂). The results in table 5 demonstrate that both constructs are able to bind human serum albumin with a similar affinity (*K_{D}*) of approximately 1µM.

**Table 5**

| **Construct** | ***kₐ* (x10⁴M⁻¹s⁻¹)** | ***k_{d}* (x10⁻²s⁻¹)** | ***K_{D}* (x10⁻⁹M)** |
|---|---|---|---|
| Fab'A-dAbL3 (CK- SG₄SE) | 3.44 | 1.42 | **411** |
| Fab'A-dAbL3 (CK-G[APAPA]₂) | 9.61 | 2.85 | **296** |

Further kinetic analysis demonstrated that all the fusion constructs retained the interaction characteristics of the original FabA towards IL-1β, table 6, with only minor differences seen in the kinetic and affinity parameters.

**Table 6**

| **Construct** | ***kₐ* (x10⁵M⁻¹s⁻¹)** | ***k_{d}* (x10⁻⁵s⁻¹)** | ***K_{D}* (x10⁻¹²M)** |
|---|---|---|---|
| Fab'A-dAbL3 (CK- SG₄SE) | 1.90 | 4.21 | **221** |
| Fab'A-dAbL3 (CK- G[APAPA]₂) | 2.17 | 3.99 | **184** |
| Fab'A | 2.02 | 6.46 | **320** |

The potential for each construct to bind simultaneously to both human serum albumin and the IL-1β antigen was assessed by capturing each construct to the sensor chip surface, before performing either separate 3 min injections of 5µM human serum albumin or 100nM IL-1β, or a mixed solution of both 5µM human serum albumin and 100nM IL-1β. For each Fab-dAb construct the response seen for the combined HSA/IL-1β solution was almost identical to the sum of the responses of the independent injections, see table 7. This shows that the Fab-dAbs are capable of simultaneous binding to both IL-1β and human serum albumin, and that binding of either IL-1β or human serum albumin does not inhibit the interaction of the other. The original FabA bound only to IL-1β, with negligible binding to human serum albumin.

**Table 7**

| **Construct** | **Analyte** | **Binding (RU)** | |
|---|---|---|---|
| Fab'A-dAbL3 (CK- SG₄SE) | HSA + IL-1β | **37.6** | |
| | HSA | 13.2 | **(37.9)** |
| | IL-1β | 24.7 | |
| Fab'A-dAbL3 (CK- G[APAPA]₂) | HSA + IL-1β | **61.9** | |
| | HSA | 30.7 | **(63.6)** |
| | IL-1β | 32.9 | |
| Fab'A | HSA + IL-1β | **30.3** | |
| | HSA | 1.3 | **(30.0)** |
| | IL-1β | 28.7 | |

The table above shows the binding response (RU) seen for each construct after separate injections of HSA or IL-1β, or injection of premixed HSA and IL-1β. In each case the final concentration was 5µM for HSA and 100nM for IL-1β. The sum of the individual HSA and IL-1β responses is shown in parentheses.

### EXAMPLE 7 FabA didAbs

### Expression of FabA-didAbs in E.coli

FabA-dAbs and FabA-didAb fusions terminating with a C-terminal histidine tag (HIS6 tag) were expressed in *Escherichia coli.* After periplasmic extraction, dAb fusion proteins were purified via the C-terminal His6 tag. Fab expression was analysed by Western blotting of a non-reduced gel with anti-CHI and anti-cKappa antibodies. FabA-dAb and FabA-didAb were expressed as full-length proteins and were shown to react to both antibody detection reagents.

### Analysis of FabA-didAbs expressed in E.coli

Further analysis was conducted to characterise the binding of HSA to FabA constructs to which one or more dAbs were fused. Binding assays were performed on a variety of constructs in which dAbL3 or dAbH4 fused to either the light or heavy chain of the FabA (see Table 8 for details of the constructs and summary of the binding data). Although constructs carrying only dAbH4, on either the light or heavy chain, were seen to bind HSA with comparatively poor affinity (≈9µM and 3µM respectively), higher affinity binding was observed for constructs carrying dAbL3, either as a single fusion (on either light or heavy chain) or partnered with a second dAb (dAbL3 or dAbH4) on the opposing chain.

**Table 8**

| **Construct** | ***kₐ* (x10⁴M⁻¹s⁻¹)** | ***k_{d}* (x10⁻³s⁻¹)** | ***K_{D}* (x10⁻⁹M)** |
|---|---|---|---|
| FabA | - | - | nb |
| FabA-dAbL3 (LC-SG4SE) | 4.46 | 16.2 | 363 |
| FabA-dAbH4 (LC SG4SE) | - | - | 9142 |
| FabA-dAbL3 (HC-DKTHTS) | 8.24 | 15.4 | 187 |
| FabA-dAbH4 (HC-DKTHTS) | - | - | 2866 |
| FabA-didAb, -dAbL3 (LC-SG4SE) & -dAbL3 (HC-DKTHTS) | 3.00 | 15.1 | 502 |
| FabA-didAb, -dAbL3 (LC-SG4SE) & -dAbH4 (HC-DKTHTS) | 4.36 | 16.3 | 373 |

Affinity and kinetic parameters determined for the binding of HSA to FabAs carrying dAbL3 or dAbH4 on either light chain (LC) or heavy chain (HC) or both as indicated. No binding (nb) of HSA to the original FabA was detected. The interaction kinetics for the binding of HSA to the FabA with (dAbH4 on HC) or (dAbH4 on LC), were too rapid to determine, therefore affinity (KD) was determined from steady-state binding.

### EXAMPLE 8

### Expression and purification of FabB-didAbs

### Mammalian expression

Prior to transfection CHO-XE cells were washed in Earls Balanced Salts Solution (EBSS), pelleted and resuspended in EBSS at 2x10⁸ cells/ml. Heavy and light chain plasmids were added to the cells at a total concentration of 400ug. Optimised electrical parameters for 800µl cells/DNA mix on the in-house electroporator were used for transfection. Transfected cells were directly transferred to 1L CD-CHO media supplied with glutamax, HT and antimycotic antibiotic solution. Cells were incubated, shaking at 37°C for 24 hours and then shifted to 32°C. Sodium Butyrate 3mM was added on day 4. Supernatants were harvested on day 14 by centrifugation at 1500xg to remove cells. Expression levels were determined by ELISA.

### Mammalian expression supernatant concentration

The mammalian supernatants containing ∼55µg/ml of FabB-didAb as assessed by ELISA were concentrated from 1.8L to 200ml using a Minisette concentrator fitted with a 10kDa molecular weight cut off polyethersulphone (PES) membrane.

### Protein-G purification

The concentrated supernatants were applied to a Gammabind Plus Sepharose (GE Healthcare) column equilibrated in 20mM phosphate, 150mM NaCl pH7.1. The column was washed with 20mM phosphate, 150mM NaCl pH7.1 and the bound material eluted with 0.1M glycine/HCl pH2.7. The elution peak was collected and pH adjusted to ∼pH7 with 2M Tris/HCl pH8.8. The pH adjusted elutions were concentrated to 1mg/ml and diafiltered into 20mM phosphate, 150mM NaCl pH7.1 using a 10kD molecular weight cut off PES membrane.

### SDS-PAGE

Samples were diluted with water where required and then to 26µl was added 10µL 4X LDS sample running buffer. For non-reduced samples, 4µL of 100mM NEM was added and for reduced samples 4µL of 10X reducing agent was added. The samples were vortexed, incubated at 85°C for 5 mins, cooled and centrifuged at 12500 rpm for 30secs. The prepared samples were loaded on to a 4-20% acrylamine Tris/Glycine SDS gel and run for 110mins at 125V. The gels were stained with Coomassie Blue protein stain.

### ELISA

The yields of Fab-didAb were measured using a sandwich ELISA. Briefly, the Fab-didAb was captured with an anti-CHI antibody then revealed with an anti-kappa-HRP.

### SDS-PAGE

FabB and FabB-didAb samples were prepared under non-reduced and reduced conditions and separated on a gel and stained as described in the methods. See Figure 9.

### EXAMPLE 9

### Thermofluor thermal stability assay on FabB-Fv

Samples (1µl of sample at ∼1mg/ml, 8µl of PBS and 1µl of 30x stock of Sypro orange fluorescent dye) were run in quadruplicate in 384 well plates. The plate is heated from 20-99°C using a 7900HT fast real-time PCR system and the fluorescence (excitation at 490nm, emission at 530nm) measured. The results are shown in Table D and Figure 10.

**Table 9**

| | **Tm °C (Fab)** | **Tm °C (Fv)** |
|---|---|---|
| FabB-didAb, | 81.9 ± 0.6 | 68.5 ± 0.5 |
| -dAbL1(CK-G₄Sx2) & | | |
| -dAbL1 (CH1-G₄Sx2) | | |
| FabB-didAb, | 82.4 ± 0.2 | 70.6 ± 0.8 |
| -dAbL2(CK-G₄Sx2) & | | |
| -dAbL2(CH1-G₄Sx2) | | |

### EXAMPLE 10

### Aggregation stability assay of FabB-Fv

Samples at 1mg/ml in PBS were incubated at 25°C with vortexing at 1400rpm. The absorbance is measured at 595nm. This absorbance is due to light scattered by particles and can be correlated with sample aggregation. Both FabB-645Fv (G₄Sx2) and FabB-648Fv (G₄Sx2) are as resistant to aggregation as FabB alone. They are all more resistant to aggregation than the IgG control. (Figure 12)

### EXAMPLE 11

### pH dependency of Fab-Fv binding to HSA

Binding affinities for the interactions of Fab-Fv constructs with HSA were determined as described in the methods except that the running buffers at pH5.0, 5.5, 6.0 and 7.0 were created by mixing 40mM citric acid, 150mM NaCl, 3mM EDTA, 0.05% v/v surfactant P20 and 80mM disodium hydrogen phosphate, 150mM NaCl, 3mM EDTA, 0.05% v/v surfactant P20 to give the desired pH.

The affinity of FabB-645Fv (G₄Sx2) for HSA is unaffected by pH from 7.4 (standard assay pH) to 5.0. The affinity of FabB-648Fv (G₄Sx2) for HSA is affected by pH and there is approximately a 10 fold loss in affinity between pH7.4 and pH5.0.

**Table 10**

| | ***K_{D}* (x10⁻⁹M)** | | | |
|---|---|---|---|---|
| | **pH7.0** | **pH6.0** | **pH5.5** | **pH5.0** |
| FabB-645Fv (G₄Sx2) | 13.3 | 12.5 | 10.7 | 7.1 |
| FabB-648Fv (G₄Sx2) | 3.3 | 11.1 | 24.1 | 47.8 |

### EXAMPLE 12

### In vivo murine PK of FabB-Fv

The pharmacokinetics of FabB-645Fv (G₄Sx2) and FabB-648Fv (G₄Sx2) in male BALB/c mouse were determined following a single administration at 10mg/kg either subcutaneously (sc) or intravenously (iv). Six mice were dosed for each construct and route of administration. Serial blood samples (30 µL) were collected from the tail vein at the following time points: 1, 4, 8, 24, 48, 72, 102 and 168 hours following subcutaneous administration and 30 minutes, 1, 8, 24, 48, 72, 96 and 168 hours following intravenous administration. The collected blood was dispensed into a Sarstedt microvette CB300Z with clot activator for serum separation, and left at room temperature for at least 20 minutes. The microvette was then centrifuged at 20°C at 10,000 rpm for 5 minutes. Serum was removed and stored frozen prior to analysis. The concentration of FabB-645Fv (G₄Sx2) or FabB-648Fv (G₄Sx2) in serum samples was assessed by ELISA. Briefly Nunc Maxisorb Immunomodule Plates were coated with hOX40-Fc in PBS and blocked with 1% BSA in PBS. Serum samples and standards were diluted in 1% BSA in PBS and applied to the plate for 1 hour. The plate was washed with PBS and the revealing antibody of goat anti-human kappa HRP conjugate applied in 1% BSA in PBS for 1 hour. The plate was washed and then developed with TMB substrate followed by stopping with 2.5M sulphuric acid. The absorbance at 630nm wash measured and the concentrations determined from the standard curve.

Both FabB-645Fv (G₄Sx2) and FabB-648Fv (G₄Sx2) have extended half-life in plasma, Figure 13. The half-lives for FabB-645Fv (G₄Sx2) are 71h sc and 62h iv and for FabB-648Fv (G₄Sx2) are 25h sc and 30h iv.

### EXAMPLE 13

### In vivo efficacy study of FabB-Fv

A study to investigate if FabB-645Fv and FabB-648Fv are efficacious *in vivo* was undertaken. Briefly this involved steady state dosing in HuSCID mice and the read out was the prevention of T cell engraftment.

CB17 SCID mice were dosed with a loading dose subcutaneously on day -2 of 2.475mg/kg FabB-645Fv or FabB-648Fv or FabB-PEG40k or PBS. On every subsequent day up to and including day 10 they were dosed with a maintenance dose subcutaneously of 0.75mg/kg FabB-645Fv or FabB-648Fv or FabB-PEG40k or PBS. Each dosing group consisted of 9-10 mice. On day -1 all the mice were treated with 0.87mg/mouse of rat anti-murine TM-β1 antibody to abrogate natural killer cell activity. On day 0 all the mice received an inter peritoneal injection of 8x10⁶ human peripheral blood mononuclear cells. On day 14 the mice are sacrificed and the blood, spleen and a peritoneal lavage were taken. The samples were analysed by FACS for CD4⁺ and CD8⁺ T cells. The data sets were analysed by one way Anova with Dunnett's post test comparison. All the test constructs FabB-645Fv, FabB-648Fv and FabB-PEG40k were equally efficacious in all the compartments tested, i.e. blood peritoneum and spleen. Figures 14A, B and C.

### EXAMPLE 14

### FabB-645Fv mutations to change the affinity of 645Fv for albumin

Point mutations were introduced into selected residues in the CDRs of the heavy chain of the 645Fv portion of FabB-645dsFv (S3xG₄S) by mutagenic PCR. For example I50A is a replacement of Ile 50 with Ala. The various mutations are given in Table 11 below. The affinity of the Fab-645Fv mutants for human albumin was assessed by BIAcore as described in the methods. All the mutations had either unchanged or reduced affinity for human albumin.

**Table 11**

| **Fv heavy mutation** | **Albumin** | **ka (1/Ms)** | **kd (1/s)** | **KD (nM)** |
|---|---|---|---|---|
| I50A | HSA | 3.12E+04 | 1.90E-03 | **60.9** |
| T56A | HSA | 4.65E+04 | 3.78E-04 | **8.12** |
| T95A | HSA | 2.81E+04 | 2.64E-03 | **94.0** |
| V96A | HSA | 2.81E+04 | 6.42E-04 | **22.9** |
| P97A | HSA | 4.60E+04 | 1.26E-02 | **275** |
| G98A | HSA | 4.73E+04 | 2.71E-04 | **5.73** |
| Y99A | HSA | 4.71E+04 | 4.79E-04 | **10.2** |
| S100A | HSA | 3.94E+04 | 1.44E-03 | **36.6** |
| T100aA | HSA | 3.60E+05 | 1.86E-02 | **51.6** |
| Y100cA | HSA | 1.23E+04 | 1.07E-03 | **87.0** |
| I50A and T95A | HSA | 2.12E+04 | 9.94E-03 | **468** |
| I50A and G98A | HSA | 1.79E+04 | 6.96E-03 | **389** |
| I50A and Y99A | HSA | | | **>3500** |
| T56A and T95A | HSA | 2.84E+04 | 8.57E-04 | **30.1** |
| T56A and G98A | HSA | 2.40E+04 | 3.68E-03 | **153** |
| T56A and Y99A | HSA | 2.24E+04 | 1.49E-02 | **664** |

### EXAMPLE 15

### 1-5 Gly4Ser linker length between Fab and Fv

### Construction of FabB-645Fv fusion plasmids for expression in mammalian cells

The FabB-645Fv's with either a SGGGGS, SGGGGSGGGGS, SGGGGSGGGGSGGGGS, SGGGGSGGGGSGGGGSGGGGS or SGGGGSGGGGSGGGGSGGGGSGGGGS linker between the C-termini of the Fab and the N-termini of the Fv were assembled by PCR then cloned into a mammalian expression vectors under the control of the HCMV-MIE promoter and SV40E polyA sequence. The relevant heavy and light chain plasmids were paired for expression in mammalian cells.

### Mammalian expression of FabB-645Fv (1-5xG₄S)

HEK293 cells were transfected with the heavy and light chain plasmids using Invitrogen's 293fectin transfection reagent according to the manufacturer's instructions. Briefly, 24µg heavy chain plasmid + 24µg light chain plasmid was incubated with 120µl 293fectin + 4080µl Optimem media for 20mins at RT. The mixture was then added to 60x10⁶ HEK293 cells in 60mL suspension and incubated for 4 days with shaking at 37°C. All the constructs were equally well expressed.

### Protein-G purification

The mammalian expression suspensions were clarified by centrifugation and the supernatants were concentrated to ∼1.8mL using lOkDa molecular weight cut off centrifugation concentrators. The concentrated supernatants were centrifuged at 16000xg for 10 min to remove any precipitate and then 1.5mL was loaded onto 1ml HiTrap Protein-G columns (GE Healthcare) at 1ml/min. The columns were washed with 20mM phosphate, 40mM NaCl pH7.4 and bound material eluted with 0.1M glycine/HCl pH2.7. The elution peak (2mL) was collected and pH adjusted to ∼pH5 with 250µL of 1M sodium acetate. The pH adjusted elutions were diafiltered into 20mM phosphate, 150mM NaCl pH7.1 using 10kDa molecular weight cut off centrifugation concentrators and concentrated to ∼250µL. All the constructs had similar purification profiles and the final concentrations were 0.5-1.1mg/ml.

### Affinity of FabB-645Fv (1-5xG₄S) for albumin

The affinities of the purified FabB-645Fv (1-5xG₄S) constructs for human and mouse albumin were determined as described in the Methods. The different linker lengths of the Fv of 1 to 5 xGly4Ser between the C-termini of the Fab and the N-termini of the Fv had no affect on the affinity of the 645Fv for either human or mouse albumin.

**Table 12**

| | **Albumin** | **KD (nM)** | **Albumin** | **KD (nM)** |
|---|---|---|---|---|
| FabB-645Fv (1xG₄S) | Human | 8.77 | Mouse | 2.18 |
| FabB-645Fv (2xG₄S) | Human | 6.72 | Mouse | 8.01 |
| FabB-645Fv (3xG₄S) | Human | 9.87 | Mouse | 8.92 |
| FabB-645Fv (4xG₄S) | Human | 7.90 | Mouse | 7.24 |
| FabB-645Fv (5xG₄S) | Human | 3.90 | Mouse | 6.09 |

### SDS-PAGE analysis of purified FabB-645Fv (1-5xG₄S)

FabB-645Fv (1-5xG4S) samples were prepared under non-reduced and reduced conditions and separated on a gel and stained as described in the methods. See Figure 15.

### Size exclusion analysis of purified FabB-645Fv (1-5xG₄S)

FabB-645Fv (1-5xG₄S) samples were analysed for size on a Superdex200 10/300GL Tricorn column (GE Healthcare) developed with an isocratic gradient of 20mM phosphate 150mM NaCl pH7.4 at 1ml/min.

A linker length between the C-termini of the Fab and the N-termini of the Fv of either 1xG₄S or 2xG₄S reduces the amount of monomer FabB-645Fv whilst increasing the amount of dimer and higher multimers. The amount of monomer is least for the 1xG₄S linker length. A linker length between the C-termini of the Fab and the N-termini of the Fv of either 3xG₄S, 4xG₄S or 5xG₄S increased the amount of monomer FabB-645Fv whilst decreasing the amount of dimer and higher multimers with the levels being similar for all three linker lengths. Figure 16.

**Table 13**

| | **Monomer** | **Dimer** | **High Multimers** |
|---|---|---|---|
| FabB-645Fv (1xG₄S) | 5 % | 47 % | 48 % |
| FabB-645Fv (2xG₄S) | 27 % | 38 % | 36 % |
| FabB-645Fv (3xG₄S) | 51 % | 32 % | 17 % |
| FabB-645Fv (4xG₄S) | 55 % | 30 % | 15 % |
| FabB-645Fv (5xG₄S) | 51 % | 31 % | 18 % |

### Thermofluor thermal stability analysis of purified FabB-645Fv (1-5xG₄S)

Samples (1µl of sample at ∼1mg/ml, 8µl of PBS and 1µl of 30x stock of Sypro orange fluorescent dye) were run in quadruplicate in 384 well plates. The plate is heated from 20-99°C using a 7900HT fast real-time PCR system and the fluorescence (excitation at 490nm, emission at 530nm) measured. The results are shown in Table 14 and Figure 17.

**Table 14**

| | **Tm °C (Fab)** | **Tm °C (Fv)** |
|---|---|---|
| FabB-645Fv (1xG₄S) | 82.8 ± 0.6 | 67.4 ± 0.4 |
| FabB-645Fv (2xG₄S) | 83.4 ± 0.3 | 68.7 ± 0.3 |
| FabB-645Fv (3xG₄S) | 83.4 ± 0.3 | 69.5 ± 0.6 |
| FabB-645Fv (4xG₄S) | 83.8 ± 0.3 | 71.3 ± 1.0 |
| FabB-645Fv (5xG₄S) | 83.8 ± 0.4 | 72.0 ± 0.7 |

### EXAMPLE 16

### Disulphide stabilisation of the Fv in a Fab-Fv

### FabB-645dsFv (2xG₄S), FabB-648dsFv (2xG₄S), FabΔB-645dsFv (2xG₄S) and FabΔB-648dsFv (2xG₄S) fusion plasmids for expression in mammalian cells

Point mutations were introduced into the FabB-645Fv (2xG₄S) and FabB-648Fv (2xG₄S) DNA sequences at selected residues in the framework region of both the heavy chain and the light chain of the Fv by mutagenic PCR. The mutations introduced to create an interchain disulphide bond between the heavy and light chains of the Fv were heavy chain G44C and light chain G100C. As well as adding the cysteins to create the interchain disulphide bond in the Fv, the natural interchain disulphide between the heavy chain and light chain of the Fab was removed by mutagenic PCR by changing the cysteines to serines. Fvs that contain an interchain disulphide bond were termed dsFv, Fabs that lack an interchain disulphide bond were termed FabΔ. The DNA for all these constructs was then cloned into a mammalian expression vectors under the control of the HCMV-MIE promoter and SV40E polyA sequence. The relevant heavy and light chain plasmids were paired for expression in mammalian cells.

### Mammalian expression of FabB-645dsFv (2xG₄S), FabB-648dsFv (2xG₄S), FabΔB-645dsFv (2xG₄S) and FabΔB-648dsFv (2xG₄S)

HEK293 cells were transfected with the heavy and light chain plasmids using Invitrogen's 293fectin transfection reagent according to the manufacturer's instructions. Briefly, 24µg heavy chain plasmid + 24µg light chain plasmid was incubated with 120µl 293fectin + 4080µl Optimem media for 20mins at RT. The mixture was then added to 60x10⁶ HEK293 cells in 60mL suspension and incubated for 4 days with shaking at 37°C. All the constructs were equally well expressed.

### Protein-G purification of FabB-645dsFv (2xG₄S), FabB-648dsFv (2xG₄S), FabΔB-645dsFv (2xG₄S) and FabΔB-648dsFv (2xG₄S)

The mammalian expression suspensions were clarified by centrifugation and the supernatants were concentrated to ∼1.8mL using 10kDa molecular weight cut off centrifugation concentrators. The concentrated supernatants were centrifuged at 16000xg for 10 min to remove any precipitate and then 1.5mL was loaded onto 1ml HiTrap Protein-G columns (GE Healthcare) at 1ml/min. The columns were washed with 20mM phosphate, 40mM NaCl pH7.4 and bound material eluted with 0.1M glycine/HCl pH2.7. The elution peak (2mL) was collected and pH adjusted to ∼pH5 with 250µL of 1M sodium acetate. The pH adjusted elutions were diafiltered into 20mM phosphate, 150mM NaCl pH7.1 using 10kDa molecular weight cut off centrifugation concentrators and concentrated to ∼250µL. All the constructs had similar purification profiles and the final concentrations were 0.5-0.8mg/ml.

### Affinity of FabB-645dsFv (2xG₄S), FabB-648dsFv (2xG₄S), FabΔB-645dsFv (2xG₄S) and FabΔB-648dsFv (2xG₄S) for albumin

The affinities of the purified FabB-645dsFv (2xG₄S), FabB-648dsFv (2xG₄S) FabΔB-645dsFv (2xG₄S), FabΔB-648dsFv (2xG₄S) constructs for human and mouse albumin were determined as described in the Methods. The disulphide stabilisation of the Fv had no affect or slightly increased the affinity of the Fv for both human or mouse albumin.

**Table 15**

| | **Albumin** | **KD (nM)** | **Albumin** | **KD (nM)** |
|---|---|---|---|---|
| FabB-645Fv (2xG₄S) | Human | 17.5 | Mouse | 24.7 |
| FabB-645dsFv (2xG₄S) | Human | 12.6 | Mouse | 14.0 |
| FabΔB-645dsFv (2xG₄S) | Human | 8.3 | Mouse | 12.2 |
| FabB-648Fv (2xG₄S) | Human | 9.4 | Mouse | 42.4 |
| FabB-648dsFv (2xG₄S) | Human | 3.1 | Mouse | 59.6 |
| FabΔB-648dsFv (2xG₄S) | Human | 8.3 | Mouse | 59.8 |

### SDS-PAGE analysis of purified FabB-645dsFv (2xG₄S), FabB-648dsFv (2xG₄S), FabΔB-645dsFv (2xG4S) and FabΔB-648dsFv (2xG₄S)

Purified FabB-645dsFv (2xG₄S), FabB-648dsFv (2xG₄S) FabΔB-645dsFv (2xG₄S), FabΔB-648dsFv (2xG₄S) samples were prepared under non-reduced and reduced conditions and separated on a gel and stained as described in the methods. See Figure 18.

### Size exclusion analysis of purified FabB-645dsFv (2xG₄S), FabB-648dsFv (2xG₄S), FabΔB-645dsFv (2xG₄S) and FabΔB-648dsFv (2xG₄S)

Purified FabB-645dsFv (2xG₄S), FabB-648dsFv (2xG₄S) FabΔB-645dsFv (2xG₄S), FabΔB-648dsFv (2xG₄S) samples were analysed for size on a Superdex200 10/300GL Tricorn column (GE Healthcare) developed with an isocratic gradient of 20mM phosphate 150mM NaCl pH7.4 at 1ml/min.

The introduction of an interchain disulphide bond into the Fv of either a 645Fv or 648Fv increased the amount of monomer Fab-Fv species compared with the Fab-Fv in which the Fv did not have an inter chain disulphide. The removal of the natural interchain disulphide bond from the Fab part of a Fab-Fv had only a small effect on the amount of monomer species present. Figure 19.

**Table 16**

| | **Monomer** | **Dimer** | **High Multimers** |
|---|---|---|---|
| FabB-645Fv (2xG₄S) | 26 % | 38 % | 35 % |
| FabB-645dsFv (2xG₄S) | 43 % | 21 % | 37 % |
| FabΔB-645dsFv (2xG₄S) | 40 % | 25 % | 34 % |
| FabB-648dsFv (2xG₄S) | 50 % | 26 % | 24 % |
| FabΔB-648dsFv (2xG₄S) | 55 % | 24% | 20 % |

### Thermofluor thermal stability analysis of purified FabB-645dsFv (2xG₄S), FabB-648dsFv (2xG₄S), FabΔB-645dsFv (2xG₄S) and FabΔB-648dsFv (2xG₄S)

Samples (1µl of sample at ∼1mg/ml, 8µl of PBS and 1µl of 30x stock of Sypro orange fluorescent dye) were run in quadruplicate in 384 well plates. The plate is heated from 20-99°C using a 7900HT fast real-time PCR system and the fluorescence (excitation at 490nm, emission at 530nm) measured.

The introduction of an interchain disulphide bond into the Fv part of a Fab-Fv of either a 645Fv or 648Fv increased the thermal stability of the Fv compared with the Fab-Fv in which the Fv did not have an inter chain disulphide. The removal of the natural interchain disulphide bond from the Fab part of a Fab-Fv decreased the thermal stability of the Fab part of the Fab-Fv

**Table 17**

| | **Tm °C (Fab)** | **Tm °C (Fv)** |
|---|---|---|
| FabB-645Fv (2xG₄S) | 81.9 ± 0.6 | 68.5 ± 0.5 |
| FabB-645dsFv (2xG₄S) | 83.6 ± 0.3 | 71.6 ± 0.3 |
| FabΔB-645dsFv (2xG₄S) | 79.5 ± 0.1 | 70.8 ± 0.6 |
| FabB-648Fv (2xG₄S) | 82.4 ± 0.2 | 70.6 ± 0.8 |
| FabB-648dsFv (2xG₄S) | 82.8 ± 0.3 | 75.0 ± 0.6 |
| FabΔB-648dsFv (2xG₄S) | n.d. | 73.6 ± 0.8 |

| | | |
|---|---|---|
| n.d. = not determined. The analysis software was unable to resolve this inflection point. | | |

### Biacore Method For FabD

Binding affinities and kinetic parameters for the interactions of Fab-dAb and Fab-didAb constructs were determined by surface plasmon resonance (SPR) conducted on a Biacore T100 using CM5 sensor chips and HBS-EP (10mM HEPES (pH7.4), 150mM NaCl, 3mM EDTA, 0.05% v/v surfactant P20) running buffer. Human Fab samples were captured to the sensor chip surface using either a human F(ab')₂-specific goat Fab (Jackson ImmunoResearch, 109-006-097) or an in-house generated anti human CH1 monoclonal antibody. Murine Fab samples were captured using a murine F(ab')2-specific goat Fab (Jackson ImmunoResearch, 115-006-072). Covalent immobilisation of the capture antibody was achieved by standard amine coupling chemistry.

Each assay cycle consisted of firstly capturing the Fab-dAb or Fab-didAb construct using a 1 min injection, before an association phase consisting of a 3 min injection of antigen, after which dissociation was monitored for 5 min. After each cycle, the capture surface was regenerated with 2 x 1 min injections of 40mM HCl followed by 30s of 5mM NaOH. The flow rates used were 10µl/min for capture, 30µl/min for association and dissociation phases, and 10µl/min for regeneration.

For kinetic assays, a titration of antigen (for human or mouse serum albumin typically 62.5nM-2µM, for IL-1β 1.25-40nM, for cell surface receptor D 20-1.25nM) was performed, a blank flow-cell was used for reference subtraction and buffer-blank injections were included to subtract instrument noise and drift.

Kinetic parameters were determined by simultaneous global-fitting of the resulting sensorgrams to a standard 1:1 binding model using Biacore T100 Evaluation software. In order to test for simultaneous binding, 3 min injections of either separate 5µM HSA or 100nM IL-1β, or a mixed solution of 5µM HSA and 100nM IL-1β were injected over the captured Fab-dAb. Simultaneous binding of albumin and cell surface receptor D was assessed in the same manner using final concentrations of 2µM HSA or MSA and 20nM murine cell surface receptor D.

### EXAMPLE 17

### Mammalian expression of mFabC-mdidAbs and mFabD-mdidAbs

HEK293 cells were transfected with the heavy and light chain plasmids using Invitrogen's 293fectin transfection reagent according to the manufacturer's instructions. Briefly, 2µg heavy chain plasmid + 2µg light chain plasmid was incubated with 10µl 293fectin + 340µl Optimem media for 20mins at RT. The mixture was then added to 5x10⁶ HEK293 cells in suspension and incubated for 6 days with shaking at 37°C.

### ELISA

The yields of mFab-mdidAb were measured using a sandwich ELISA. Briefly, the mFab-mdidAb was captured with an anti-CHI antibody then revealed with an anti-kappa-HRP.

**Table 18**

| | **ELISA expression (ug/mL)** |
|---|---|
| mFabD-mdidAb, -dAbL1(CK-G₄Sx2) & -dAbH1(CH1-G₄Sx2) | 44 |
| mFabD-mdidAb, -dAbL2(CK-G₄Sx2) & -dAbH2(CH1-G₄Sx2) | 35 |
| mFabC-mdidAb, -dAbL1(CK-G₄Sx2) & -dAbH1(CH1-G₄Sx2) | 11 |
| mFabC-mdidAb, -dAbL2(CK-G₄Sx2) & -dAbH2(CH1-G₄Sx2) | 14 |

### EXAMPLE 18

Further kinetic analysis was conducted to assess the interactions of serum albumin and human OX40 to the purified FabB-didAb, -dAbL1(CK-G4Sx2) & -dAbH1(CH1-G4Sx2) and FabB-didAb, -dAbL2(CK-G4Sx2) & -dAbH2(CH1-G4Sx2) fusions (Table 19). Both FabB-didAb, -dAbL1(CK-G4Sx2) & -dAbH1(CH1-G4Sx2) and FabB-didAb, -dAbL2(CK-G4Sx2) & -dAbH2(CH1-G4Sx2) retained the affinity for human OX40 of the original FabB (Table 20).

The potential for the FabB-didAb, -dAbL1(CK-G4Sx2) & -dAbH1(CH1-G4Sx2) and FabB-didAb, -dAbL2(CK-G4Sx2) & -dAbH2(CH1-G4Sx2) constructs to bind simultaneously to both human or mouse serum albumin and human OX40 was assessed by capturing each Fab-didAb construct to the sensor chip surface, before performing either separate 3 min injections of 2µM albumin (human or mouse) or 50nM human OX40, or a mixed solution of both 2µM albumin and 50nM OX40. HSA binding was seen for both Fab-didAb constructs. For each Fab-didAb construct the response seen for the combined albumin/OX40 solution was almost identical to the sum of the responses of the independent injections (summarised in table 21). This shows that the Fab-didAbs are capable of simultaneous binding to both OX40 and serum albumin. The original FabB bound only OX40, with no significant binding to either human or mouse albumin.

**Table 19**

| **Construct** | **Albumin** | ***kₐ* (x10⁴M⁻¹s⁻¹)** | *k_{d}***(x10⁻⁵s⁻¹)** | ***K_{D}* (x10⁻⁹M)** |
|---|---|---|---|---|
| FabB-didAb, -dAbL1(CK-G4Sx2) & -dAbH1(CH1-G4Sx2 | HSA | 1.65 | 2.06 | 12.5 |
| FabB-didAb, -dAbL2(CK-G4Sx2) & -dAbH2(CH1-G4Sx2 | HSA | 1.80 | 1.24 | 6.92 |
| FabB-didAb, -dAbL1(CK-G4Sx2) & -dAbH1(CH1-G4Sx2 | MSA | 1.83 | 1.82 | 9.94 |
| FabB-didAb, - dAbL2(CK-G4Sx2) & -dAbH2(CH1-G4Sx2 | MSA | nd | nd | - |

Affinity and kinetic parameters determined for HSA and MSA binding to Fab-didAb fusions.

**Table 20**

| **Construct** | ***kₐ* (x10⁵M⁻¹s⁻¹)** | ***k_{d}* (x10⁻⁵s⁻¹)** | ***K_{D}* (x10⁻¹²M)** |
|---|---|---|---|
| FabB | 2.92 | 22.6 | 775 |
| FabB-didAb, -dAbL1(CK-G4Sx2) & -dAbH1(CH1-G4Sx2 | 3.58 | 8.54 | 238 |
| FabB-didAb, -dAbL2(CK-G4Sx2) & -dAbH2(CH1-G4Sx2 | 3.27 | 13.6 | 415 |

Affinity and kinetic parameters for hOX40-Fc binding to FabB and FabB-didAb fusions.

**Table 21**

| **Construct** | **Analyte** | **Binding (RU)** | |
|---|---|---|---|
| FabB | HSA | 2.5 | |
| | MSA | -2.5 | |
| | OX40 | 89.5 | |
| | HSA + OX40 | 90.1 | (92) |
| | MSA + OX40 | 86.5 | (87) |
| FabB-didAb, -dAbL1(CK-G4Sx2) & -dAbH1(CH1-G4Sx2 | HSA | 109.1 | |
| | MSA | 93.3 | |
| | OX40 | 73.7 | |
| | HSA + OX40 | 186.1 | (182.8) |
| | MSA + OX40 | 170.3 | (167) |
| FabB-didAb, -dAbL2(CK-G4Sx2) & -dAbH2(CH1-G4Sx2 | HSA | 50.9 | |
| | MSA | 2.4 | |
| | OX40 | 52.9 | |
| | HSA + OX40 | 104.2 | (103.8) |
| | MSA + OX40 | 54.9 | (55.3) |

The table above shows the binding response (RU) seen for each construct after separate injections of HSA or MSA or hOX40-Fc, or injection of premixed albumin and hOX40-Fc. In each case the final concentration was 2µM albumin HSA and 50nM hOX40-Fc. The sum of the individual albumin and hOX40-Fc responses is shown in parentheses.

### EXAMPLE 19

Further kinetic analysis was conducted to assess the interactions of serum albumin and murine cell surface receptor D to mFabD-mdidAb, -mdAbL1(CK-G₄Sx2) & mdAbH1(CH1-G₄Sx2) and mFabD-mdidAb, -mdAbL2(CK-G₄Sx2) & mdAbH2(CH1-G₄Sx2) (Table 22). Both mFabD-mdidAbs showed relatively high affinity binding to HSA (*K_{D}* = 2.78nM and 8.97nM respectively). mFabD-mdidAb,-mdAbL2(CK-G₄Sx2) & mdAbH2(CH1-G₄Sx2) also bound MSA with a similar affinity (*K_{D}* = 22nM), however no binding to MSA was seen for mFabD-mdidAb,-mdAbL1(CK-G₄Sx2) & mdAbH1(CH1-G₄Sx2). Both mFabD-mdidAbs retained the affinity for murine cell surface receptor Dof the original mFabD (Table 23).

The potential for mFabD-mdidAb, -mdAbL1(CK-G₄Sx2) & mdAbH1(CH1-G₄Sx2) and mFabD-mdidAb, -mdAbL2(CK-G₄Sx2) & mdAbH2(CH1-G₄Sx2) to bind simultaneously to both human or mouse serum albumin and murine cell surface receptor D was assessed by capturing each mFab-mdidAb construct to the sensor chip surface, before performing either separate 3 min injections of 2µM albumin (human or mouse) or 20nM murine cell surface receptor D, or a mixed solution of both 2µM albumin and 20nM cell surface receptor D. Again HSA binding was seen for both mFab-mdidAb constructs whereas only mFabD-mdidAb, -mdAbL2(CK-G₄Sx2) & mdAbH2(CH1-G₄Sx2) bound MSA. For each mFab-mdidAb construct the response seen for the combined albumin/ cell surface receptor D solution was almost identical to the sum of the responses of the independent injections (summarised in table 24). This shows that the mFab-mdidAbs are capable of simultaneous binding to both cell surface receptor D and serum albumin. The original mFabD bound only cell surface receptor D, with no significant binding to either human or mouse albumin.

**Table 22**

| **Construct** | **Albumin** | ***kₐ* (x10⁴M⁻¹s⁻¹)** | ***k_{d}* (x10⁻⁵s⁻¹)** | ***K_{D}* (x10⁻⁹M)** |
|---|---|---|---|---|
| mFabD-mdidAb, -mdAbL1(CK-G₄Sx2) & mdAbH1 (CH1-G₄Sx2) | HSA | 1.01 | 2.82 | **2.78** |
| mFabD-mdidAb, -mdAbL2(CK-G₄Sx2) & mdAbH2(CH1-G₄Sx2) | HSA | 1.19 | 10.69 | **8.97** |
| mFabD-mdidAb, -mdAbL1(CK-G₄Sx2) & mdAbH1(CH1-G₄Sx2) | MSA | - | - | - |
| mFabD-mdidAb, -mdAbL2(CK-G₄Sx2) & mdAbH2(CH1-G₄Sx2) | MSA | 1.03 | 22.73 | **22.06** |

Affinity and kinetic parameters determined for HSA and MSA binding to mFabD-mdidAb, -mdAbL1(CK-G₄Sx2) & mdAbH1(CH1-G4Sx2) and mFabD-mdidAb, -mdAbL2(CK-G4Sx2) & mdAbH2(CH1-G4Sx2).

**Table 23**

| **Construct** | ***kₐ* (x10⁵M⁻¹s⁻¹)** | ***k_{d}* (x10⁻⁵s⁻¹)** | ***K_{D}* (x10⁻¹²M)** |
|---|---|---|---|
| mFabD | 1.98 | 2.50 | 126 |
| mFabD-mdidAb, -mdAbL1(CK-G₄Sx2) & mdAbH1 (CH1-G₄Sx2) | 2.01 | 4.67 | 233 |
| mFabD-mdidAb, -mdAbL2(CK-G₄Sx2) & mdAbH2(CH1-G₄Sx2) | 3.62 | 6.36 | 176 |

Affinity and kinetic parameters for murine cell surface receptor D-Fc binding to mFabD, mFabD-mdidAb, -mdAbL1(CK-G₄Sx2) & mdAbH1(CH1-G₄Sx2) and mFabD-mdidAb, -mdAbL2(CK-G₄Sx2) & mdAbH2(CH1-G₄Sx2).

**Table 24**

| **Construct** | **Analyte** | **Binding (RU)** | |
|---|---|---|---|
| mFabD | receptor D | 61.3 | |
| | HSA | 0.9 | |
| | MSA | -1.1 | |
| | receptor D + HSA | **62.9** | **(62.2)** |
| | receptor D + MSA | **59.2** | **(60.2)** |
| mFabD-mdidAb, -mdAbL1(CK-G₄Sx2) & mdAbH1 (CH1-G₄Sx2) | receptor D | 39.8 | |
| | HSA | 59.9 | |
| | MSA | -0.6 | |
| | receptor D + HSA | **101.2** | **(99.7)** |
| | receptor D + MSA | **39.9** | **(39.2)** |
| mFabD-mdidAb, -mdAbL2(CK-G₄Sx2) & mdAbH2(CH1-G₄Sx2) | receptor D | 42.6 | |
| | HSA | 61.9 | |
| | MSA | 43.5 | |
| | receptor D + HSA | **105.3** | **(104.5)** |
| | receptor D + MSA | **86.3** | **(86.1)** |

The table above shows the binding response (RU) seen for each construct after separate injections of HSA or MSA or murine cell surface receptor D-Fc, or injection of premixed albumin and murine cell surface receptor D-Fc. In each case the final concentration was 2µM albumin HSA and 20nM murine cell surface receptor D-Fc. The sum of the individual albumin and murine cell surface receptor D-Fc responses is shown in parentheses.

### EXAMPLE 20

Further analysis was conducted to assess the simultaneous interaction of mFabD-mdidAb, -mdAbL1(CK-G₄Sx2) & mdAbH1(CH1-G₄Sx2) or mFabD-mdidAb, - mdAbL2(CK-G₄Sx2) & mdAbH2(CH1-G₄Sx2) with serum albumin and murine cell surface receptor D expressed on the cell surface. Both mFabD-mdidAbs were capable of binding FITC labelled HSA and cell surface receptor X expressed on the cell surface of activated murine T-cells simultaneously (figure 11). mFabD was capable of binding cell surface receptor X expressed on the cell surface of activated murine T-cells, data not shown, but did not bind FITC labelled HSA.

### EXAMPLE 21

### Expression and purification of FabB-645dsFv (3xG4S)

### Mammalian expression

Prior to transfection 1.4 x 1010 CHO-SV cells were washed in Earls Balanced Salts Solution (EBSS) and pelleted. 7 mg of heavy and 7mg of light chain plasmid DNA were added to the cells. EBBS buffer is added to a final volume of 10ml. 800µl of the above per cuvette was electroporated using optimised electrical parameters on the in-house electroporator. Transfected cells were directly transferred to 7x1L CD-CHO media supplied with glutamax, HT and antimycotic antibiotic solution. Cells were incubated, shaking at 37°C for 24 hours and then shifted to 32°C. Sodium Butyrate 3mM was added on day 4. Supernatants were harvested on day 10 or 14 by centrifugation at 1500xg to remove cells. Expression levels were determined by Protein-G assay.

### Mammalian supernatant concentration

The pooled mammalian supernatants containing 15µg/ml of FabB-645dsFv (3xG4S) were concentrated from 6.5L to 800ml using a Minisette concentrator fitted with 2 x 10kDa molecular weight cut off polyethersulphone (PES) membranes.

### Protein-G purification

The concentrated supernatant was applied at 135cm/hr to a 50ml Gammabind Plus Sepharose (GE Healthcare) column equilibrated in 20mM phosphate, 150mM NaCl pH7.4. The column was washed with 20mM phosphate, 150mM NaCl pH7.4 and the bound material eluted with 0.1M glycine/HCl pH2.7. The elution peak was collected and pH adjusted to ∼pH7 with 2M Tris/HCl pH8.8. The pH adjusted elution was concentrated to 7ml and diafiltered into 20mM phosphate, 150mM NaCl pH7.4 using Amicon Ultra-15 concentrators with a 10kDa molecular weight cut off membrane and centrifugation at 4000xg in a swing out rotor.

### Superdex200 purification

The concentrated and diafiltered protein-G eluate was applied to a XK26/60 Superdex200 (GE Healthcare) column equilibrated in 20mM phosphate, 150mM NaCl pH7.4. The column was developed with an isocratic gradient of 20mM phosphate, 150mM NaCl pH7.4 at 30cm/hr. 5ml fractions were collected and analysed by Superdex200 10/300GL Tricon column (GE Healthcare) developed with an isocratic gradient of 20mM phosphate, 150mM NaCl pH7.4 at 1ml/min. Fractions containing only monomer were pooled and concentrated to ∼10mg/ml using an Amicon Ultra-15 concentrator with a 10kDa molecular weight cut off membrane and centrifugation at 4000xg in a swing out rotor.

### SDS-PAGE analysis of FabB-645dsFv (3xG4S)

FabB-645dsFv (3xG4S) was diluted to 0.32mg/ml with PBS and to 26µl was added 10µL 4X LDS (Invitrogen) sample running buffer. For non-reduced samples, 4µL of 100mM NEM was added and for reduced samples 4µL of 10X reducing agent (Invitrogen) was added. The samples were vortexed, incubated at 100°C for 3 mins, cooled and centrifuged at 12500 rpm for 30secs. The prepared samples were loaded, 10µl / 2µg, on to a 4-20% acrylamine Tris/Glycine SDS gel and run for 110mins at 125V. The gels were stained with Coomassie Blue protein stain and destained with 7.5% acetic acid. See Figure 25. Under both reducing and non-reducing conditions the FabB-645dsFv (3xG4S) is essentially one band. The 2 minor bands above and below the main band on the non-reduced gel are where one or other of the interchain disulphide bonds have not formed. The 1 minor band above the main band on the reduced gel is non-reducible FabB-645dsFv (3xG4S). See Figure 25.

### Size exclusion analysis of FabB-645dsFv (3xG4S)

FabB-645dsFv (3xG4S) was diluted to 0.5mg/ml with PBS. 100µl of this was injected onto a Superdex200 10/300GL Tricon column (GE Healthcare) and developed with an isocratic gradient of PBS at 1ml/min. Peaks were detected by absorbance at 280nm and 214nm. See Figure 26. There is a single, symmetrical peak in the chromatogram with a retention time of 13.44 metric minutes. This peak retention time was converted to an apparent molecular weight using a standard curve created from the retention times of BioRad gel filtration standards (151-1901) run under the same conditions. The apparent molecular weight of the FabB-645dsFv (3xG4S) was 87kDa.

### Thermal stability analysis of FabB-645dsFv (3xG4S)

To measure the thermal stability, FabB-645dsFv (3xG4S) was diluted to 1mg/ml with PBS. In quadruplicate, to 1µl of this diluted sample was added 8µl of PBS and 1µl of 30x stock of Sypro orange fluorescent dye in a 384 well plate. The plate was heated from 20-99°C using a 7900HT fast real-time PCR system and the fluorescence (excitation at 490nm, emission at 530nm) measured. See Figure 27A. The FabB-645dsFv (3xG4S) is a thermally stable molecule with a Tm in excess of 70°C.

To measure the thermal stability over a range of pHs, FabB-645dsFv (3xG4S) at 10mg/ml was diluted to 0.11mg/ml with buffers at pH2.2 - 8.0 in 0.2 increments. The pH buffers were prepared by mixing 0.1M citric acid and 0.2M disodium hydrogen phosphate and adding NaCl to equalize for ionic strength. To 45µl of each pH diluted sample was added 5µl of 30x stock of Sypro orange fluorescent dye. 10µl aliquots of these were analysed in quadruplicate in 384 well plates. The plate is heated from 20-99°C using a 7900HT fast real-time PCR system and the fluorescence (excitation at 490nm, emission at 530nm) measured. The Tm was then plotted against pH. See Figure 27B. Both the gA26Fab and 645dsFv domains of FabB-645dsFv (3xG4S) have Tms which are largely unaffected by pH over the range pH4.5-8.0. Below pH 4.5 the Tm of both domains decreases until at pH4.0 the 2 separate unfolding events can't be distinguished, this single event has a Tm of 65°C. The Tm of this undistinguished single unfolding event continues to decrease with decreasing pH, but is still above 50°C at pH 2.2.

### In vitro efficacy of FabB-645dsFv (3xG4S)

The in vitro efficacy of FabB-645dsFv (3xG4S) was assessed by a cell based OX40 ligand blocking assay. Briefly, human PBMC were isolated and activated by incubation with 5µg/ml PHA-L (phytohaemagglutinin-L) for 24-72 hours at 37°C/5% CO₂. The cells were then washed in PBS/0.09% sodium azide and plated at 0.25x10⁶ cells/well of a 96 well culture plate. Dilutions of FabB-645dsFv (3xG4S) were prepared in PBS/5% HSA. A solution of 4µg/ml biotinylated CD252-CD8 fusion protein was also prepared in PBS/5% HSA. 50µl of each FabB-645dsFv (3xG4S) dilution was added to 50µl of CD252-CD8 fusion protein and the mixture incubated with the activated T-cells for 30 minutes at 4oC. Following this incubation, the cells were washed in PBS/0.09% sodium azide. The activated T-cells were then incubated with 100µl of streptavidin-PE in PBS for 30 minutes at 4oC. The cells were again washed in PBS/0.09% sodium azide and then re-suspended in buffer and analysed by flow cytometry. FabB-645dsFv (3xG4S) was shown to block the binding of OX40 ligand to OX40 expressed on the surface of human PBMC's with and EC50 of ∼3.5nM, see Figure 28.

### In vivo efficacy of FabB-645dsFv (3xG4S)

To study the dose response relationship in vivo, a study undertaken with FabB-645dsFv (3xG4S). Briefly this involved steady state dosing at 0.3, 3 and 30µg/ml in HuSCID mice with a read out of prevention of T cell engraftment.

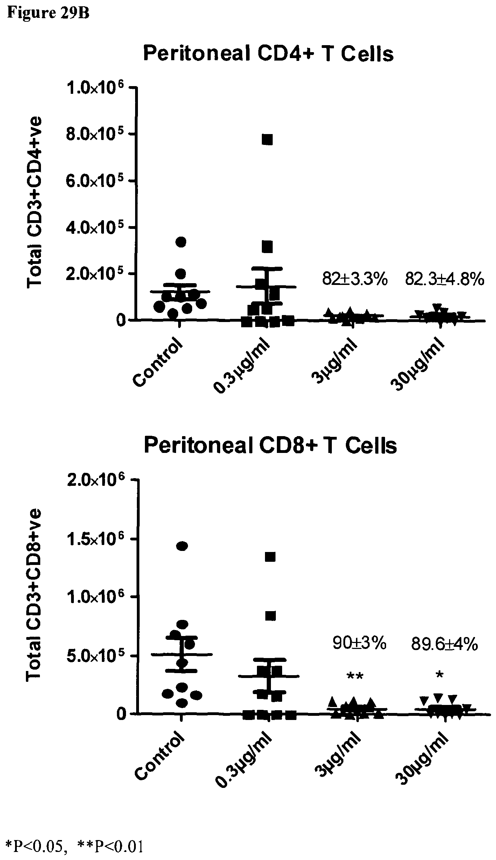

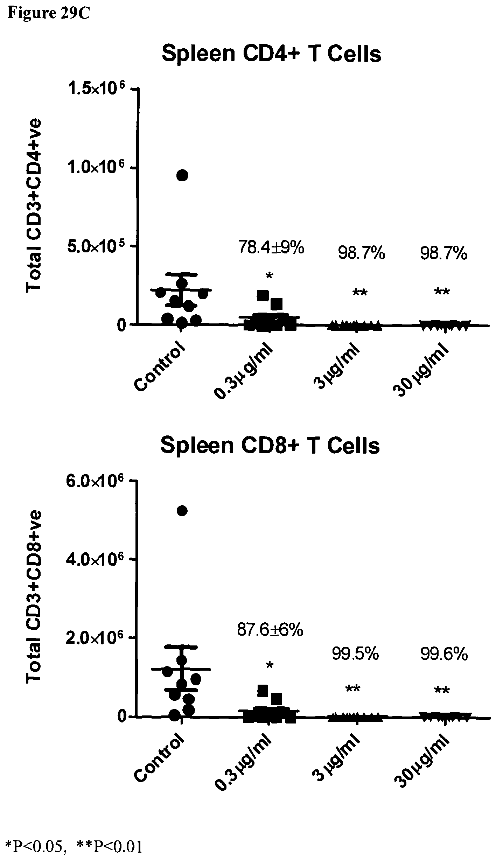

CB17 SCID mice were dosed with a loading dose subcutaneously on day -2 of 2.475mg/kg or 0.2475mg/kg or 0.02475mg/kg FabB-645dsFv (3xG4S) PBS. On every subsequent day up to and including day 14 they were dosed with a maintenance dose subcutaneously of 0.75mg/kg or 0.075mg/kg or 0.0075mg/kg FabB-645dsFv (3xG4S) or PBS. Each dosing group consisted of 9-10 mice. On day -1 all the mice were treated with 0.87mg/mouse of rat anti-murine TM-β1 antibody to abrogate natural killer cell activity. On day 0 all the mice received an inter peritoneal injection of 8x106 human peripheral blood mononuclear cells. On day 14 the mice are sacrificed and the blood, spleen and a peritoneal lavage were taken. The samples were analysed by FACS for CD4+ and CD8+ T cells. The data sets were analysed by one way Anova with Dunnett's post test comparison. See Figures 29A, B and C. The 30 and 3µg/ml dosings were equally efficacious in all compartments, where as the 0.3µg/ml dosing was statistically efficacious in the blood and spleen but not to the maximal level produced by the 30 and 3µg/ml dosing.

### COMPARATIVE DATA E22

### Construction, expression and antigen binding of 645Fv-652Fabs

### Construction of 645Fv-652Fab plasmids

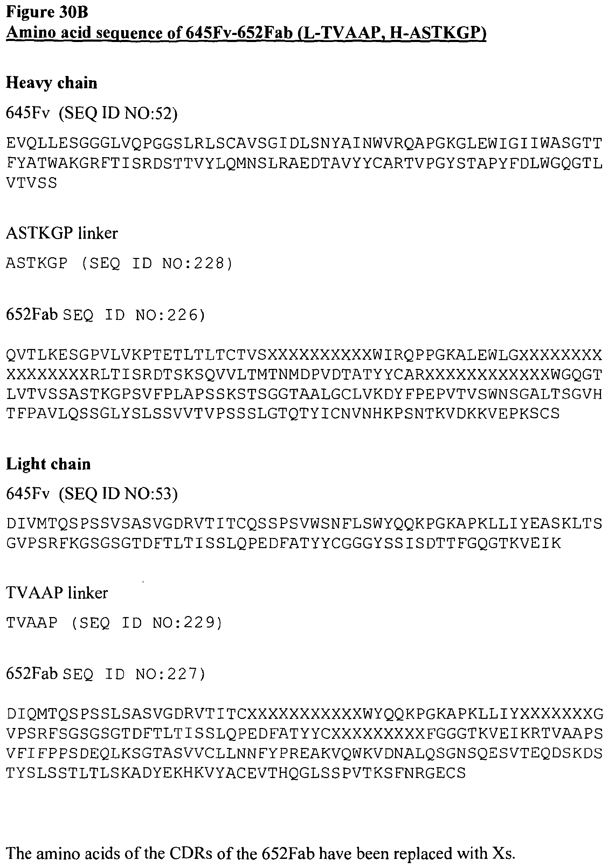

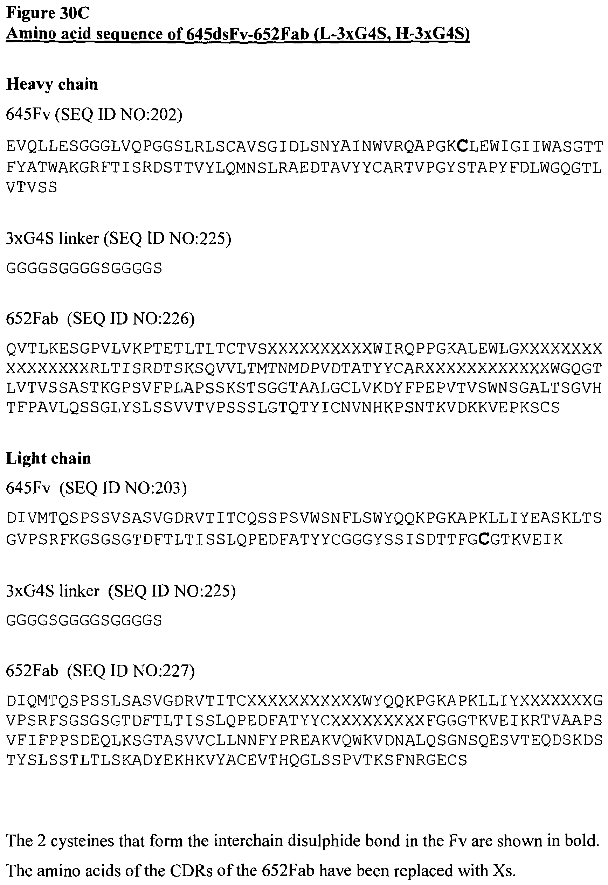

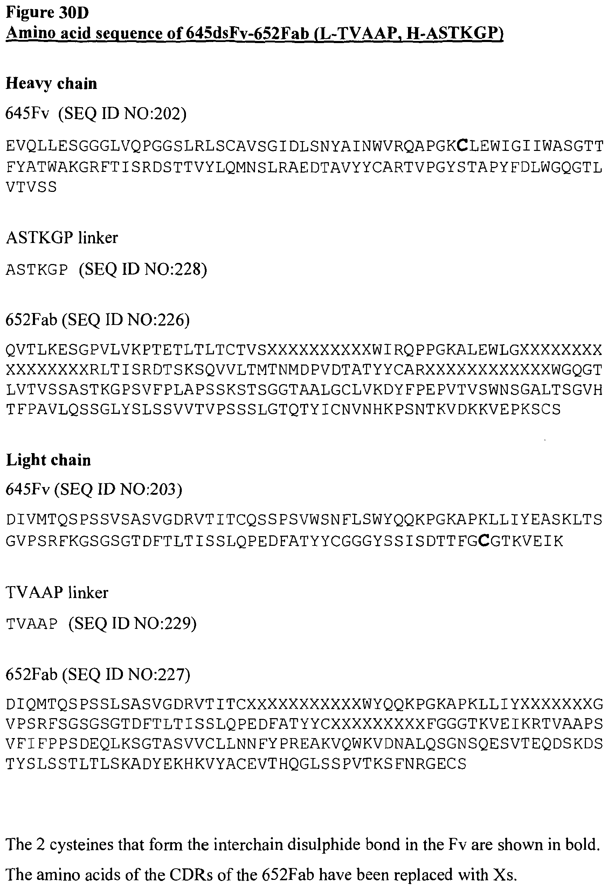

The total gene synthesis of 645Fv-652Fab (L-3xG4S, H-3xG4S), 645Fv-652Fab (L-TVAAP, H-ASTKGP), 645dsFv-652Fab (L-3xG4S, H-3xG4S), 645dsFv-652Fab (L-TVAAP, H-ASTKGP) was done by a third party contractor (DNA2.0). See Figures 30A, B, C and D for the amino acid sequence of the 645Fv-652Fabs. All the genes were cloned into UCB's proprietary mammalian expression vector under the control of the HCMV-MIE promoter and SV40E polyA sequence.

### Mammalian expression of 645Fv-652Fabs

HEK293 cells were transfected with the heavy and light chain plasmids using Invitrogen's 293fectin transfection reagent according to the manufacturer's instructions. Briefly, 2µg heavy chain plasmid and 2µg light chain plasmid were incubated with 10µl 293fectin and 340µl Optimem media for 20mins at RT. The mixture was then added to 5x10⁶ HEK293 cells in suspension and incubated for 4 days with shaking at 37°C. After 4 days the supernatant was collected by centrifugation at 1500xg to remove the cells and then 0.22µm sterile filtered.

### 645Fv-652Fab quantification

The concentration of Fv-Fab in the mammalian supernatants was measured using a sandwich ELISA. The Fv-Fab in the sample was captured with an anti-CHI antibody and detected with an anti-kappa-HRP conjugate. The detection antibody was developed with TMB and the concentration of the unknown samples calculated from a standard curve. All the 645Fv-652Fabs had similar expression levels but the disulphide stabilised Fv versions were expressed at 55%-75% of the level of the non-disulphide stabilised versions, see Figure 31.

### Antigen binding of 645Fv-652Fab

### Biacore method

Kinetic constants and binding responses for the interactions of Fv-Fab constructs were determined by surface plasmon resonance (SPR) conducted on a Biacore 3000 using CM5 sensor chips. The running buffer, HBS-EP consisted of 10mM HEPES, 150mM NaCl, 3mM EDTA, 0.05% v/v surfactant P20 at pH7.4. Samples were captured on the sensor chip surface using an in-house generated anti human CH1 monoclonal antibody. Covalent immobilisation of the capture antibody was achieved by standard amine coupling chemistry.

An assay cycle consisted of capturing the Fv-Fab construct for 1 min, followed by an association phase (3 min for HSA or 6 min for hIL13) after which dissociation was monitored for 5 min (HSA) or 20min (hIL13). After each cycle, the capture surface was regenerated with 2 x 1 min injections of 40mM HCl followed by 30s of 5mM NaOH. The flow rates used were 10µl/min for capture, 30µl/min for the association and dissociation phases, and 10µl/min for regeneration.

Kinetic assays were performed by titration of antigen (for HSA doubling dilutions from 50nM-0.3125nM, for hIL13 single concentration-20nM). A blank flow-cell and buffer-blank injections enabled double referencing of the data.

Kinetic parameters were determined by simultaneous global-fitting of the resulting sensorgrams to a standard 1:1 binding model using Biacore 3000, 4.1 Evaluation software.

In order to test for simultaneous binding, 6 min injections of separate 50nM HSA or 20nM hIL13, or a mixed solution of 50nM HSA and 20nM hIL13 were injected over the captured FvFab.

### Biacore affinity experiments

Kinetic analysis was conducted to assess the affinity of the interactions of HSA and hIL13 to 645Fv-652Fab (L-3xG4S, H-3xG4S), 645Fv-652Fab (L-TVAAP, H-ASTKGP), 645dsFv-652Fab (L-3xG4S, H-3xG4S) and 645dsFv-652Fab (L-TVAAP, H-ASTKGP), see Figure 30A and 30B. All Fv-Fabs bound HSA with equivalent affinity and binding level. 645Fv-652Fab (L-3xG4S, H-3xG4S) and 645dsFv-652Fab (L-3xG4S, H-3xG4S) bind hIL13 with an affinity of ∼0.1nM whereas 645Fv-652Fab (L-TVAAP, H-ASTKGP) and 645dsFv-652Fab (L-TVAAP, H-ASTKGP) bind with an affinity of ∼0.6nM. The difference in affinity of hIL13 for the Fv-Fabs with the 3xG4S linker compared to the TVAAP/ASTKGP linker is primarily in the association rate. The affinity of hIL13 and HSA for the disulphide stabilized and non-disulphide stabilized Fv-Fabs were equivalent. The binding levels of ML13 to all constructs are also equivalent.

The simultaneous binding of Fv-Fabs 645Fv-652Fab (L-3xG4S, H-3xG4S), 645Fv-652Fab (L-TVAAP, H-ASTKGP), 645dsFv-652Fab (L-3xG4S, H-3xG4S) and 645dsFv-652Fab (L-TVAAP, H-ASTKGP) to both HSA and hIL13 was assessed. Each Fv-Fab construct was captured to the sensor chip surface, followed by separate 6 min injections of 50nM HSA or 20nM hIL13, or a mixed solution of both 50nM HSA and 20nM hIL13. For each Fv-Fab construct the binding response for the combined HSA/hIL13 solution was equivalent to the sum of the responses of the independent injections, see Figure 32C. This demonstrates that the Fv-Fabs are capable of simultaneous binding to both hIL13 and HSA.

Figure 32C shows the binding response (RU) seen for each construct after separate injections of HSA or hIL13, or injection of premixed HSA and hIL13. In each case the final concentration was 50nM HSA and 20nM hIL13. The sum of the individual HSA and hIL13 responses is shown in parentheses.

## Claims

1. A multivalent antibody fusion protein consisting of a Fab or Fab' fragment, with a first specificity for an antigen of interest, and two single domain antibodies (dAb) which are a VH/VL pair with specificity for a second antigen of interest, wherein the two single domain antibodies are linked by a disulfide bond between two cysteine residues, one in VH and one in VL, wherein the position of the two cysteine residues is VH44 and VL100 according to Kabat numbering, and wherein the V_{H} dAb is directly or indirectly connected to the C-terminus of the Fab or Fab' heavy chain by genetic fusion and the V_{L} dAb is directly or indirectly connected to the C-terminus of the Fab or Fab' light chain by genetic fusion.

2. A multivalent antibody fusion protein according to claim 1 wherein the two single domain antibodies are a complementary VH/VL pair which bind the second antigen cooperatively.

3. A multivalent antibody fusion protein according to any one of claims 1-2, wherein the first antigen and second antigen are different entities.

4. A multivalent antibody fusion protein according to any one of claims 1-3 wherein the VH and/or the VL domain is linked to the Fab or Fab' fragment via a linker having the sequence given in SEQ ID NO:224 or SEQ ID NO:225.

5. A multivalent antibody fusion protein according to any one of claims 1 to 4, wherein the second antigen is albumin.

6. A multivalent antibody fusion protein according to any one of claims 1 to 5, wherein the second antigen is human serum albumin

7. A multivalent antibody fusion protein according to claim 6 wherein said VH single domain antibody comprises a CDR having the sequence given in Figure 5 (e) SEQ ID NO:56 or Figure 5 (k) SEQ ID NO:62 for CDR-H1, a CDR having the sequence given in Figure 5(f) SEQ ID NO:57 or Figure 5 (l) SEQ ID NO:63 for CDR-H2 and a CDR having the sequence given in Figure 5 (g) SEQ ID NO:58 or Figure 5 (m) SEQ ID NO:64 for CDR-H3.

8. A multivalent antibody fusion protein according to claim 6 wherein said VL single domain antibody comprises a CDR having the sequence given in Figure 5 (h) SEQ ID NO:59 or Figure 5 (n) SEQ ID NO:65 for CDR-L1, a CDR having the sequence given in Figure 5(i) SEQ ID NO:60 or Figure 5 (o) SEQ ID NO:66 for CDR-L2 and a CDR having the sequence given in Figure 5 (j) SEQ ID NO:61 or Figure 5 (p) SEQ ID NO:67 for CDR-L3.

9. A multivalent antibody fusion protein according to claim 6 wherein the VH single domain antibody comprises the sequence given in SEQ ID NO:202 and the VL single domain antibody comprises the sequence given in SEQ ID NO:203.

10. A multivalent antibody fusion protein according to claim 6 wherein the VH single domain antibody comprises the sequence given in SEQ ID NO:204 and the VL single domain antibody comprises the sequence given in SEQ ID NO:205.

## Patentansprüche

1. Multivalentes Antikörper-Fusionsprotein, bestehend aus einem Fab- oder Fab'-Fragment, mit einer ersten Spezifität für ein interessierendes Antigen, und zwei Einzeldomäne-Antikörpern (dAb), bei denen es sich um ein VH/VL-Paar mit Spezifität für ein zweites interessierendes Antigen handelt, wobei die beiden Einzeldomäne-Antikörper über eine Disulfidbindung zwischen zwei Cysteinresten, einem in VH und einem in VL, verknüpft sind, wobei die Position der beiden Cysteinreste nach Kabat-Nummerierung bei VH44 bzw. VL100 liegt und wobei der V_{H}-dAb durch genetische Fusion direkt oder indirekt mit dem C-Terminus der Fab- oder Fab'-schwere-Kette und der V_{L}-dAb durch genetische Fusion direkt oder indirekt mit dem C-Terminus der Fab- oder Fab'-leichte-Kette verbunden ist.

2. Multivalentes Antikörper-Fusionsprotein nach Anspruch 1, wobei es sich bei den beiden Einzeldomäne-Antikörpern um ein komplementäres VH/VL-Paar handelt, die das zweite Antigen kooperativ binden.

3. Multivalentes Antikörper-Fusionsprotein nach einem der Ansprüche 1-2, wobei es sich bei dem ersten Antigen und dem zweiten Antigen um unterschiedliche Gebilde handelt.

4. Multivalentes Antikörper-Fusionsprotein nach einem der Ansprüche 1-3, wobei die VH- und/oder die VL-Domäne mit dem Fab- oder Fab'-Fragment über einen Linker mit der Sequenz gemäß SEQ ID NO:224 oder SEQ ID NO:225 verknüpft ist.

5. Multivalentes Antikörper-Fusionsprotein nach einem der Ansprüche 1 bis 4, wobei es sich bei dem zweiten Antigen um Albumin handelt.

6. Multivalentes Antikörper-Fusionsprotein nach einem der Ansprüche 1 bis 5, wobei es sich bei dem zweiten Antigen um menschliches Serumalbumin handelt.

7. Multivalentes Antikörper-Fusionsprotein nach Anspruch 6, wobei der VH-Einzeldomäne-Antikörper eine CDR mit der Sequenz gemäß Figur 5 (e) SEQ ID NO:56 oder Figur 5 (k) SEQ ID NO:62 für CDR-H1, eine CDR mit der Sequenz gemäß Figur 5 (f) SEQ ID NO:57 oder Figur 5 (l) SEQ ID NO:63 für CDR-H2 und eine CDR mit der Sequenz gemäß Figur 5 (g) SEQ ID NO.58 oder Figur 5 (m) SEQ ID NO:64 für CDR-H3 umfasst.

8. Multivalentes Antikörper-Fusionsprotein nach Anspruch 6, wobei der VL-Einzeldomäne-Antikörper eine CDR mit der Sequenz gemäß Figur 5 (h) SEQ ID NO:59 oder Figur 5 (n) SEQ ID NO:65 für CDR-L1, eine CDR mit der Sequenz gemäß Figur 5 (i) SEQ ID NO:60 oder Figur 5 (o) SEQ ID NO:66 für CDR-L2 und eine CDR mit der Sequenz gemäß Figur 5 (j) SEQ ID NO:61 oder Figur 5 (p) SEQ ID NO:67 für CDR-L3 umfasst.

9. Multivalentes Antikörper-Fusionsprotein nach Anspruch 6, wobei der VH-Einzeldomäne-Antikörper die Sequenz gemäß SEQ ID NO:202 und der VL-Einzeldomäne-Antikörper die Sequenz gemäß SEQ ID NO:203 umfasst.

10. Multivalentes Antikörper-Fusionsprotein nach Anspruch 6, wobei der VH-Einzeldomäne-Antikörper die Sequenz gemäß SEQ ID NO:204 und der VL-Einzeldomäne-Antikörper die Sequenz gemäß SEQ ID NO:205 umfasst.

## Revendications

1. Protéine de fusion d'anticorps multivalent constitué d'un fragment Fab ou Fab', ayant une première spécificité pour un antigène d'intérêt, et deux anticorps à domaine unique (dAb) qui sont une paire VH/VL ayant une spécificité pour un deuxième antigène d'intérêt, dans laquelle les deux anticorps à domaine unique sont liés par une liaison disulfure entre deux résidus cystéine, un dans VH et un dans VL, dans laquelle la position des deux résidus cystéine est VH44 et VL100 selon la numérotation de Kabat, et dans laquelle le dAb VH est directement ou indirectement relié à l'extrémité C-terminale de la chaîne lourde Fab ou Fab' par fusion génétique et le dAb VL est directement ou indirectement relié à l'extrémité C-terminale de la chaîne légère Fab ou Fab' par fusion génétique.

2. Protéine de fusion d'anticorps multivalent selon la revendication 1, dans laquelle les deux anticorps à domaine unique sont une paire VH/VL complémentaire qui se lient au deuxième antigène de façon coopérative.

3. Protéine de fusion d'anticorps multivalent selon l'une quelconque des revendications 1 à 2, dans laquelle le premier antigène et le deuxième antigène sont des entités différentes.

4. Protéine de fusion d'anticorps multivalent selon l'une quelconque des revendications 1 à 3, dans laquelle le domaine VH et/ou VL est lié au fragment Fab ou Fab' par l'intermédiaire d'un lieur ayant la séquence décrite dans SEQ ID NO: 224 ou SEQ ID NO: 225.

5. Protéine de fusion d'anticorps multivalent selon l'une quelconque des revendications 1 à 4, dans laquelle le deuxième antigène est l'albumine.

6. Protéine de fusion d'anticorps multivalent selon l'une quelconque des revendications 1 à 5, dans laquelle le deuxième antigène est la sérum-albumine humaine.

7. Protéine de fusion d'anticorps multivalent selon la revendication 6, dans laquelle ledit anticorps à domaine unique VH comprend une CDR ayant la séquence décrite sur la figure 5 (e) SEQ ID NO: 56 ou la figure 5 (k) SEQ ID NO: 62 pour CDR-H1, une CDR ayant la séquence décrite sur la figure 5 (f) SEQ ID NO: 57 ou la figure 5 (l) SEQ ID NO: 63 pour CDR-H2 et une CDR ayant la séquence décrite dans la figure 5(g) SEQ ID NO: 58 ou la figure 5 (m) SEQ ID NO: 64 pour CDR-H3.

8. Protéine de fusion d'anticorps multivalent selon la revendication 6, dans laquelle ledit anticorps à domaine unique VL comprend une CDR ayant la séquence décrite sur la figure 5 (h) SEQ ID NO: 59 ou la figure 5 (n) SEQ ID NO: 65 pour CDR-L1, une CDR ayant la séquence décrite sur la figure 5 (i) SEQ ID NO: 60 ou la figure 5 (o) SEQ ID NO: 66 pour CDR-L2 et une CDR ayant la séquence décrite sur la figure 5 (j) SEQ ID NO: 61 ou la figure 5 (p) SEQ ID NO: 67 pour CDR-L3.

9. Protéine de fusion d'anticorps multivalent selon la revendication 6, dans laquelle l'anticorps à domaine unique VH comprend la séquence décrite dans SEQ ID NO: 202 et l'anticorps à domaine unique VL comprend la séquence décrite dans SEQ ID NO: 203.

10. Protéine de fusion d'anticorps multivalent selon la revendication 6, dans laquelle l'anticorps à domaine unique VH comprend la séquence décrite dans SEQ ID NO: 204 et l'anticorps à domaine unique VL comprend la séquence décrite dans SEQ ID NO: 205.


### SEQUENCE LISTING

<110> UCB Pharma SA
<120> Disulphide Stabilised Multivalent Antibodies
<130> G0103_WO01
<160> 229
<170> PatentIn version 3.5
<210> 1
   <211> 7
   <212> PRT
   <213> Artificial
<220>
   <223> Linker
<400> 1
   Ser Gly Gly Gly Gly Ser Glu
   1 5
<210> 2
   <211> 6
   <212> PRT
   <213> Artificial
<220>
   <223> Linker
<400> 2
   Asp Lys Thr His Thr Ser
   1 5
<210> 3
   <211> 6
   <212> PRT
   <213> Artificial
<220>
   <223> Linker
<400> 3
   Ser Gly Gly Gly Gly Ser
   1 5
<210> 4
   <211> 11
   <212> PRT
   <213> Artificial
<220>
   <223> Linker
<400> 4
   Ala Ala Ala Gly Ser Gly Gly Ala Ser Ala Ser
   1 5 10
<210> 5
   <211> 16
   <212> PRT
   <213> Artificial
<220>
   <223> Linker
<220>
   <221> misc_feature
   <222> (7)..(7)
   <223> xaa can be any naturally occurring amino acid
<400> 5
   Ala Ala Ala Gly Ser Gly Xaa Gly Gly Gly Ser Gly Ala Ser Ala Ser
   1 5 10 15
<210> 6
   <211> 13
   <212> PRT
   <213> Artificial
<220>
   <223> Linker
<220>
   <221> misc_feature
   <222> (7)..(7)
   <223> xaa can be any naturally occurring amino acid
<400> 6
   Ala Ala Ala Gly Ser Gly Xaa Ser Gly Ala Ser Ala Ser
   1 5 10
<210> 7
   <211> 28
   <212> PRT
   <213> Artificial
<220>
   <223> Linker
<400> 7
   Pro Gly Gly Asn Arg Gly Thr Thr Thr Thr Arg Arg Pro Ala Thr Thr
   1 5 10 15
   Thr Gly Ser Ser Pro Gly Pro Thr Gln Ser His Tyr
   20 25
<210> 8
   <211> 11
   <212> PRT
   <213> Artificial
<220>
   <223> Linker
<400> 8
   Ala Thr Thr Thr Gly Ser Ser Pro Gly Pro Thr
   1 5 10
<210> 9
   <211> 6
   <212> PRT
   <213> Artificial
<220>
   <223> Linker
<400> 9
   Ala Thr Thr Thr Gly Ser
   1 5
<210> 10
   <211> 21
   <212> PRT
   <213> Artificial
<220>
   <223> Linker
<400> 10
   Glu Pro Ser Gly Pro Ile Ser Thr Ile Asn Ser Pro Pro Ser Lys Glu
   1 5 10 15
   Ser His Lys Ser Pro
   20
<210> 11
   <211> 15
   <212> PRT
   <213> Artificial
<220>
   <223> Linker
<400> 11
   Gly Thr Val Ala Ala Pro Ser Val Phe Ile Phe Pro Pro Ser Asp
   1 5 10 15
<210> 12
   <211> 15
   <212> PRT
   <213> Artificial
<220>
   <223> Linker
<400> 12
   Gly Gly Gly Gly Ile Ala Pro Ser Met Val Gly Gly Gly Gly Ser
   1 5 10 15
<210> 13
   <211> 15
   <212> PRT
   <213> Artificial
<220>
   <223> Linker
<400> 13
   Gly Gly Gly Gly Lys Val Glu Gly Ala Gly Gly Gly Gly Gly Ser
   1               5                   10                  15
<210> 14
   <211> 15
   <212> PRT
   <213> Artificial
<220>
   <223> Linker
<400> 14
   Gly Gly Gly Gly Ser Met Lys Ser His Asp Gly Gly Gly Gly Ser
   1               5                   10                  15
<210> 15
   <211> 15
   <212> PRT
   <213> Artificial
<220>
   <223> Linker
<400> 15
   Gly Gly Gly Gly Asn Leu Ile Thr Ile Val Gly Gly Gly Gly Ser
   1               5                   10                  15
<210> 16
   <211> 15
   <212> PRT
   <213> Artificial
<220>
   <223> Linker
<400> 16
   Gly Gly Gly Gly Val Val Pro Ser Leu Pro Gly Gly Gly Gly Ser
   1               5                   10                  15
<210> 17
   <211> 12
   <212> PRT
   <213> Artificial
<220>
   <223> Linker
<400> 17
   Gly Gly Glu Lys Ser Ile Pro Gly Gly Gly Gly Ser
   1 5 10
<210> 18
   <211> 18
   <212> PRT
   <213> Artificial
<220>
   <223> Linker
<400> 18
   Arg Pro Leu Ser Tyr Arg Pro Pro Phe Pro Phe Gly Phe Pro Ser Val
   1 5 10 15
   Arg Pro
<210> 19
   <211> 18
   <212> PRT
   <213> Artificial
<220>
   <223> Linker
<400> 19
   Tyr Pro Arg Ser Ile Tyr Ile Arg Arg Arg His Pro Ser Pro Ser Leu
   1 5 10 15
   Thr Thr
<210> 20
   <211> 18
   <212> PRT
   <213> Artificial
<220>
   <223> Linker
<400> 20
   Thr Pro Ser His Leu Ser His Ile Leu Pro Ser Phe Gly Leu Pro Thr
   1 5 10 15
   Phe Asn
<210> 21
   <211> 18
   <212> PRT
   <213> Artificial
<220>
   <223> Linker
<400> 21
   Arg Pro Val Ser Pro Phe Thr Phe Pro Arg Leu Ser Asn Ser Trp Leu
   1               5                   10                  15
   Pro Ala
<210> 22
   <211> 18
   <212> PRT
   <213> Artificial
<220>
   <223> Linker
<400> 22
   Ser Pro Ala Ala His Phe Pro Arg Ser Ile Pro Arg Pro Gly Pro Ile
   1               5                   10                  15
   Arg Thr
<210> 23
   <211> 18
   <212> PRT
   <213> Artificial
<220>
   <223> Linker
<400> 23
   Ala Pro Gly Pro Ser Ala Pro Ser His Arg Ser Leu Pro Ser Arg Ala
   1               5                   10                  15
   Phe Gly
<210> 24
   <211> 18
   <212> PRT
   <213> Artificial
<220>
   <223> Linker
<400> 24
   Pro Arg Asn Ser Ile His Phe Leu His Pro Leu Leu Val Ala Pro Leu
   1               5                   10                  15
   Gly Ala
<210> 25
   <211> 18
   <212> PRT
   <213> Artificial
<220>
   <223> Linker
<400> 25
   Met Pro Ser Leu Ser Gly Val Leu Gln Val Arg Tyr Leu Ser Pro Pro
   1               5                   10                  15
   Asp Leu
<210> 26
   <211> 18
   <212> PRT
   <213> Artificial
<220>
   <223> Linker
<400> 26
   Ser Pro Gln Tyr Pro Ser Pro Leu Thr Leu Thr Leu Pro Pro His Pro
   1               5                   10                  15
   Ser Leu
<210> 27
   <211> 18
   <212> PRT
   <213> Artificial
<220>
   <223> Linker
<400> 27
   Asn Pro Ser Leu Asn Pro Pro Ser Tyr Leu His Arg Ala Pro Ser Arg
   1               5                   10                  15
   Ile Ser
<210> 28
   <211> 17
   <212> PRT
   <213> Artificial
<220>
   <223> Linker
<400> 28
   Leu Pro Trp Arg Thr Ser Leu Leu Pro Ser Leu Pro Leu Arg Arg Arg
   1 5 10 15
   Pro
<210> 29
   <211> 18
   <212> PRT
   <213> Artificial
<220>
   <223> Linker
<400> 29
   Pro Pro Leu Phe Ala Lys Gly Pro Val Gly Leu Leu Ser Arg Ser Phe
   1 5 10 15
   Pro Pro
<210> 30
   <211> 18
   <212> PRT
   <213> Artificial
<220>
   <223> Linker
<400> 30
   Val Pro Pro Ala Pro Val Val Ser Leu Arg Ser Ala His Ala Arg Pro
   1 5 10 15
   Pro Tyr
<210> 31
   <211> 17
   <212> PRT
   <213> Artificial
<220>
   <223> Linker
<400> 31
   Leu Arg Pro Thr Pro Pro Arg Val Arg Ser Tyr Thr Cys Cys Pro Thr
   1 5 10 15
   Pro
<210> 32
   <211> 18
   <212> PRT
   <213> Artificial
<220>
   <223> Linker
<400> 32
   Pro Asn Val Ala His Val Leu Pro Leu Leu Thr Val Pro Trp Asp Asn
   1 5 10 15
   Leu Arg
<210> 33
   <211> 18
   <212> PRT
   <213> Artificial
<220>
   <223> Linker
<400> 33
   Cys Asn Pro Leu Leu Pro Leu Cys Ala Arg Ser Pro Ala Val Arg Thr
   1 5 10 15
   Phe Pro
<210> 34
   <211> 11
   <212> PRT
   <213> Artificial
<220>
   <223> Linker
<400> 34
   Gly Ala Pro Ala Pro Ala Ala Pro Ala Pro Ala
   1 5 10
<210> 35
   <211> 4
   <212> PRT
   <213> Artificial
<220>
   <223> Linker
<400> 35
   Pro Pro Pro Pro
   1
<210> 36
   <211> 8
   <212> PRT
   <213> Artificial
<220>
   <223> Hinge
<400> 36
   Asp Lys Thr His Thr Cys Ala Ala
   1 5
<210> 37
   <211> 11
   <212> PRT
   <213> Artificial
<220>
   <223> Linker
<400> 37
   Asp Lys Thr His Thr Cys Pro Pro Cys Pro Ala
   1 5 10
<210> 38
   <211> 18
   <212> PRT
   <213> Artificial
<220>
   <223> Hinge
<400> 38
   Asp Lys Thr His Thr Cys Pro Pro Cys Pro Ala Thr Cys Pro Pro Cys
   1 5 10 15
   Pro Ala
<210> 39
   <211> 25
   <212> PRT
   <213> Artificial
<220>
   <223> Linker
<400> 39
   Asp Lys Thr His Thr Cys Pro Pro Cys Pro Ala Thr Cys Pro Pro Cys
   1               5                   10                  15
   Pro Ala Thr Cys Pro Pro Cys Pro Ala
               20                  25
<210> 40
   <211> 30
   <212> PRT
   <213> Artificial
<220>
   <223> Hinge
<400> 40
   Asp Lys Thr His Thr Cys Pro Pro Cys Pro Ala Gly Lys Pro Thr Leu
   1               5                   10                  15
   Tyr Asn Ser Leu Val Met Ser Asp Thr Ala Gly Thr Cys Tyr
               20                  25                  30
<210> 41
   <211> 31
   <212> PRT
   <213> Artificial
<220>
   <223> Hinge
<400> 41
   Asp Lys Thr His Thr Cys Pro Pro Cys Pro Ala Gly Lys Pro Thr His
   1               5                   10                  15
   Val Asn Val Ser Val Val Met Ala Glu Val Asp Gly Thr Cys Tyr
               20                  25                  30
<210> 42
   <211> 15
   <212> PRT
   <213> Artificial
<220>
   <223> Linker
<400> 42
   Asp Lys Thr His Thr Cys Cys Val Glu Cys Pro Pro Cys Pro Ala
   1               5                   10                  15
<210> 43
   <211> 26
   <212> PRT
   <213> Artificial
<220>
   <223> Linker
<400> 43
   Asp Lys Thr His Thr Cys Pro Arg Cys Pro Glu Pro Lys Ser Cys Asp
   1 5 10 15
   Thr Pro Pro Pro Cys Pro Arg Cys Pro Ala
   20 25
<210> 44
   <211> 11
   <212> PRT
   <213> Artificial
<220>
   <223> Linker
<400> 44
   Asp Lys Thr His Thr Cys Pro Ser Cys Pro Ala
   1 5 10
<210> 45
   <211> 11
   <212> PRT
   <213> Artificial
<220>
   <223> Linker
<400> 45
   Ser Gly Gly Gly Gly Ser Gly Gly Gly Gly Ser
   1 5 10
<210> 46
   <211> 16
   <212> PRT
   <213> Artificial
<220>
   <223> Linker
<400> 46
   Ser Gly Gly Gly Gly Ser Gly Gly Gly Gly Ser Gly Gly Gly Gly Ser
   1 5 10 15
<210> 47
   <211> 21
   <212> PRT
   <213> Artificial
<220>
   <223> Linker
<400> 47
   Ser Gly Gly Gly Gly Ser Gly Gly Gly Gly Ser Gly Gly Gly Gly Ser
   1               5                   10                  15
   Gly Gly Gly Gly Ser
               20
<210> 48
   <211> 26
   <212> PRT
   <213> Artificial
<220>
   <223> Linker
<400> 48
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 49
   <211> 21
   <212> PRT
   <213> Artificial
<220>
   <223> Linker
<220>
   <221> misc_feature
   <222> (7)..(7)
   <223> Xaa can be any naturally occurring amino acid
<220>
   <221> misc_feature
   <222> (12)..(12)
   <223> Xaa can be any naturally occurring amino acid
<400> 49
   Ala Ala Ala Gly Ser Gly Xaa Gly Gly Gly Ser Xaa Gly Gly Gly Ser
   1               5                   10                  15
   Gly Ala Ser Ala Ser
               20
<210> 50
   <211> 26
   <212> PRT
   <213> Artificial
<220>
   <223> Linker
<220>
   <221> misc_feature
   <222> (7)..(7)
   <223> Xaa can be any naturally occurring amino acid
<220>
   <221> misc_feature
   <222> (12)..(12)
   <223> Xaa can be any naturally occurring amino acid
<220>
   <221> misc_feature
   <222> (17)..(17)
   <223> Xaa can be any naturally occurring amino acid
<400> 50
   Ala Ala Ala Gly Ser Gly Xaa Gly Gly Gly Ser Xaa Gly Gly Gly Ser
   1               5                   10                  15
   Xaa Gly Gly Gly Ser Gly Ala Ser Ala Ser
               20                  25
<210> 51
   <211> 31
   <212> PRT
   <213> Artificial
<220>
   <223> Linker
<220>
   <221> misc_feature
   <222> (7)..(7)
   <223> Xaa can be any naturally occurring amino acid
<220>
   <221> misc_feature
   <222> (12)..(12)
   <223> Xaa can be any naturally occurring amino acid
<220>
   <221> misc_feature
   <222> (17)..(17)
   <223> Xaa can be any naturally occurring amino acid
<220>
   <221> misc_feature
   <222> (22)..(22)
   <223> Xaa can be any naturally occurring amino acid
<400> 51
   Ala Ala Ala Gly Ser Gly Xaa Gly Gly Gly Ser Xaa Gly Gly Gly Ser
   1               5                   10                  15
   Xaa Gly Gly Gly Ser Xaa Gly Gly Gly Ser Gly Ala Ser Ala Ser
               20                  25                  30
<210> 52
   <211> 119
   <212> PRT
   <213> Artificial
<220>
   <223> Domain antibody H1
<400> 52
   Glu Val Gln Leu Leu Glu Ser Gly Gly Gly Leu Val Gln Pro Gly Gly
   1               5                   10                  15
   Ser Leu Arg Leu Ser Cys Ala Val Ser Gly Ile Asp Leu Ser Asn Tyr
               20                  25                  30
   Ala Ile Asn Trp Val Arg Gln Ala Pro Gly Lys Gly Leu Glu Trp Ile
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Arg Asp Ser Thr Thr Val Tyr Leu Gln Met
   65                  70                  75                  80
   Asn Ser Leu Arg Ala Glu Asp Thr Ala Val Tyr Tyr Cys Ala Arg Thr
                   85                  90                  95
   Val Pro Gly Tyr Ser Thr Ala Pro Tyr Phe Asp Leu Trp Gly Gln Gly
               100                 105                 110
   Thr Leu Val Thr Val Ser Ser
           115
<210> 53
   <211> 110
   <212> PRT
   <213> Artificial
<220>
   <223> Domain antibody L1
<400> 53
   Asp Ile Val Met Thr Gln Ser Pro Ser Ser Val Ser Ala Ser Val Gly
   1               5                   10                  15
   Asp Arg Val Thr Ile Thr Cys Gln Ser Ser Pro Ser Val Trp Ser Asn
               20                  25                  30
   Phe Leu Ser Trp Tyr Gln Gln Lys Pro Gly Lys Ala Pro Lys Leu Leu
           35                  40                  45
   Ile Tyr Glu Ala Ser Lys Leu Thr Ser Gly Val Pro Ser Arg Phe Lys
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gly Gly Gly Tyr Ser Ser Ile
                   85                  90                  95
   Ser Asp Thr Thr Phe Gly Gly Gly Thr Lys Val Glu Ile Lys
               100                 105                 110
<210> 54
   <211> 120
   <212> PRT
   <213> Artificial
<220>
   <223> Domain antibody H2
<400> 54
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Phe Ser Leu Ser Arg Tyr
               20                  25                  30
   Ala Met Thr Trp Val Arg Gln Ala Pro Gly Lys Gly Leu Glu Trp Ile
           35                  40                  45
   Gly Thr Ile Thr Thr Gly Gly Asn Thr Asn Tyr Ala Asn Trp Ala Lys
       50                  55                  60
   Gly Arg Phe Thr Ile Ser Lys Asp Ser Thr Thr Val Tyr Leu Gln Met
   65                  70                  75                  80
   Asn Ser Leu Arg Ala Glu Asp Thr Ala Val Tyr Tyr Cys Ala Arg Gly
                   85                  90                  95
   Gly Tyr Val Ser Tyr Ala Asp Ala Thr Glu Leu Ser Leu Trp Gly Gln
               100                 105                 110
   Gly Thr Leu Val Thr Val Ser Ser
           115                 120
<210> 55
   <211> 110
   <212> PRT
   <213> Artificial
<220>
   <223> Domain antibody L2
<400> 55
   Asp Ile Val Met Thr Gln Ser Pro Ser Thr Leu Ser Ala Ser Val Gly
   1 5 10 15
   Asp Arg Val Thr Ile Thr Cys Gln Ala Ser Gln Ser Ile Gly Ser Arg
   20 25 30
   Leu Ala Trp Tyr Gln Gln Lys Pro Gly Lys Ala Pro Lys Leu Leu Ile
   35 40 45
   Tyr Tyr Ala Ser Thr Val Ala Ser Gly Val Pro Ser Arg Phe Lys Gly
   50 55 60
   Ser Gly Ser Gly Thr Glu Phe Thr Leu Thr Ile Ser Ser Leu Gln Pro
   65 70 75 80
   Asp Asp Phe Ala Thr Tyr Tyr Cys Gln Ser Tyr Asp Tyr Ser Ser Ser
   85 90 95
   Ser Ser Tyr Ala Phe Gly Gly Gly Thr Lys Val Glu Ile Lys
   100 105 110
<210> 56
   <211> 10
   <212> PRT
   <213> Artificial
<220>
   <223> Domain antibody H1 CDR-H1
<400> 56
   Gly Ile Asp Leu Ser Asn Tyr Ala Ile Asn
   1 5 10
<210> 57
   <211> 16
   <212> PRT
   <213> Artificial
<220>
   <223> Domain antibody H1 CDR-H2
<400> 57
   Ile Ile Trp Ala Ser Gly Thr Thr Phe Tyr Ala Thr Trp Ala Lys Gly
   1 5 10 15
<210> 58
   <211> 13
   <212> PRT
   <213> Artificial
<220>
   <223> Domain antibody H1 CDR-H3
<400> 58
   Thr Val Pro Gly Tyr Ser Thr Ala Pro Tyr Phe Asp Leu
   1 5 10
<210> 59
   <211> 12
   <212> PRT
   <213> Artificial
<220>
   <223> Domain antibody L1 CDR-L1
<400> 59
   Gln Ser Ser Pro Ser Val Trp Ser Asn Phe Leu Ser
   1 5 10
<210> 60
   <211> 7
   <212> PRT
   <213> Artificial
<220>
   <223> Domain antibody L1 CDRL2
<400> 60
   Glu Ala Ser Lys Leu Thr Ser
   1 5
<210> 61
   <211> 11
   <212> PRT
   <213> Artificial
<220>
   <223> Domain antibody L1 CDR-L3
<400> 61
   Gly Gly Gly Tyr Ser Ser Ile Ser Asp Thr Thr
   1 5 10
<210> 62
   <211> 10
   <212> PRT
   <213> Artificial
<220>
   <223> Domain antibody H2 CDR-H1
<400> 62
   Gly Phe Ser Leu Ser Arg Tyr Ala Met Thr
   1               5                   10
<210> 63
   <211> 16
   <212> PRT
   <213> Artificial
<220>
   <223> Domain antibody H2 CDR-H2
<400> 63
   Thr Ile Thr Thr Gly Gly Asn Thr Asn Tyr Ala Asn Trp Ala Lys Gly
   1               5                   10                  15
<210> 64
   <211> 14
   <212> PRT
   <213> Artificial
<220>
   <223> Domain antibody H2 CDR-H3
<400> 64
   Gly Gly Tyr Val Ser Tyr Ala Asp Ala Thr Glu Leu Ser Leu
   1               5                   10
<210> 65
   <211> 11
   <212> PRT
   <213> Artificial
<220>
   <223> Domain antibody L2 CDR-L1
<400> 65
   Gln Ala Ser Gln Ser Ile Gly Ser Arg Leu Ala
   1               5                   10
<210> 66
   <211> 7
   <212> PRT
   <213> Artificial
<220>
   <223> Domain antibody L2 CDR-L2
<400> 66
   Tyr Ala Ser Thr Val Ala Ser
   1 5
<210> 67
   <211> 12
   <212> PRT
   <213> Artificial
<220>
   <223> Domain antibody L2 CDR-L3
<400> 67
   Gln Ser Tyr Asp Tyr Ser Ser Ser Ser Ser Tyr Ala
   1 5 10
<210> 68
   <211> 232
   <212> PRT
   <213> Artificial
<220>
   <223> Fab B-dAbH1 (CH1-G4S4)
<400> 68
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Gly Gly Gly Gly Ser Gly Gly Gly Gly
   100 105 110
   Ser Glu Val Gln Leu Leu Glu Ser Gly Gly Gly Leu Val Gln Pro Gly
   115 120 125
   Gly Ser Leu Arg Leu Ser Cys Ala Val Ser Gly Ile Asp Leu Ser Asn
   130 135 140
   Tyr Ala Ile Asn Trp Val Arg Gln Ala Pro Gly Lys Gly Leu Glu Trp
   145 150 155 160
   Ile Gly Ile Ile Trp Ala Ser Gly Thr Thr Phe Tyr Ala Thr Trp Ala
   165 170 175
   Lys Gly Arg Phe Thr Ile Ser Arg Asp Ser Thr Thr Val Tyr Leu Gln
   180 185 190
   Met Asn Ser Leu Arg Ala Glu Asp Thr Ala Val Tyr Tyr Cys Ala Arg
   195 200 205
   Thr Val Pro Gly Tyr Ser Thr Ala Pro Tyr Phe Asp Leu Trp Gly Gln
   210 215 220
   Gly Thr Leu Val Thr Val Ser Ser
   225 230
<210> 69
   <211> 233
   <212> PRT
   <213> Artificial
<220>
   <223> FabB-dAbH2 (CH1-G4Sx2)
<400> 69
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Gly Gly Gly Gly Ser Gly Gly Gly Gly
   100 105 110
   Ser Glu Val Gln Leu Val Glu Ser Gly Gly Gly Leu Val Gln Pro Gly
   115 120 125
   Gly Ser Leu Arg Leu Ser Cys Ala Val Ser Gly Phe Ser Leu Ser Arg
   130 135 140
   Tyr Ala Met Thr Trp Val Arg Gln Ala Pro Gly Lys Gly Leu Glu Trp
   145 150 155 160
   Ile Gly Thr Ile Thr Thr Gly Gly Asn Thr Asn Tyr Ala Asn Trp Ala
   165 170 175
   Lys Gly Arg Phe Thr Ile Ser Lys Asp Ser Thr Thr Val Tyr Leu Gln
   180 185 190
   Met Asn Ser Leu Arg Ala Glu Asp Thr Ala Val Tyr Tyr Cys Ala Arg
   195 200 205
   Gly Gly Tyr Val Ser Tyr Ala Asp Ala Thr Glu Leu Ser Leu Trp Gly
   210 215 220
   Gln Gly Thr Leu Val Thr Val Ser Ser
   225 230
<210> 70
   <211> 223
   <212> PRT
   <213> Artificial
<220>
   <223> FabB dAbL1 (cCH1-G45x2)
<400> 70
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Gly Gly Gly Gly Ser Gly Gly Gly Gly
               100                 105                 110
   Ser Asp Ile Val Met Thr Gln Ser Pro Ser Ser Val Ser Ala Ser Val
           115                 120                 125
   Gly Asp Arg Val Thr Ile Thr Cys Gln Ser Ser Pro Ser Val Trp Ser
       130                 135                 140
   Asn Phe Leu Ser Trp Tyr Gln Gln Lys Pro Gly Lys Ala Pro Lys Leu
   145                 150                 155                 160
   Leu Ile Tyr Glu Ala Ser Lys Leu Thr Ser Gly Val Pro Ser Arg Phe
                   165                 170                 175
   Lys Gly Ser Gly Ser Gly Thr Asp Phe Thr Leu Thr Ile Ser Ser Leu
               180                 185                 190
   Gln Pro Glu Asp Phe Ala Thr Tyr Tyr Cys Gly Gly Gly Tyr Ser Ser
           195                 200                 205
   Ile Ser Asp Thr Thr Phe Gly Gly Gly Thr Lys Val Glu Ile Lys
       210                 215                 220
<210> 71
   <211> 223
   <212> PRT
   <213> Artificial
<220>
   <223> FabB dAbL2 (CH1-G4Sx2)
<400> 71
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Gly Gly Gly Gly Ser Gly Gly Gly Gly
               100                 105                 110
   Ser Asp Ile Val Met Thr Gln Ser Pro Ser Thr Leu Ser Ala Ser Val
           115                 120                 125
   Gly Asp Arg Val Thr Ile Thr Cys Gln Ala Ser Gln Ser Ile Gly Ser
       130                 135                 140
   Arg Leu Ala Trp Tyr Gln Gln Lys Pro Gly Lys Ala Pro Lys Leu Leu
   145                 150                 155                 160
   Ile Tyr Tyr Ala Ser Thr Val Ala Ser Gly Val Pro Ser Arg Phe Lys
                   165                 170                 175
   Gly Ser Gly Ser Gly Thr Glu Phe Thr Leu Thr Ile Ser Ser Leu Gln
               180                 185                 190
   Pro Asp Asp Phe Ala Thr Tyr Tyr Cys Gln Ser Tyr Asp Tyr Ser Ser
           195                 200                 205
   Ser Ser Ser Tyr Ala Phe Gly Gly Gly Thr Lys Val Glu Ile Lys
       210                 215                 220
<210> 72
   <211> 227
   <212> PRT
   <213> Artificial
<220>
   <223> FabB dABL1 (CK1 G4sx2)
<400> 72
   Arg Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn Phe
               20                  25                  30
   Tyr Pro Arg Glu Ala Lys Val Gln Trp Lys Val Asp Asn Ala Leu Gln
           35                  40                  45
   Ser Gly Asn Ser Gln Glu Ser Val Thr Glu Gln Asp Ser Lys Asp Ser
       50                  55                  60
   Thr Tyr Ser Leu Ser Ser Thr Leu Thr Leu Ser Lys Ala Asp Tyr Glu
   65                  70                  75                  80
   Lys His Lys Val Tyr Ala Cys Glu Val Thr His Gln Gly Leu Ser Ser
                   85                  90                  95
   Pro Val Thr Lys Ser Phe Asn Arg Gly Glu Cys Gly Gly Gly Gly Ser
               100                 105                 110
   Gly Gly Gly Gly Ser Asp Ile Val Met Thr Gln Ser Pro Ser Ser Val
           115                 120                 125
   Ser Ala Ser Val Gly Asp Arg Val Thr Ile Thr Cys Gln Ser Ser Pro
       130                 135                 140
   Ser Val Trp Ser Asn Phe Leu Ser Trp Tyr Gln Gln Lys Pro Gly Lys
   145                 150                 155                 160
   Ala Pro Lys Leu Leu Ile Tyr Glu Ala Ser Lys Leu Thr Ser Gly Val
                   165                 170                 175
   Pro Ser Arg Phe Lys Gly Ser Gly Ser Gly Thr Asp Phe Thr Leu Thr
               180                 185                 190
   Ile Ser Ser Leu Gln Pro Glu Asp Phe Ala Thr Tyr Tyr Cys Gly Gly
           195                 200                 205
   Gly Tyr Ser Ser Ile Ser Asp Thr Thr Phe Gly Gly Gly Thr Lys Val
       210                 215                 220
   Glu Ile Lys
   225
<210> 73
   <211> 227
   <212> PRT
   <213> Artificial
<220>
   <223> FabB dAbL2 (CK1 G4Sx2)
<400> 73
   Arg Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn Phe
               20                  25                  30
   Tyr Pro Arg Glu Ala Lys Val Gln Trp Lys Val Asp Asn Ala Leu Gln
           35                  40                  45
   Ser Gly Asn Ser Gln Glu Ser Val Thr Glu Gln Asp Ser Lys Asp Ser
       50                  55                  60
   Thr Tyr Ser Leu Ser Ser Thr Leu Thr Leu Ser Lys Ala Asp Tyr Glu
   65                  70                  75                  80
   Lys His Lys Val Tyr Ala Cys Glu Val Thr His Gln Gly Leu Ser Ser
                   85                  90                  95
   Pro Val Thr Lys Ser Phe Asn Arg Gly Glu Cys Gly Gly Gly Gly Ser
               100                 105                 110
   Gly Gly Gly Gly Ser Asp Ile Val Met Thr Gln Ser Pro Ser Thr Leu
           115                 120                 125
   Ser Ala Ser Val Gly Asp Arg Val Thr Ile Thr Cys Gln Ala Ser Gln
       130                 135                 140
   Ser Ile Gly Ser Arg Leu Ala Trp Tyr Gln Gln Lys Pro Gly Lys Ala
   145                 150                 155                 160
   Pro Lys Leu Leu Ile Tyr Tyr Ala Ser Thr Val Ala Ser Gly Val Pro
                   165                 170                 175
   Ser Arg Phe Lys Gly Ser Gly Ser Gly Thr Glu Phe Thr Leu Thr Ile
               180                 185                 190
   Ser Ser Leu Gln Pro Asp Asp Phe Ala Thr Tyr Tyr Cys Gln Ser Tyr
           195                 200                 205
   Asp Tyr Ser Ser Ser Ser Ser Tyr Ala Phe Gly Gly Gly Thr Lys Val
       210                 215                 220
   Glu Ile Lys
   225
<210> 74
   <211> 255
   <212> PRT
   <213> Artificial
<220>
   <223> Fab'A heavy chain
<400> 74
   Met Lys Lys Thr Ala Ile Ala Ile Ala Val Ala Leu Ala Gly Phe Ala
   1               5                   10                  15
   Thr Val Ala Gln Ala Glu Val Gln Leu Val Glu Ser Gly Gly Gly Leu
               20                  25                  30
   Val Gln Pro Gly Gly Ser Leu Arg Leu Ser Cys Ala Phe Ser Gly Phe
           35                  40                  45
   Ser Leu Ser Thr Ser Gly Val Gly Val Gly Trp Val Arg Gln Ala Pro
       50                  55                  60
   Gly Lys Gly Leu Glu Trp Val Ala His Ile Trp Trp Asp Gly Asp Glu
   65                  70                  75                  80
   Ser Tyr Asn Pro Ser Leu Lys Thr Gln Phe Thr Ile Ser Lys Asp Thr
                   85                  90                  95
   Ser Lys Asn Thr Val Tyr Leu Gln Met Asn Ser Leu Arg Ala Glu Asp
               100                 105                 110
   Thr Ala Val Tyr Tyr Cys Ala Arg Asn Arg Tyr Asp Pro Pro Trp Phe
           115                 120                 125
   Val Asp Trp Gly Gln Gly Thr Leu Val Thr Val Ser Ser Ala Ser Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
           195                 200                 205
   Val Val Thr Val Pro Ser Ser Ser Leu Gly Thr Gln Thr Tyr Ile Cys
       210                 215                 220
   Asn Val Asn His Lys Pro Ser Asn Thr Lys Val Asp Lys Lys Val Glu
   225                 230                 235                 240
   Pro Lys Thr Cys Asp Lys Thr His Thr Cys Pro Pro Cys Pro Ala
                   245                 250                 255
<210> 75
   <211> 235
   <212> PRT
   <213> Artificial
<220>
   <223> FabA light chain
<400> 75
   Met Lys Lys Thr Ala Ile Ala Ile Ala Val Ala Leu Ala Gly Phe Ala
   1               5                   10                  15
   Thr Val Ala Gln Ala Asp Ile Gln Met Thr Gln Ser Pro Ser Ser Leu
               20                  25                  30
   Ser Ala Ser Val Gly Asp Arg Val Thr Ile Thr Cys Arg Ala Ser Gln
           35                  40                  45
   Asp Ile Ser Asn Tyr Leu Ser Trp Tyr Gln Gln Lys Pro Gly Lys Ala
       50                  55                  60
   Pro Lys Leu Leu Ile Tyr Tyr Thr Ser Lys Leu His Ser Gly Val Pro
   65                  70                  75                  80
   Ser Arg Phe Ser Gly Ser Gly Ser Gly Thr Asp Tyr Thr Leu Thr Ile
                   85                  90                  95
   Ser Ser Leu Gln Pro Glu Asp Phe Ala Thr Tyr Tyr Cys Gln Gln Gly
               100                 105                 110
   Lys Met Leu Pro Trp Thr Phe Gly Gln Gly Thr Lys Val Glu Ile Lys
           115                 120                 125
   Arg Thr Val Ala Ala Pro Ser Val Phe Ile Phe Pro Pro Ser Asp Glu
       130                 135                 140
   Gln Leu Lys Ser Gly Thr Ala Ser Val Val Cys Leu Leu Asn Asn Phe
   145                 150                 155                 160
   Tyr Pro Arg Glu Ala Lys Val Gln Trp Lys Val Asp Asn Ala Val Gln
                   165                 170                 175
   Ser Gly Asn Ser Gln Glu Ser Val Thr Glu Gln Asp Ser Lys Asp Ser
               180                 185                 190
   Thr Tyr Ser Leu Ser Ser Thr Leu Thr Leu Ser Lys Ala Asp Tyr Glu
           195                 200                 205
   Lys His Lys Val Tyr Ala Cys Glu Val Thr His Gln Gly Leu Ser Ser
       210                 215                 220
   Pro Val Thr Lys Ser Phe Asn Arg Gly Glu Cys
   225                 230                 235
<210> 76
   <211> 250
   <212> PRT
   <213> Artificial
<220>
   <223> Fab'A heavy chain (modified linker)
<400> 76
   Met Lys Lys Thr Ala Ile Ala Ile Ala Val Ala Leu Ala Gly Phe Ala
   1               5                   10                  15
   Thr Val Ala Gln Ala Glu Val Gln Leu Val Glu Ser Gly Gly Gly Leu
               20                  25                  30
   Val Gln Pro Gly Gly Ser Leu Arg Leu Ser Cys Ala Phe Ser Gly Phe
           35                  40                  45
   Ser Leu Ser Thr Ser Gly Val Gly Val Gly Trp Val Arg Gln Ala Pro
       50                  55                  60
   Gly Lys Gly Leu Glu Trp Val Ala His Ile Trp Trp Asp Gly Asp Glu
   65                  70                  75                  80
   Ser Tyr Asn Pro Ser Leu Lys Thr Gln Phe Thr Ile Ser Lys Asp Thr
                   85                  90                  95
   Ser Lys Asn Thr Val Tyr Leu Gln Met Asn Ser Leu Arg Ala Glu Asp
               100                 105                 110
   Thr Ala Val Tyr Tyr Cys Ala Arg Asn Arg Tyr Asp Pro Pro Trp Phe
           115                 120                 125
   Val Asp Trp Gly Gln Gly Thr Leu Val Thr Val Ser Ser Ala Ser Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
           195                 200                 205
   Val Val Thr Val Pro Ser Ser Ser Leu Gly Thr Gln Thr Tyr Ile Cys
       210                 215                 220
   Asn Val Asn His Lys Pro Ser Asn Thr Lys Val Asp Lys Lys Val Glu
   225                 230                 235                 240
   Pro Lys Thr Cys Asp Lys Thr His Thr Ser
                   245                 250
<210> 77
   <211> 244
   <212> PRT
   <213> Artificial
<220>
   <223> FabA heavy chain
<400> 77
   Met Lys Lys Thr Ala Ile Ala Ile Ala Val Ala Leu Ala Gly Phe Ala
   1               5                   10                  15
   Thr Val Ala Gln Ala Glu Val Gln Leu Val Glu Ser Gly Gly Gly Leu
               20                  25                  30
   Val Gln Pro Gly Gly Ser Leu Arg Leu Ser Cys Ala Phe Ser Gly Phe
           35                  40                  45
   Ser Leu Ser Thr Ser Gly Val Gly Val Gly Trp Val Arg Gln Ala Pro
       50                  55                  60
   Gly Lys Gly Leu Glu Trp Val Ala His Ile Trp Trp Asp Gly Asp Glu
   65                  70                  75                  80
   Ser Tyr Asn Pro Ser Leu Lys Thr Gln Phe Thr Ile Ser Lys Asp Thr
                   85                  90                  95
   Ser Lys Asn Thr Val Tyr Leu Gln Met Asn Ser Leu Arg Ala Glu Asp
               100                 105                 110
   Thr Ala Val Tyr Tyr Cys Ala Arg Asn Arg Tyr Asp Pro Pro Trp Phe
           115                 120                 125
   Val Asp Trp Gly Gln Gly Thr Leu Val Thr Val Ser Ser Ala Ser Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
   165 170 175
   Pro Val Thr Val Ser Trp Asn Ser Gly Ala Leu Thr Ser Gly Val His
   180 185 190
   Thr Phe Pro Ala Val Leu Gln Ser Ser Gly Leu Tyr Ser Leu Ser Ser
   195 200 205
   Val Val Thr Val Pro Ser Ser Ser Leu Gly Thr Gln Thr Tyr Ile Cys
   210 215 220
   Asn Val Asn His Lys Pro Ser Asn Thr Lys Val Asp Lys Lys Val Glu
   225 230 235 240
   Pro Lys Thr Cys
<210> 78
   <211> 119
   <212> PRT
   <213> Artificial
<220>
   <223> mdAbH1
<400> 78
   Gln Val Gln Leu Lys Glu Ser Gly Pro Gly Leu Val Ala Pro Ser Gln
   1 5 10 15
   Ser Leu Ser Ile Thr Cys Thr Val Ser Gly Ile Asp Leu Ser Asn Tyr
   20 25 30
   Ala Ile Asn Trp Val Arg Gln Pro Pro Gly Lys Gly Leu Glu Trp Ile
   35 40 45
   Gly Ile Ile Trp Ala Ser Gly Thr Thr Phe Tyr Ala Thr Trp Ala Lys
   50 55 60
   Gly Arg Phe Ser Ile Ser Arg Asp Ser Thr Thr Val Phe Leu Lys Met
   65 70 75 80
   Asn Ser Leu Gln Thr Asp Asp Thr Ala Arg Tyr Tyr Cys Ala Arg Thr
   85 90 95
   Val Pro Gly Tyr Ser Thr Ala Pro Tyr Phe Asp Leu Trp Gly Gln Gly
   100 105 110
   Thr Leu Val Thr Val Ser Ser
   115
<210> 79
   <211> 111
   <212> PRT
   <213> Artificial
<220>
   <223> mdAbL1
<400> 79
   Asp Ile Val Met Thr Gln Ser Pro Ser Ser Leu Thr Val Thr Ala Gly
   1               5                   10                  15
   Glu Lys Val Thr Met Ser Cys Gln Ser Ser Pro Ser Val Trp Ser Asn
               20                  25                  30
   Phe Leu Ser Trp Tyr Gln Gln Lys Pro Gly Gln Pro Pro Lys Leu Leu
           35                  40                  45
   Ile Tyr Glu Ala Ser Lys Leu Thr Ser Gly Val Pro Asp Arg Phe Lys
       50                  55                  60
   Gly Ser Gly Ser Gly Thr Asp Phe Thr Leu Thr Ile Ser Ser Val Gln
   65                  70                  75                  80
   Ala Glu Asp Leu Ala Thr Tyr Tyr Cys Gly Gly Gly Tyr Ser Ser Ile
                   85                  90                  95
   Ser Asp Thr Thr Phe Gly Gly Gly Thr Lys Leu Glu Ile Lys Arg
               100                 105                 110
<210> 80
   <211> 120
   <212> PRT
   <213> Artificial
<220>
   <223> mdAbH2
<400> 80
   Gln Val Gln Leu Lys Glu Ser Gly Pro Gly Leu Val Ala Pro Ser Gln
   1               5                   10                  15
   Ser Leu Ser Ile Thr Cys Thr Val Ser Gly Phe Ser Leu Ser Arg Tyr
               20                  25                  30
   Ala Met Thr Trp Val Arg Gln Pro Pro Gly Lys Gly Leu Glu Trp Ile
           35                  40                  45
   Gly Thr Ile Thr Thr Gly Gly Asn Thr Asn Tyr Ala Asn Trp Ala Lys
       50                  55                  60
   Gly Arg Phe Ser Ile Ser Lys Asp Ser Thr Gln Val Phe Leu Lys Met
   65                  70                  75                  80
   Asn Ser Leu Gln Thr Asp Asp Thr Ala Met Tyr Tyr Cys Ala Arg Gly
                   85                  90                  95
   Gly Tyr Val Ser Tyr Ala Asp Ala Thr Glu Leu Ser Leu Trp Gly Gln
               100                 105                 110
   Gly Thr Leu Val Thr Val Ser Ser
           115                 120
<210> 81
   <211> 111
   <212> PRT
   <213> Artificial
<220>
   <223> mdAbL2
<400> 81
   Asp Ile Val Met Thr Gln Ser Pro Ser Ser Leu Ser Ala Ser Leu Gly
   1               5                   10                  15
   Glu Arg Val Thr Ile Thr Cys Gln Ala Ser Gln Ser Ile Gly Ser Arg
               20                  25                  30
   Leu Ala Trp Tyr Gln Gln Lys Pro Gly Lys Thr Pro Lys Leu Leu Ile
           35                  40                  45
   Phe Tyr Ala Ser Thr Val Ala Ser Gly Val Pro Ser Arg Phe Lys Gly
       50                  55                  60
   Ser Gly Ser Gly Thr Glu Phe Ser Leu Thr Ile Ser Ser Leu Gln Pro
   65                  70                  75                  80
   Glu Asp Phe Ala Thr Tyr Tyr Cys Gln Ser Tyr Asp Tyr Ser Ser Ser
                   85                  90                  95
   Ser Ser Tyr Ala Phe Gly Gly Gly Thr Lys Leu Glu Ile Lys Arg
               100                 105                 110
<210> 82
   <211> 239
   <212> PRT
   <213> Artificial
<220>
   <223> mFabD-mdidAb dAbH1(CH1-G4sx2)
<400> 82
   Ala Lys Thr Thr Pro Pro Ser Val Tyr Pro Leu Ala Pro Gly Ser Ala
   1               5                   10                  15
   Ala Gln Thr Asn Ser Met Val Thr Leu Gly Cys Leu Val Lys Gly Tyr
               20                  25                  30
   Phe Pro Glu Pro Val Thr Val Thr Trp Asn Ser Gly Ser Leu Ser Ser
           35                  40                  45
   Gly Val His Thr Phe Pro Ala Val Leu Gln Ser Asp Leu Tyr Thr Leu
       50                  55                  60
   Ser Ser Ser Val Thr Val Pro Ser Ser Thr Trp Pro Ser Glu Thr Val
   65                  70                  75                  80
   Thr Cys Asn Val Ala His Pro Ala Ser Ser Thr Lys Val Asp Lys Lys
                   85                  90                  95
   Ile Val Pro Arg Asp Cys Gly Gly Gly Gly Ser Gly Gly Gly Gly Ser
               100                 105                 110
   Gln Val Gln Leu Lys Glu Ser Gly Pro Gly Leu Val Ala Pro Ser Gln
           115                 120                 125
   Ser Leu Ser Ile Thr Cys Thr Val Ser Gly Ile Asp Leu Ser Asn Tyr
       130                 135                 140
   Ala Ile Asn Trp Val Arg Gln Pro Pro Gly Lys Gly Leu Glu Trp Ile
   145                 150                 155                 160
   Gly Ile Ile Trp Ala Ser Gly Thr Thr Phe Tyr Ala Thr Trp Ala Lys
                   165                 170                 175
   Gly Arg Phe Ser Ile Ser Arg Asp Ser Thr Thr Val Phe Leu Lys Met
               180                 185                 190
   Asn Ser Leu Gln Thr Asp Asp Thr Ala Arg Tyr Tyr Cys Ala Arg Thr
           195                 200                 205
   Val Pro Gly Tyr Ser Thr Ala Pro Tyr Phe Asp Leu Trp Gly Gln Gly
       210                 215                 220
   Thr Leu Val Thr Val Ser Ser Asp Tyr Lys Asp Asp Asp Asp Lys
   225                 230                 235
<210> 83
   <211> 237
   <212> PRT
   <213> Artificial
<220>
   <223> mFabD-mdidAb, -dAbL1(CK-G4sx2)
<400> 83
   Thr Asp Ala Ala Pro Thr Val Ser Ile Phe Pro Pro Ser Ser Glu Gln
   1               5                   10                  15
   Leu Thr Ser Gly Gly Ala Ser Val Val Cys Phe Leu Asn Asn Phe Tyr
               20                  25                  30
   Pro Lys Asp Ile Asn Val Lys Trp Lys Ile Asp Gly Ser Glu Arg Gln
           35                  40                  45
   Asn Gly Val Leu Asn Ser Trp Thr Asp Gln Asp Ser Lys Asp Ser Thr
       50                  55                  60
   Tyr Ser Met Ser Ser Thr Leu Thr Leu Thr Lys Asp Glu Tyr Glu Arg
   65                  70                  75                  80
   His Asn Ser Tyr Thr Cys Glu Ala Thr His Lys Thr Ser Thr Ser Pro
                   85                  90                  95
   Ile Val Lys Ser Phe Asn Arg Asn Glu Cys Gly Gly Gly Gly Ser Gly
               100                 105                 110
   Gly Gly Gly Ser Asp Ile Val Met Thr Gln Ser Pro Ser Ser Leu Thr
           115                 120                 125
   Val Thr Ala Gly Glu Lys Val Thr Met Ser Cys Gln Ser Ser Pro Ser
       130                 135                 140
   Val Trp Ser Asn Phe Leu Ser Trp Tyr Gln Gln Lys Pro Gly Gln Pro
   145                 150                 155                 160
   Pro Lys Leu Leu Ile Tyr Glu Ala Ser Lys Leu Thr Ser Gly Val Pro
                   165                 170                 175
   Asp Arg Phe Lys Gly Ser Gly Ser Gly Thr Asp Phe Thr Leu Thr Ile
               180                 185                 190
   Ser Ser Val Gln Ala Glu Asp Leu Ala Thr Tyr Tyr Cys Gly Gly Gly
           195                 200                 205
   Tyr Ser Ser Ile Ser Asp Thr Thr Phe Gly Gly Gly Thr Lys Leu Glu
       210                 215                 220
   Ile Lys Arg Glu Gln Lys Leu Ile Ser Glu Glu Asp Leu
   225                 230                 235
<210> 84
   <211> 240
   <212> PRT
   <213> Artificial
<220>
   <223> mFabD-mdidAb, -dAbH2(CH1-G4sx2)
<400> 84
   Ala Lys Thr Thr Pro Pro Ser Val Tyr Pro Leu Ala Pro Gly Ser Ala
   1               5                   10                  15
   Ala Gln Thr Asn Ser Met Val Thr Leu Gly Cys Leu Val Lys Gly Tyr
               20                  25                  30
   Phe Pro Glu Pro Val Thr Val Thr Trp Asn Ser Gly Ser Leu Ser Ser
           35                  40                  45
   Gly Val His Thr Phe Pro Ala Val Leu Gln Ser Asp Leu Tyr Thr Leu
       50                  55                  60
   Ser Ser Ser Val Thr Val Pro Ser Ser Thr Trp Pro Ser Glu Thr Val
   65                  70                  75                  80
   Thr Cys Asn Val Ala His Pro Ala Ser Ser Thr Lys Val Asp Lys Lys
                   85                  90                  95
   Ile Val Pro Arg Asp Cys Gly Gly Gly Gly Ser Gly Gly Gly Gly Ser
               100                 105                 110
   Gln Val Gln Leu Lys Glu Ser Gly Pro Gly Leu Val Ala Pro Ser Gln
           115                 120                 125
   Ser Leu Ser Ile Thr Cys Thr Val Ser Gly Phe Ser Leu Ser Arg Tyr
       130                 135                 140
   Ala Met Thr Trp Val Arg Gln Pro Pro Gly Lys Gly Leu Glu Trp Ile
   145                 150                 155                 160
   Gly Thr Ile Thr Thr Gly Gly Asn Thr Asn Tyr Ala Asn Trp Ala Lys
                   165                 170                 175
   Gly Arg Phe Ser Ile Ser Lys Asp Ser Thr Gln Val Phe Leu Lys Met
               180                 185                 190
   Asn Ser Leu Gln Thr Asp Asp Thr Ala Met Tyr Tyr Cys Ala Arg Gly
           195                 200                 205
   Gly Tyr Val Ser Tyr Ala Asp Ala Thr Glu Leu Ser Leu Trp Gly Gln
       210                 215                 220
   Gly Thr Leu Val Thr Val Ser Ser Asp Tyr Lys Asp Asp Asp Asp Lys
   225                 230                 235                 240
<210> 85
   <211> 237
   <212> PRT
   <213> Artificial
<220>
   <223> mFabD-mdidAb, -dAbL2(CK-G4Sx2)
<400> 85
   Thr Asp Ala Ala Pro Thr Val Ser Ile Phe Pro Pro Ser Ser Glu Gln
   1               5                   10                  15
   Leu Thr Ser Gly Gly Ala Ser Val Val Cys Phe Leu Asn Asn Phe Tyr
               20                  25                  30
   Pro Lys Asp Ile Asn Val Lys Trp Lys Ile Asp Gly Ser Glu Arg Gln
           35                  40                  45
   Asn Gly Val Leu Asn Ser Trp Thr Asp Gln Asp Ser Lys Asp Ser Thr
       50                  55                  60
   Tyr Ser Met Ser Ser Thr Leu Thr Leu Thr Lys Asp Glu Tyr Glu Arg
   65                  70                  75                  80
   His Asn Ser Tyr Thr Cys Glu Ala Thr His Lys Thr Ser Thr Ser Pro
                   85                  90                  95
   Ile Val Lys Ser Phe Asn Arg Asn Glu Cys Gly Gly Gly Gly Ser Gly
               100                 105                 110
   Gly Gly Gly Ser Asp Ile Val Met Thr Gln Ser Pro Ser Ser Leu Ser
           115                 120                 125
   Ala Ser Leu Gly Glu Arg Val Thr Ile Thr Cys Gln Ala Ser Gln Ser
       130                 135                 140
   Ile Gly Ser Arg Leu Ala Trp Tyr Gln Gln Lys Pro Gly Lys Thr Pro
   145                 150                 155                 160
   Lys Leu Leu Ile Phe Tyr Ala Ser Thr Val Ala Ser Gly Val Pro Ser
                   165                 170                 175
   Arg Phe Lys Gly Ser Gly Ser Gly Thr Glu Phe Ser Leu Thr Ile Ser
               180                 185                 190
   Ser Leu Gln Pro Glu Asp Phe Ala Thr Tyr Tyr Cys Gln Ser Tyr Asp
           195                 200                 205
   Tyr Ser Ser Ser Ser Ser Tyr Ala Phe Gly Gly Gly Thr Lys Leu Glu
       210                 215                 220
   Ile Lys Arg Glu Gln Lys Leu Ile Ser Glu Glu Asp Leu
   225                 230                 235
<210> 86
   <211> 239
   <212> PRT
   <213> Artificial
<220>
   <223> dAbH1(CH1-G4Sx2)mFabC-mdAbH1
<400> 86
   Ala Lys Thr Thr Pro Pro Ser Val Tyr Pro Leu Ala Pro Gly Ser Ala
   1 5 10 15
   Ala Gln Thr Asn Ser Met Val Thr Leu Gly Cys Leu Val Lys Gly Tyr
   20 25 30
   Phe Pro Glu Pro Val Thr Val Thr Trp Asn Ser Gly Ser Leu Ser Ser
   35 40 45
   Gly Val His Thr Phe Pro Ala Val Leu Gln Ser Asp Leu Tyr Thr Leu
   50 55 60
   Ser Ser Ser Val Thr Val Pro Ser Ser Thr Trp Pro Ser Glu Thr Val
   65 70 75 80
   Thr Cys Asn Val Ala His Pro Ala Ser Ser Thr Lys Val Asp Lys Lys
   85 90 95
   Ile Val Pro Arg Asp Cys Gly Gly Gly Gly Ser Gly Gly Gly Gly Ser
   100 105 110
   Gln Val Gln Leu Lys Glu Ser Gly Pro Gly Leu Val Ala Pro Ser Gln
   115 120 125
   Ser Leu Ser Ile Thr Cys Thr Val Ser Gly Ile Asp Leu Ser Asn Tyr
   130 135 140
   Ala Ile Asn Trp Val Arg Gln Pro Pro Gly Lys Gly Leu Glu Trp Ile
   145 150 155 160
   Gly Ile Ile Trp Ala Ser Gly Thr Thr Phe Tyr Ala Thr Trp Ala Lys
   165 170 175
   Gly Arg Phe Ser Ile Ser Arg Asp Ser Thr Thr Val Phe Leu Lys Met
   180 185 190
   Asn Ser Leu Gln Thr Asp Asp Thr Ala Arg Tyr Tyr Cys Ala Arg Thr
   195 200 205
   Val Pro Gly Tyr Ser Thr Ala Pro Tyr Phe Asp Leu Trp Gly Gln Gly
   210 215 220
   Thr Leu Val Thr Val Ser Ser Asp Tyr Lys Asp Asp Asp Asp Lys
   225 230 235
<210> 87
   <211> 237
   <212> PRT
   <213> Artificial
<220>
   <223> mFabC-mdidAb, -dAbL1(CK-G4Sx2)
<400> 87
   Thr Asp Ala Ala Pro Thr Val Ser Ile Phe Pro Pro Ser Ser Glu Gln
   1               5                   10                  15
   Leu Thr Ser Gly Gly Ala Ser Val Val Cys Phe Leu Asn Asn Phe Tyr
               20                  25                  30
   Pro Lys Asp Ile Asn Val Lys Trp Lys Ile Asp Gly Ser Glu Arg Gln
           35                  40                  45
   Asn Gly Val Leu Asn Ser Trp Thr Asp Gln Asp Ser Lys Asp Ser Thr
       50                  55                  60
   Tyr Ser Met Ser Ser Thr Leu Thr Leu Thr Lys Asp Glu Tyr Glu Arg
   65                  70                  75                  80
   His Asn Ser Tyr Thr Cys Glu Ala Thr His Lys Thr Ser Thr Ser Pro
                   85                  90                  95
   Ile Val Lys Ser Phe Asn Arg Asn Glu Cys Gly Gly Gly Gly Ser Gly
               100                 105                 110
   Gly Gly Gly Ser Asp Ile Val Met Thr Gln Ser Pro Ser Ser Leu Thr
           115                 120                 125
   Val Thr Ala Gly Glu Lys Val Thr Met Ser Cys Gln Ser Ser Pro Ser
       130                 135                 140
   Val Trp Ser Asn Phe Leu Ser Trp Tyr Gln Gln Lys Pro Gly Gln Pro
   145                 150                 155                 160
   Pro Lys Leu Leu Ile Tyr Glu Ala Ser Lys Leu Thr Ser Gly Val Pro
                   165                 170                 175
   Asp Arg Phe Lys Gly Ser Gly Ser Gly Thr Asp Phe Thr Leu Thr Ile
               180                 185                 190
   Ser Ser Val Gln Ala Glu Asp Leu Ala Thr Tyr Tyr Cys Gly Gly Gly
           195                 200                 205
   Tyr Ser Ser Ile Ser Asp Thr Thr Phe Gly Gly Gly Thr Lys Leu Glu
       210                 215                 220
   Ile Lys Arg Glu Gln Lys Leu Ile Ser Glu Glu Asp Leu
   225                 230                 235
<210> 88
   <211> 240
   <212> PRT
   <213> Artificial
<220>
   <223> mFabC-mdidAb dAbH2(CH1-G4Sx2)
<400> 88
   Ala Lys Thr Thr Pro Pro Ser Val Tyr Pro Leu Ala Pro Gly Ser Ala
   1 5 10 15
   Ala Gln Thr Asn Ser Met Val Thr Leu Gly Cys Leu Val Lys Gly Tyr
   20 25 30
   Phe Pro Glu Pro Val Thr Val Thr Trp Asn Ser Gly Ser Leu Ser Ser
   35 40 45
   Gly Val His Thr Phe Pro Ala Val Leu Gln Ser Asp Leu Tyr Thr Leu
   50 55 60
   Ser Ser Ser Val Thr Val Pro Ser Ser Thr Trp Pro Ser Glu Thr Val
   65 70 75 80
   Thr Cys Asn Val Ala His Pro Ala Ser Ser Thr Lys Val Asp Lys Lys
   85 90 95
   Ile Val Pro Arg Asp Cys Gly Gly Gly Gly Ser Gly Gly Gly Gly Ser
   100 105 110
   Gln Val Gln Leu Lys Glu Ser Gly Pro Gly Leu Val Ala Pro Ser Gln
   115 120 125
   Ser Leu Ser Ile Thr Cys Thr Val Ser Gly Phe Ser Leu Ser Arg Tyr
   130 135 140
   Ala Met Thr Trp Val Arg Gln Pro Pro Gly Lys Gly Leu Glu Trp Ile
   145 150 155 160
   Gly Thr Ile Thr Thr Gly Gly Asn Thr Asn Tyr Ala Asn Trp Ala Lys
   165 170 175
   Gly Arg Phe Ser Ile Ser Lys Asp Ser Thr Gln Val Phe Leu Lys Met
   180 185 190
   Asn Ser Leu Gln Thr Asp Asp Thr Ala Met Tyr Tyr Cys Ala Arg Gly
   195 200 205
   Gly Tyr Val Ser Tyr Ala Asp Ala Thr Glu Leu Ser Leu Trp Gly Gln
   210 215 220
   Gly Thr Leu Val Thr Val Ser Ser Asp Tyr Lys Asp Asp Asp Asp Lys
   225 230 235 240
<210> 89
   <211> 237
   <212> PRT
   <213> Artificial
<220>
   <223> mFabc-mdidAb, -dAbL2(CK-G4Sx2)
<400> 89
   Thr Asp Ala Ala Pro Thr Val Ser Ile Phe Pro Pro Ser Ser Glu Gln
   1               5                   10                  15
   Leu Thr Ser Gly Gly Ala Ser Val Val Cys Phe Leu Asn Asn Phe Tyr
               20                  25                  30
   Pro Lys Asp Ile Asn Val Lys Trp Lys Ile Asp Gly Ser Glu Arg Gln
           35                  40                  45
   Asn Gly Val Leu Asn Ser Trp Thr Asp Gln Asp Ser Lys Asp Ser Thr
       50                  55                  60
   Tyr Ser Met Ser Ser Thr Leu Thr Leu Thr Lys Asp Glu Tyr Glu Arg
   65                  70                  75                  80
   His Asn Ser Tyr Thr Cys Glu Ala Thr His Lys Thr Ser Thr Ser Pro
                   85                  90                  95
   Ile Val Lys Ser Phe Asn Arg Asn Glu Cys Gly Gly Gly Gly Ser Gly
               100                 105                 110
   Gly Gly Gly Ser Asp Ile Val Met Thr Gln Ser Pro Ser Ser Leu Ser
           115                 120                 125
   Ala Ser Leu Gly Glu Arg Val Thr Ile Thr Cys Gln Ala Ser Gln Ser
       130                 135                 140
   Ile Gly Ser Arg Leu Ala Trp Tyr Gln Gln Lys Pro Gly Lys Thr Pro
   145                 150                 155                 160
   Lys Leu Leu Ile Phe Tyr Ala Ser Thr Val Ala Ser Gly Val Pro Ser
                   165                 170                 175
   Arg Phe Lys Gly Ser Gly Ser Gly Thr Glu Phe Ser Leu Thr Ile Ser
               180                 185                 190
   Ser Leu Gln Pro Glu Asp Phe Ala Thr Tyr Tyr Cys Gln Ser Tyr Asp
           195                 200                 205
   Tyr Ser Ser Ser Ser Ser Tyr Ala Phe Gly Gly Gly Thr Lys Leu Glu
       210                 215                 220
   Ile Lys Arg Glu Gln Lys Leu Ile Ser Glu Glu Asp Leu
   225                 230                 235
<210> 90
   <211> 13
   <212> PRT
   <213> Artificial
<220>
   <223> 646Fv CDRH3
<400> 90
   Thr Val Pro Gly Tyr Ser Thr Ala Pro Tyr Phe Asp Leu
   1 5 10
<210> 91
   <211> 12
   <212> PRT
   <213> Artificial
<220>
   <223> 646Fv CDRL1
<400> 91
   Gln Ser Ser Pro Ser Val Trp Ser Asp Phe Leu Ser
   1 5 10
<210> 92
   <211> 7
   <212> PRT
   <213> Artificial
<220>
   <223> 646Fv CDRL2
<400> 92
   Gly Ala Ser Thr Leu Ala Ser
   1 5
<210> 93
   <211> 11
   <212> PRT
   <213> Artificial
<220>
   <223> 646Fv CDRL3
<400> 93
   Gly Gly Gly Tyr Ser Ser Ile Ser Asp Thr Thr
   1 5 10
<210> 94
   <211> 11
   <212> PRT
   <213> Artificial
<220>
   <223> 647FV CDRH1
<400> 94
   Gly Phe Thr Leu Ser Asn Asn Tyr Trp Met Cys
   1 5 10
<210> 95
   <211> 17
   <212> PRT
   <213> Artificial
<220>
   <223> 647FV CDRH2
<400> 95
   Cys Ile Tyr Thr Gly Asp Gly Asp Thr Ala Tyr Thr Ser Trp Ala Lys
   1 5 10 15
   Gly
<210> 96
   <211> 12
   <212> PRT
   <213> Artificial
<220>
   <223> 647Fv CDRH3
<400> 96
   Ser Gly Gly Ser Tyr Tyr Asp Tyr Val Phe Ile Leu
   1 5 10
<210> 97
   <211> 11
   <212> PRT
   <213> Artificial
<220>
   <223> 647Fv QASQSLGNRLA
<400> 97
   Gln Ala Ser Gln Ser Leu Gly Asn Arg Leu Ala
   1 5 10
<210> 98
   <211> 7
   <212> PRT
   <213> Artificial
<220>
   <223> 647Fv CDRL2
<400> 98
   Arg Ala Ser Thr Leu Ala Ser
   1 5
<210> 99
   <211> 11
   <212> PRT
   <213> Artificial
<220>
   <223> 647Fv CDRL3
<400> 99
   Gln Cys Thr Tyr Ile Gly Ser Lys Met Gly Ala
   1 5 10
<210> 100
   <211> 11
   <212> PRT
   <213> Artificial
<220>
   <223> 649Fv CDRH1
<400> 100
   Gly Phe Ser Phe Ser Gly Asn Tyr Trp Ile Cys
   1 5 10
<210> 101
   <211> 17
   <212> PRT
   <213> Artificial
<220>
   <223> 649Fv CDRH2
<400> 101
   Cys Ile Phe Thr Ala Asp Gly Asp Thr Ala Tyr Thr Ser Trp Ala Lys
   1 5 10 15
   Gly
<210> 102
   <211> 12
   <212> PRT
   <213> Artificial
<220>
   <223> 649Fv CDRH3
<400> 102
   Ser Gly Gly Ser Ala Phe Asp Tyr Val Phe Ile Leu
   1 5 10
<210> 103
   <211> 11
   <212> PRT
   <213> Artificial
<220>
   <223> 649Fv CDRL1
<400> 103
   Gln Ala Ser Gln Ser Ile Gly Asn Arg Leu Gly
   1 5 10
<210> 104
   <211> 7
   <212> PRT
   <213> Artificial
<220>
   <223> 649Fv CDRL2
<400> 104
   Arg Ala Ser Thr Leu Glu Ser
   1 5
<210> 105
   <211> 11
   <212> PRT
   <213> Artificial
<220>
   <223> 649Fv CDRL3
<400> 105
   Gln Cys Thr Tyr Ile Gly Lys Leu Met Gly Ala
   1 5 10
<210> 106
   <211> 10
   <212> PRT
   <213> Artificial
<220>
   <223> 645HeavyI50AFv CDRH1
<400> 106
   Gly Ile Asp Leu Ser Asn Tyr Ala Ile Asn
   1               5                   10
<210> 107
   <211> 16
   <212> PRT
   <213> Artificial
<220>
   <223> 645HeavyI50AFv CDRH2
<400> 107
   Ala Ile Trp Ala Ser Gly Thr Thr Phe Tyr Ala Thr Trp Ala Lys Gly
   1               5                   10                  15
<210> 108
   <211> 13
   <212> PRT
   <213> Artificial
<220>
   <223> 645HeavyI50AFv CDRH3
<400> 108
   Thr Val Pro Gly Tyr Ser Thr Ala Pro Tyr Phe Asp Leu
   1               5                   10
<210> 109
   <211> 12
   <212> PRT
   <213> Artificial
<220>
   <223> 645HeavyI50AFv CDRL1
<400> 109
   Gln Ser Ser Pro Ser Val Trp Ser Asn Phe Leu Ser
   1               5                   10
<210> 110
   <211> 7
   <212> PRT
   <213> Artificial
<220>
   <223> 645HeavyI50AFv CDRL2
<400> 110
   Glu Ala Ser Lys Leu Thr Ser
   1               5
<210> 111
   <211> 11
   <212> PRT
   <213> Artificial
<220>
   <223> 645HeavyI50AFV CDRL3
<400> 111
   Gly Gly Gly Tyr Ser Ser Ile Ser Asp Thr Thr
   1               5                   10
<210> 112
   <211> 10
   <212> PRT
   <213> Artificial
<220>
   <223> 645HeavyT56AFv CDRH1
<400> 112
   Gly Ile Asp Leu Ser Asn Tyr Ala Ile Asn
   1               5                   10
<210> 113
   <211> 16
   <212> PRT
   <213> Artificial
<220>
   <223> 645HeavyT56AFv CDRH2
<400> 113
   Ile Ile Trp Ala Ser Gly Ala Thr Phe Tyr Ala Thr Trp Ala Lys Gly
   1               5                   10                  15
<210> 114
   <211> 13
   <212> PRT
   <213> Artificial
<220>
   <223> 645HeavyT56AFv CDRL2
<400> 114
   Thr Val Pro Gly Tyr Ser Thr Ala Pro Tyr Phe Asp Leu
   1 5 10
<210> 115
   <211> 12
   <212> PRT
   <213> Artificial
<220>
   <223> 645HeavyT56AFv CDRL1
<400> 115
   Gln Ser Ser Pro Ser Val Trp Ser Asn Phe Leu Ser
   1 5 10
<210> 116
   <211> 7
   <212> PRT
   <213> Artificial
<220>
   <223> 645HeavyT56AFv CDRL2
<400> 116
   Glu Ala Ser Lys Leu Thr Ser
   1 5
<210> 117
   <211> 11
   <212> PRT
   <213> Artificial
<220>
   <223> 645HeavyT56AFv CDRL3
<400> 117
   Gly Gly Gly Tyr Ser Ser Ile Ser Asp Thr Thr
   1 5 10
<210> 118
   <211> 10
   <212> PRT
   <213> Artificial
<220>
   <223> 645HeavyT95AFv CDRH1
<400> 118
   Gly Ile Asp Leu Ser Asn Tyr Ala Ile Asn
   1 5 10
<210> 119
   <211> 16
   <212> PRT
   <213> Artificial
<220>
   <223> 645HeavyT95AFv CDRH2
<400> 119
   Ile Ile Trp Ala Ser Gly Thr Thr Phe Tyr Ala Thr Trp Ala Lys Gly
   1 5 10 15
<210> 120
   <211> 13
   <212> PRT
   <213> Artificial
<220>
   <223> 645HeavyT95AFv CDRH3
<400> 120
   Ala Val Pro Gly Tyr Ser Thr Ala Pro Tyr Phe Asp Leu
   1 5 10
<210> 121
   <211> 12
   <212> PRT
   <213> Artificial
<220>
   <223> 645HeavyT95AFv CDRL1
<400> 121
   Gln Ser Ser Pro Ser Val Trp Ser Asn Phe Leu Ser
   1 5 10
<210> 122
   <211> 7
   <212> PRT
   <213> Artificial
<220>
   <223> 645HeavyT95AFv CDRL2
<400> 122
   Glu Ala Ser Lys Leu Thr Ser
   1 5
<210> 123
   <211> 11
   <212> PRT
   <213> Artificial
<220>
   <223> 645HeavyT95AFv CDRL3
<400> 123
   Gly Gly Gly Tyr Ser Ser Ile Ser Asp Thr Thr
   1 5 10
<210> 124
   <211> 10
   <212> PRT
   <213> Artificial
<220>
   <223> 645Heavyv96AFv CDRH1
<400> 124
   Gly Ile Asp Leu Ser Asn Tyr Ala Ile Asn
   1 5 10
<210> 125
   <211> 16
   <212> PRT
   <213> Artificial
<220>
   <223> 645Heavyv96AFv CDRH2
<400> 125
   Ile Ile Trp Ala Ser Gly Thr Thr Phe Tyr Ala Thr Trp Ala Lys Gly
   1 5 10 15
<210> 126
   <211> 13
   <212> PRT
   <213> Artificial
<220>
   <223> 645Heavyv96AFv CDRH3
<400> 126
   Thr Ala Pro Gly Tyr Ser Thr Ala Pro Tyr Phe Asp Leu
   1 5 10
<210> 127
   <211> 12
   <212> PRT
   <213> Artificial
<220>
   <223> 645Heavyv96AFv CDRL1
<400> 127
   Gln Ser Ser Pro Ser Val Trp Ser Asn Phe Leu Ser
   1 5 10
<210> 128
   <211> 7
   <212> PRT
   <213> Artificial
<220>
   <223> 645HeavyV96AFv CDRL2
<400> 128
   Glu Ala Ser Lys Leu Thr Ser
   1 5
<210> 129
   <211> 11
   <212> PRT
   <213> Artificial
<220>
   <223> 645Heavyv96AFv CDRL3
<400> 129
   Gly Gly Gly Tyr Ser Ser Ile Ser Asp Thr Thr
   1 5 10
<210> 130
   <211> 10
   <212> PRT
   <213> Artificial
<220>
   <223> 645HeavyP97AFv CDRH1
<400> 130
   Gly Ile Asp Leu Ser Asn Tyr Ala Ile Asn
   1 5 10
<210> 131
   <211> 16
   <212> PRT
   <213> Artificial
<220>
   <223> 645HeavyP97AFv CDRH2
<400> 131
   Ile Ile Trp Ala Ser Gly Thr Thr Phe Tyr Ala Thr Trp Ala Lys Gly
   1 5 10 15
<210> 132
   <211> 13
   <212> PRT
   <213> Artificial
<220>
   <223> 645HeavyP97AFv CDRH3
<400> 132
   Thr Val Ala Gly Tyr Ser Thr Ala Pro Tyr Phe Asp Leu
   1 5 10
<210> 133
   <211> 12
   <212> PRT
   <213> Artificial
<220>
   <223> 645HeavyP97AFv CDRL1
<400> 133
   Gln Ser Ser Pro Ser Val Trp Ser Asn Phe Leu Ser
   1 5 10
<210> 134
   <211> 7
   <212> PRT
   <213> Artificial
<220>
   <223> 645HeavyP97AFv CDRL2
<400> 134
   Glu Ala Ser Lys Leu Thr Ser
   1 5
<210> 135
   <211> 11
   <212> PRT
   <213> Artificial
<220>
   <223> 645HeavyP97AFv CDRL3
<400> 135
   Gly Gly Gly Tyr Ser Ser Ile Ser Asp Thr Thr
   1 5 10
<210> 136
   <211> 10
   <212> PRT
   <213> Artificial
<220>
   <223> 645HeavyG98AFv CDRH1
<400> 136
   Gly Ile Asp Leu Ser Asn Tyr Ala Ile Asn
   1 5 10
<210> 137
   <211> 16
   <212> PRT
   <213> Artificial
<220>
   <223> 645HeavyG98AFv CDRH2
<400> 137
   Ile Ile Trp Ala Ser Gly Thr Thr Phe Tyr Ala Thr Trp Ala Lys Gly
   1 5 10 15
<210> 138
   <211> 13
   <212> PRT
   <213> Artificial
<220>
   <223> 645HeavyG98AFv CDRH3
<400> 138
   Thr Val Pro Ala Tyr Ser Thr Ala Pro Tyr Phe Asp Leu
   1 5 10
<210> 139
   <211> 12
   <212> PRT
   <213> Artificial
<220>
   <223> 645HeavyG98AFv CDRL1
<400> 139
   Gln Ser Ser Pro Ser Val Trp Ser Asn Phe Leu Ser
   1 5 10
<210> 140
   <211> 7
   <212> PRT
   <213> Artificial
<220>
   <223> 645HeavyG98AFv CDRL2
<400> 140
   Glu Ala Ser Lys Leu Thr Ser
   1 5
<210> 141
   <211> 11
   <212> PRT
   <213> Artificial
<220>
   <223> 645HeavyG98AFv CDRL3
<400> 141
   Gly Gly Gly Tyr Ser Ser Ile Ser Asp Thr Thr
   1 5 10
<210> 142
   <211> 10
   <212> PRT
   <213> Artificial
<220>
   <223> 645HeavyY99AFv CDRH1
<400> 142
   Gly Ile Asp Leu Ser Asn Tyr Ala Ile Asn
   1 5 10
<210> 143
   <211> 16
   <212> PRT
   <213> Artificial
<220>
   <223> 645HeavyY99AFv CDRH2
<400> 143
   Ile Ile Trp Ala Ser Gly Thr Thr Phe Tyr Ala Thr Trp Ala Lys Gly
   1 5 10 15
<210> 144
   <211> 13
   <212> PRT
   <213> Artificial
<220>
   <223> 645HeavyY99AFv CDRH3
<400> 144
   Thr Val Pro Gly Ala Ser Thr Ala Pro Tyr Phe Asp Leu
   1 5 10
<210> 145
   <211> 12
   <212> PRT
   <213> Artificial
<220>
   <223> 645HeavyY99AFv CDRL1
<400> 145
   Gln Ser Ser Pro Ser Val Trp Ser Asn Phe Leu Ser
   1 5 10
<210> 146
   <211> 7
   <212> PRT
   <213> Artificial
<220>
   <223> 645HeavyY99AFv CDRL2
<400> 146
   Glu Ala Ser Lys Leu Thr Ser
   1 5
<210> 147
   <211> 11
   <212> PRT
   <213> Artificial
<220>
   <223> 645HeavyY99AFv CDRL3
<400> 147
   Gly Gly Gly Tyr Ser Ser Ile Ser Asp Thr Thr
   1 5 10
<210> 148
   <211> 10
   <212> PRT
   <213> Artificial
<220>
   <223> 645HeavyS100AFv CDRH1
<400> 148
   Gly Ile Asp Leu Ser Asn Tyr Ala Ile Asn
   1 5 10
<210> 149
   <211> 16
   <212> PRT
   <213> Artificial
<220>
   <223> 645HeavyS100AFv CDRH2
<400> 149
   Ile Ile Trp Ala Ser Gly Thr Thr Phe Tyr Ala Thr Trp Ala Lys Gly
   1 5 10 15
<210> 150
   <211> 13
   <212> PRT
   <213> Artificial
<220>
   <223> 645HeavyS100AFv CDRH3
<400> 150
   Thr Val Pro Gly Tyr Ala Thr Ala Pro Tyr Phe Asp Leu
   1 5 10
<210> 151
   <211> 12
   <212> PRT
   <213> Artificial
<220>
   <223> 645HeavyS100AFv CDRL1
<400> 151
   Gln Ser Ser Pro Ser Val Trp Ser Asn Phe Leu Ser
   1 5 10
<210> 152
   <211> 7
   <212> PRT
   <213> Artificial
<220>
   <223> 645HeavyS100AFv CDRL2
<400> 152
   Glu Ala Ser Lys Leu Thr Ser
   1 5
<210> 153
   <211> 11
   <212> PRT
   <213> Artificial
<220>
   <223> 645HeavyS100AFv CDRL3
<400> 153
   Gly Gly Gly Tyr Ser Ser Ile Ser Asp Thr Thr
   1 5 10
<210> 154
   <211> 10
   <212> PRT
   <213> Artificial
<220>
   <223> 645HeavyT100aAFv CDRH1
<400> 154
   Gly Ile Asp Leu Ser Asn Tyr Ala Ile Asn
   1 5 10
<210> 155
   <211> 16
   <212> PRT
   <213> Artificial
<220>
   <223> 645HeavyT100aAFv CDRH2
<400> 155
   Ile Ile Trp Ala Ser Gly Thr Thr Phe Tyr Ala Thr Trp Ala Lys Gly
   1 5 10 15
<210> 156
   <211> 13
   <212> PRT
   <213> Artificial
<220>
   <223> 645HeavyT100aAFv CDRH3
<400> 156
   Thr Val Pro Gly Tyr Ser Ala Ala Pro Tyr Phe Asp Leu
   1 5 10
<210> 157
   <211> 12
   <212> PRT
   <213> Artificial
<220>
   <223> 645HeavyT100aAFv CDRL1
<400> 157
   Gln Ser Ser Pro Ser Val Trp Ser Asn Phe Leu Ser
   1 5 10
<210> 158
   <211> 7
   <212> PRT
   <213> Artificial
<220>
   <223> 645HeavyT100aAFv CDRL2
<400> 158
   Glu Ala Ser Lys Leu Thr Ser
   1               5
<210> 159
   <211> 11
   <212> PRT
   <213> Artificial
<220>
   <223> 645HeavyT100aAFv CDRL3
<400> 159
   Gly Gly Gly Tyr Ser Ser Ile Ser Asp Thr Thr
   1               5                   10
<210> 160
   <211> 10
   <212> PRT
   <213> Artificial
<220>
   <223> 645HeavyP100cAFv CDRH1
<400> 160
   Gly Ile Asp Leu Ser Asn Tyr Ala Ile Asn
   1               5                   10
<210> 161
   <211> 16
   <212> PRT
   <213> Artificial
<220>
   <223> 645HeavyP100cAFv CDRH2
<400> 161
   Ile Ile Trp Ala Ser Gly Thr Thr Phe Tyr Ala Thr Trp Ala Lys Gly
   1               5                   10                  15
<210> 162
   <211> 13
   <212> PRT
   <213> Artificial
<220>
   <223> 645HeavyP100cAFv CDRH3
<400> 162
   Thr Val Pro Gly Tyr Ser Thr Ala Ala Tyr Phe Asp Leu
   1               5                   10
<210> 163
   <211> 12
   <212> PRT
   <213> Artificial
<220>
   <223> 645HeavyP100cAFv CDRL1
<400> 163
   Gln Ser Ser Pro Ser Val Trp Ser Asn Phe Leu Ser
   1               5                   10
<210> 164
   <211> 7
   <212> PRT
   <213> Artificial
<220>
   <223> 645HeavyP100cAFv CDRL2
<400> 164
   Glu Ala Ser Lys Leu Thr Ser
   1               5
<210> 165
   <211> 11
   <212> PRT
   <213> Artificial
<220>
   <223> 645HeavyP100cAFv CDRL3
<400> 165
   Gly Gly Gly Tyr Ser Ser Ile Ser Asp Thr Thr
   1               5                   10
<210> 166
   <211> 10
   <212> PRT
   <213> Artificial
<220>
   <223> 645HeavyI50A+T95AFv CDRH1
<400> 166
   Gly Ile Asp Leu Ser Asn Tyr Ala Ile Asn
   1               5                   10
<210> 167
   <211> 16
   <212> PRT
   <213> Artificial
<220>
   <223> 645HeavyI50A+T95AFv CDRH2
<400> 167
   Ala Ile Trp Ala Ser Gly Thr Thr Phe Tyr Ala Thr Trp Ala Lys Gly
   1 5 10 15
<210> 168
   <211> 13
   <212> PRT
   <213> Artificial
<220>
   <223> 645HeavyI50A+T95AFv CDRH3
<400> 168
   Ala Val Pro Gly Tyr Ser Thr Ala Pro Tyr Phe Asp Leu
   1 5 10
<210> 169
   <211> 12
   <212> PRT
   <213> Artificial
<220>
   <223> 645HeavyI50A+T95AFv CDRL1
<400> 169
   Gln Ser Ser Pro Ser Val Trp Ser Asn Phe Leu Ser
   1 5 10
<210> 170
   <211> 7
   <212> PRT
   <213> Artificial
<220>
   <223> 645HeavyI50A+T95AFv CDRL2
<400> 170
   Glu Ala Ser Lys Leu Thr Ser
   1 5
<210> 171
   <211> 11
   <212> PRT
   <213> Artificial
<220>
   <223> 645HeavyI50A+T95AFv CDRL3
<400> 171
   Gly Gly Gly Tyr Ser Ser Ile Ser Asp Thr Thr
   1 5 10
<210> 172
   <211> 10
   <212> PRT
   <213> Artificial
<220>
   <223> 645HeavyI50A+G98AFv CDRH1
<400> 172
   Gly Ile Asp Leu Ser Asn Tyr Ala Ile Asn
   1 5 10
<210> 173
   <211> 16
   <212> PRT
   <213> Artificial
<220>
   <223> 645HeavyI50A+G98AFv CDRH2
<400> 173
   Ala Ile Trp Ala Ser Gly Thr Thr Phe Tyr Ala Thr Trp Ala Lys Gly
   1 5 10 15
<210> 174
   <211> 13
   <212> PRT
   <213> Artificial
<220>
   <223> 645HeavyI50A+G98AFv CDRH3
<400> 174
   Thr Val Pro Ala Tyr Ser Thr Ala Pro Tyr Phe Asp Leu
   1 5 10
<210> 175
   <211> 12
   <212> PRT
   <213> Artificial
<220>
   <223> 645HeavyI50A+G98AFv CDRL1
<400> 175
   Gln Ser Ser Pro Ser Val Trp Ser Asn Phe Leu Ser
   1 5 10
<210> 176
   <211> 7
   <212> PRT
   <213> Artificial
<220>
   <223> 645HeavyI50A+G98AFv CDRL2
<400> 176
   Glu Ala Ser Lys Leu Thr Ser
   1 5
<210> 177
   <211> 11
   <212> PRT
   <213> Artificial
<220>
   <223> 645HeavyI50A+G98AFv CDRL3
<400> 177
   Gly Gly Gly Tyr Ser Ser Ile Ser Asp Thr Thr
   1 5 10
<210> 178
   <211> 10
   <212> PRT
   <213> Artificial
<220>
   <223> 645HeavyI50A+Y99AFv CDRH1
<400> 178
   Gly Ile Asp Leu Ser Asn Tyr Ala Ile Asn
   1 5 10
<210> 179
   <211> 16
   <212> PRT
   <213> Artificial
<220>
   <223> 645HeavyI50A+Y99AFv CDRH2
<400> 179
   Ala Ile Trp Ala Ser Gly Thr Thr Phe Tyr Ala Thr Trp Ala Lys Gly
   1 5 10 15
<210> 180
   <211> 13
   <212> PRT
   <213> Artificial
<220>
   <223> 645HeavyI50A+Y99AFv CDRH3
<400> 180
   Thr Val Pro Gly Ala Ser Thr Ala Pro Tyr Phe Asp Leu
   1 5 10
<210> 181
   <211> 12
   <212> PRT
   <213> Artificial
<220>
   <223> 645HeavyI50A+Y99AFv CDRL1
<400> 181
   Gln Ser Ser Pro Ser Val Trp Ser Asn Phe Leu Ser
   1 5 10
<210> 182
   <211> 7
   <212> PRT
   <213> Artificial
<220>
   <223> 645HeavyI50A+Y99AFv CDRL2
<400> 182
   Glu Ala Ser Lys Leu Thr Ser
   1 5
<210> 183
   <211> 11
   <212> PRT
   <213> Artificial
<220>
   <223> 645HeavyI50A+Y99AFv CDRL3
<400> 183
   Gly Gly Gly Tyr Ser Ser Ile Ser Asp Thr Thr
   1 5 10
<210> 184
   <211> 10
   <212> PRT
   <213> Artificial
<220>
   <223> 645HeavyT56A+T95AFv CDRH1
<400> 184
   Gly Ile Asp Leu Ser Asn Tyr Ala Ile Asn
   1 5 10
<210> 185
   <211> 16
   <212> PRT
   <213> Artificial
<220>
   <223> 645HeavyT56A+T95AFv CDRH2
<400> 185
   Ile Ile Trp Ala Ser Gly Ala Thr Phe Tyr Ala Thr Trp Ala Lys Gly
   1 5 10 15
<210> 186
   <211> 13
   <212> PRT
   <213> Artificial
<220>
   <223> 645HeavyT56A+T95AFv CDRH3
<400> 186
   Ala Val Pro Gly Tyr Ser Thr Ala Pro Tyr Phe Asp Leu
   1 5 10
<210> 187
   <211> 12
   <212> PRT
   <213> Artificial
<220>
   <223> 645HeavyT56A+T95AFv CDRL1
<400> 187
   Gln Ser Ser Pro Ser Val Trp Ser Asn Phe Leu Ser
   1 5 10
<210> 188
   <211> 7
   <212> PRT
   <213> Artificial
<220>
   <223> 645HeavyT56A+T95AFv CDRL2
<400> 188
   Glu Ala Ser Lys Leu Thr Ser
   1 5
<210> 189
   <211> 11
   <212> PRT
   <213> Artificial
<220>
   <223> 645HeavyT56A+T95AFv CDRL3
<400> 189
   Gly Gly Gly Tyr Ser Ser Ile Ser Asp Thr Thr
   1 5 10
<210> 190
   <211> 10
   <212> PRT
   <213> Artificial
<220>
   <223> 645HeavyT56A+G98AFv CDRH1
<400> 190
   Gly Ile Asp Leu Ser Asn Tyr Ala Ile Asn
   1 5 10
<210> 191
   <211> 16
   <212> PRT
   <213> Artificial
<220>
   <223> 645HeavyT56A+G98AFv CDRH2
<400> 191
   Ile Ile Trp Ala Ser Gly Ala Thr Phe Tyr Ala Thr Trp Ala Lys Gly
   1 5 10 15
<210> 192
   <211> 13
   <212> PRT
   <213> Artificial
<220>
   <223> 645HeavyT56A+G98AFv CDRH3
<400> 192
   Thr Val Pro Ala Tyr Ser Thr Ala Pro Tyr Phe Asp Leu
   1 5 10
<210> 193
   <211> 12
   <212> PRT
   <213> Artificial
<220>
   <223> 645HeavyT56A+G98AFv CDRL1
<400> 193
   Gln Ser Ser Pro Ser Val Trp Ser Asn Phe Leu Ser
   1 5 10
<210> 194
   <211> 7
   <212> PRT
   <213> Artificial
<220>
   <223> 645HeavyT56A+G98AFv CDRL2
<400> 194
   Glu Ala Ser Lys Leu Thr Ser
   1 5
<210> 195
   <211> 11
   <212> PRT
   <213> Artificial
<220>
   <223> 645HeavyT56A+G98AFv CDRL3
<400> 195
   Gly Gly Gly Tyr Ser Ser Ile Ser Asp Thr Thr
   1 5 10
<210> 196
   <211> 10
   <212> PRT
   <213> Artificial
<220>
   <223> 645HeavyT56A+Y99AFv CDRH1
<400> 196
   Gly Ile Asp Leu Ser Asn Tyr Ala Ile Asn
   1 5 10
<210> 197
   <211> 16
   <212> PRT
   <213> Artificial
<220>
   <223> 645HeavyT56A+Y99AFv CDRH2
<400> 197
   Ile Ile Trp Ala Ser Gly Ala Thr Phe Tyr Ala Thr Trp Ala Lys Gly
   1 5 10 15
<210> 198
   <211> 13
   <212> PRT
   <213> Artificial
<220>
   <223> 645HeavyT56A+Y99AFv CDRH3
<400> 198
   Thr Val Pro Gly Ala Ser Thr Ala Pro Tyr Phe Asp Leu
   1 5 10
<210> 199
   <211> 12
   <212> PRT
   <213> Artificial
<220>
   <223> 645HeavyT56A+Y99AFv CDRL1
<400> 199
   Gln Ser Ser Pro Ser Val Trp Ser Asn Phe Leu Ser
   1 5 10
<210> 200
   <211> 7
   <212> PRT
   <213> Artificial
<220>
   <223> 645HeavyT56A+Y99AFv CDRL2
<400> 200
   Glu Ala Ser Lys Leu Thr Ser
   1 5
<210> 201
   <211> 11
   <212> PRT
   <213> Artificial
<220>
   <223> 645HeavyT56A+Y99AFv CDRL3
<400> 201
   Gly Gly Gly Tyr Ser Ser Ile Ser Asp Thr Thr
   1 5 10
<210> 202
   <211> 119
   <212> PRT
   <213> Artificial
<220>
   <223> 645dsFv Heavy chain Fv
<400> 202
   Glu Val Gln Leu Leu Glu Ser Gly Gly Gly Leu Val Gln Pro Gly Gly
   1 5 10 15
   Ser Leu Arg Leu Ser Cys Ala Val Ser Gly Ile Asp Leu Ser Asn Tyr
   20 25 30
   Ala Ile Asn Trp Val Arg Gln Ala Pro Gly Lys Cys Leu Glu Trp Ile
   35 40 45
   Gly Ile Ile Trp Ala Ser Gly Thr Thr Phe Tyr Ala Thr Trp Ala Lys
   50 55 60
   Gly Arg Phe Thr Ile Ser Arg Asp Ser Thr Thr Val Tyr Leu Gln Met
   65 70 75 80
   Asn Ser Leu Arg Ala Glu Asp Thr Ala Val Tyr Tyr Cys Ala Arg Thr
   85 90 95
   Val Pro Gly Tyr Ser Thr Ala Pro Tyr Phe Asp Leu Trp Gly Gln Gly
   100 105 110
   Thr Leu Val Thr Val Ser Ser
   115
<210> 203
   <211> 110
   <212> PRT
   <213> Artificial
<220>
   <223> 645dsFv Light chain Fv
<400> 203
   Asp Ile Val Met Thr Gln Ser Pro Ser Ser Val Ser Ala Ser Val Gly
   1 5 10 15
   Asp Arg Val Thr Ile Thr Cys Gln Ser Ser Pro Ser Val Trp Ser Asn
   20 25 30
   Phe Leu Ser Trp Tyr Gln Gln Lys Pro Gly Lys Ala Pro Lys Leu Leu
   35 40 45
   Ile Tyr Glu Ala Ser Lys Leu Thr Ser Gly Val Pro Ser Arg Phe Lys
   50 55 60
   Gly Ser Gly Ser Gly Thr Asp Phe Thr Leu Thr Ile Ser Ser Leu Gln
   65 70 75 80
   Pro Glu Asp Phe Ala Thr Tyr Tyr Cys Gly Gly Gly Tyr Ser Ser Ile
   85 90 95
   Ser Asp Thr Thr Phe Gly Cys Gly Thr Lys Val Glu Ile Lys
   100 105 110
<210> 204
   <211> 120
   <212> PRT
   <213> Artificial
<220>
   <223> 648dsFv Heavy chain Fv
<400> 204
   Glu Val Gln Leu Val Glu Ser Gly Gly Gly Leu Val Gln Pro Gly Gly
   1 5 10 15
   Ser Leu Arg Leu Ser Cys Ala Val Ser Gly Phe Ser Leu Ser Arg Tyr
   20 25 30
   Ala Met Thr Trp Val Arg Gln Ala Pro Gly Lys Cys Leu Glu Trp Ile
   35 40 45
   Gly Thr Ile Thr Thr Gly Gly Asn Thr Asn Tyr Ala Asn Trp Ala Lys
   50 55 60
   Gly Arg Phe Thr Ile Ser Lys Asp Ser Thr Thr Val Tyr Leu Gln Met
   65 70 75 80
   Asn Ser Leu Arg Ala Glu Asp Thr Ala Val Tyr Tyr Cys Ala Arg Gly
   85 90 95
   Gly Tyr Val Ser Tyr Ala Asp Ala Thr Glu Leu Ser Leu Trp Gly Gln
   100 105 110
   Gly Thr Leu Val Thr Val Ser Ser
   115 120
<210> 205
   <211> 110
   <212> PRT
   <213> Artificial
<220>
   <223> 648dsFv Light chain Fv
<400> 205
   Asp Ile Val Met Thr Gln Ser Pro Ser Thr Leu Ser Ala Ser Val Gly
   1               5                   10                  15
   Asp Arg Val Thr Ile Thr Cys Gln Ala Ser Gln Ser Ile Gly Ser Arg
               20                  25                  30
   Leu Ala Trp Tyr Gln Gln Lys Pro Gly Lys Ala Pro Lys Leu Leu Ile
           35                  40                  45
   Tyr Tyr Ala Ser Thr Val Ala Ser Gly Val Pro Ser Arg Phe Lys Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Ser Tyr Asp Tyr Ser Ser Ser
                   85                  90                  95
   Ser Ser Tyr Ala Phe Gly Cys Gly Thr Lys Val Glu Ile Lys
               100                 105                 110
<210> 206
   <211> 119
   <212> PRT
   <213> Artificial
<220>
   <223> Heavy chain of 645Fv
<400> 206
   Glu Val Gln Leu Leu Glu Ser Gly Gly Gly Leu Val Gln Pro Gly Gly
   1               5                   10                  15
   Ser Leu Arg Leu Ser Cys Ala Val Ser Gly Ile Asp Leu Ser Asn Tyr
               20                  25                  30
   Ala Ile Asn Trp Val Arg Gln Ala Pro Gly Lys Gly Leu Glu Trp Ile
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Arg Asp Ser Thr Thr Val Tyr Leu Gln Met
   65                  70                  75                  80
   Asn Ser Leu Arg Asp Glu Asp Thr Ala Val Tyr Tyr Cys Ala Arg Thr
                   85                  90                  95
   Val Pro Gly Tyr Ser Thr Ala Pro Tyr Phe Asp Leu Trp Gly Gln Gly
   100 105 110
   Thr Leu Val Thr Val Ser Ser
   115
<210> 207
   <211> 110
   <212> PRT
   <213> Artificial
<220>
   <223> Light chain of 645Fv
<400> 207
   Asp Ile Val Met Thr Gln Ser Pro Ser Ser Val Ser Ala Ser Val Gly
   1 5 10 15
   Asp Arg Val Thr Ile Thr Cys Gln Ser Ser Pro Ser Val Trp Ser Asn
   20 25 30
   Phe Leu Ser Trp Tyr Gln Gln Lys Pro Gly Lys Ala Pro Lys Leu Leu
   35 40 45
   Ile Tyr Glu Ala Ser Lys Leu Thr Ser Gly Val Pro Ser Arg Phe Lys
   50 55 60
   Gly Ser Gly Ser Gly Thr Asp Phe Thr Leu Thr Ile Ser Ser Leu Gln
   65 70 75 80
   Pro Glu Asp Phe Ala Thr Tyr Tyr Cys Gly Gly Gly Tyr Ser Ser Ile
   85 90 95
   Ser Asp Thr Thr Phe Gly Gly Gly Thr Lys Val Glu Ile Lys
   100 105 110
<210> 208
   <211> 11
   <212> PRT
   <213> Artificial
<220>
   <223> Albumin binding peptide
<400> 208
   Asp Leu Cys Leu Arg Asp Trp Gly Cys Leu Trp
   1 5 10
<210> 209
   <211> 11
   <212> PRT
   <213> Artificial
<220>
   <223> Albumin binding peptide
<400> 209
   Asp Ile Cys Leu Pro Arg Trp Gly Cys Leu Trp
   1 5 10
<210> 210
   <211> 15
   <212> PRT
   <213> Artificial
<220>
   <223> Albumin binding peptide
<400> 210
   Met Glu Asp Ile Cys Leu Pro Arg Trp Gly Cys Leu Trp Gly Asp
   1 5 10 15
<210> 211
   <211> 20
   <212> PRT
   <213> Artificial
<220>
   <223> Albumin binding peptide
<400> 211
   Gln Arg Leu Met Glu Asp Ile Cys Leu Pro Arg Trp Gly Cys Leu Trp
   1 5 10 15
   Glu Asp Asp Glu
   20
<210> 212
   <211> 20
   <212> PRT
   <213> Artificial
<220>
   <223> Albumin binding peptide
<400> 212
   Gln Gly Leu Ile Gly Asp Ile Cys Leu Pro Arg Trp Gly Cys Leu Trp
   1 5 10 15
   Gly Arg Ser Val
   20
<210> 213
   <211> 21
   <212> PRT
   <213> Artificial
<220>
   <223> Albumin binding peptide
<400> 213
   Gln Gly Leu Ile Gly Asp Ile Cys Leu Pro Arg Trp Gly Cys Leu Trp
   1 5 10 15
   Gly Arg Ser Val Lys
   20
<210> 214
   <211> 15
   <212> PRT
   <213> Artificial
<220>
   <223> Albumin binding peptide
<400> 214
   Glu Asp Ile Cys Leu Pro Arg Trp Gly Cys Leu Trp Glu Asp Asp
   1 5 10 15
<210> 215
   <211> 18
   <212> PRT
   <213> Artificial
<220>
   <223> Albumin binding peptide
<400> 215
   Arg Leu Met Glu Asp Ile Cys Leu Pro Arg Trp Gly Cys Leu Trp Glu
   1 5 10 15
   Asp Asp
<210> 216
   <211> 16
   <212> PRT
   <213> Artificial
<220>
   <223> Albumin binding peptide
<400> 216
   Met Glu Asp Ile Cys Leu Pro Arg Trp Gly Cys Leu Trp Glu Asp Asp
   1 5 10 15
<210> 217
   <211> 15
   <212> PRT
   <213> Artificial
<220>
   <223> Albumin binding peptide
<400> 217
   Met Glu Asp Ile Cys Leu Pro Arg Trp Gly Cys Leu Trp Glu Asp
   1 5 10 15
<210> 218
   <211> 18
   <212> PRT
   <213> Artificial
<220>
   <223> Albumin binding peptide
<400> 218
   Arg Leu Met Glu Asp Ile Cys Leu Ala Arg Trp Gly Cys Leu Trp Glu
   1 5 10 15
   Asp Asp
<210> 219
   <211> 20
   <212> PRT
   <213> Artificial
<220>
   <223> Albumin binding peptide
<400> 219
   Glu Val Arg Ser Phe Cys Thr Arg Trp Pro Ala Glu Lys Ser Cys Lys
   1 5 10 15
   Pro Leu Arg Gly
   20
<210> 220
   <211> 20
   <212> PRT
   <213> Artificial
<220>
   <223> Albumin binding peptide
<400> 220
   Arg Ala Pro Glu Ser Phe Val Cys Tyr Trp Glu Thr Ile Cys Phe Glu
   1               5                   10                  15
   Arg Ser Glu Gln
               20
<210> 221
   <211> 11
   <212> PRT
   <213> Artificial
<220>
   <223> Albumin binding peptide
<400> 221
   Glu Met Cys Tyr Phe Pro Gly Ile Cys Trp Met
   1               5                   10
<210> 222
   <211> 10
   <212> PRT
   <213> Artificial
<220>
   <223> 646FV CDRH1
<400> 222
   Gly Ile Asp Leu Ser Asn Tyr Ala Ile Asn
   1               5                   10
<210> 223
   <211> 16
   <212> PRT
   <213> Artificial
<220>
   <223> 646FV CDRH2
<400> 223
   Ile Ile Trp Ala Ser Gly Thr Thr Phe Tyr Ala Thr Trp Ala Lys Gly
   1               5                   10                  15
<210> 224
   <211> 10
   <212> PRT
   <213> Artificial
<220>
   <223> Linker
<400> 224
   Gly Gly Gly Gly Ser Gly Gly Gly Gly Ser
   1               5                   10
<210> 225
   <211> 15
   <212> PRT
   <213> Artificial
<220>
   <223> linker
<400> 225
   Gly Gly Gly Gly Ser Gly Gly Gly Gly Ser Gly Gly Gly Gly Ser
   1               5                   10                  15
<210> 226
   <211> 224
   <212> PRT
   <213> Artificial
<220>
   <223> 652 VH domain
<220>
   <221> misc_feature
   <222> (26)..(35)
   <223> Xaa can be any naturally occurring amino acid
<220>
   <221> misc_feature
   <222> (50)..(65)
   <223> Xaa can be any naturally occurring amino acid
<220>
   <221> misc_feature
   <222> (98)..(109)
   <223> Xaa can be any naturally occurring amino acid
<400> 226
   Gln Val Thr Leu Lys Glu Ser Gly Pro Val Leu Val Lys Pro Thr Glu
   1               5                   10                  15
   Thr Leu Thr Leu Thr Cys Thr Val Ser Xaa Xaa Xaa Xaa Xaa Xaa Xaa
               20                  25                  30
   Xaa Xaa Xaa Trp Ile Arg Gln Pro Pro Gly Lys Ala Leu Glu Trp Leu
           35                  40                  45
   Gly Xaa Xaa Xaa Xaa Xaa Xaa Xaa Xaa Xaa Xaa Xaa Xaa Xaa Xaa Xaa
       50                  55                  60
   Xaa Arg Leu Thr Ile Ser Arg Asp Thr Ser Lys Ser Gln Val Val Leu
   65                  70                  75                  80
   Thr Met Thr Asn Met Asp Pro Val Asp Thr Ala Thr Tyr Tyr Cys Ala
                   85                  90                  95
   Arg Xaa Xaa Xaa Xaa Xaa Xaa Xaa Xaa Xaa Xaa Xaa Xaa Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Ser
       210                 215                 220
<210> 227
   <211> 215
   <212> PRT
   <213> Artificial
<220>
   <223> 652 VL domain
<220>
   <221> misc_feature
   <222> (24)..(34)
   <223> Xaa can be any naturally occurring amino acid
<220>
   <221> misc_feature
   <222> (50)..(56)
   <223> Xaa can be any naturally occurring amino acid
<220>
   <221> misc_feature
   <222> (89)..(97)
   <223> Xaa can be any naturally occurring amino acid
<400> 227
   Asp Ile Gln Met Thr Gln Ser Pro Ser Ser Leu Ser Ala Ser Val Gly
   1               5                   10                  15
   Asp Arg Val Thr Ile Thr Cys Xaa Xaa Xaa Xaa Xaa Xaa Xaa Xaa Xaa
               20                  25                  30
   Xaa Xaa Trp Tyr Gln Gln Lys Pro Gly Lys Ala Pro Lys Leu Leu Ile
           35                  40                  45
   Tyr Xaa Xaa Xaa Xaa Xaa Xaa Xaa Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Xaa Xaa Xaa Xaa Xaa Xaa Xaa Xaa
                   85                  90                  95
   Xaa Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser
   210 215
<210> 228
   <211> 6
   <212> PRT
   <213> Artificial
<220>
   <223> linker
<400> 228
   Ala Ser Thr Lys Gly Pro
   1 5
<210> 229
   <211> 5
   <212> PRT
   <213> Artificial
<220>
   <223> linker
<400> 229
   Thr Val Ala Ala Pro
   1 5